(19) Europäisches Patentamt
European Patent Office
Office européen des brevets

(11) EP 4 049 675 A1

(12) **EUROPEAN PATENT APPLICATION**
published in accordance with Art. 153(4) EPC

(43) Date of publication:
**31.08.2022 Bulletin 2022/35**

(21) Application number: **20879730.8**

(22) Date of filing: **23.10.2020**

(51) International Patent Classification (IPC):
***A61K 39/395*** (2006.01) ***A61K 45/00*** (2006.01)
***A61P 35/00*** (2006.01) ***A61P 37/02*** (2006.01)
***A61P 43/00*** (2006.01) ***C12N 15/13*** (2006.01)

(52) Cooperative Patent Classification (CPC):
**A61K 39/395; A61K 45/00; A61P 35/00; A61P 37/02; A61P 43/00**

(86) International application number:
**PCT/JP2020/039802**

(87) International publication number:
**WO 2021/079958 (29.04.2021 Gazette 2021/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.10.2019 JP 2019194717**

(71) Applicant: **Daiichi Sankyo Company, Limited**
**Tokyo 103-8426 (JP)**

(72) Inventors:
- **HAYASHI, Shinko**
  **Tokyo 103-8426 (JP)**
- **ISHIDA, Saori**
  **Tokyo 103-8426 (JP)**
- **SATOH, Kazuki**
  **Tokyo 103-8426 (JP)**
- **HATA, Masato**
  **Tokyo 103-8426 (JP)**

(74) Representative: **Marks & Clerk LLP**
**15 Fetter Lane**
**London EC4A 1BW (GB)**

# (54) COMBINATION OF ANTI-GARP ANTIBODY AND IMMUNOREGULATOR

(57) Provided is a pharmaceutical composition for use in the treatment or prevention of cancer, etc. The present invention provides a pharmaceutical composition or a method for treating cancer, wherein an anti-GARP antibody and an immunomodulator are administered in combination.

Figure 13

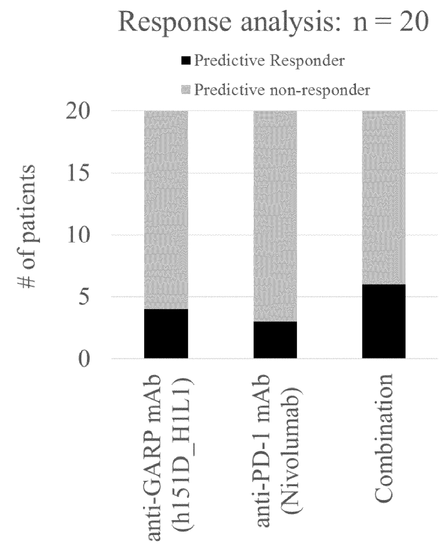




Processed by Luminess, 75001 PARIS (FR)

## Description

Technical Field

The present invention relates to a pharmaceutical composition for cancer treatment and a method for treating cancer, wherein a particular anti-GARP antibody and an immunomodulator or immunomodulators are administered in combination.

Background Art

Regulatory T cells (Tregs) are principal causative cells that bring about immune tolerance found in the tumor areas of cancer patients. Specifically, in cancer patients, antitumor immune cell groups, which supposed to function, are inhibited by Tregs activated by the tumor, and this state leads to the growth of the tumor (Non Patent Literature 1).

Glycoprotein-A repetitions predominant (GARP) is a protein having a single pass transmembrane structure (Non Patent Literature 2). GARP is expressed on the cell surface of activated Tregs and forms a complex with latent TGF-β (a precursor of TGF-β, an important factor for the induction of immune tolerance) (Non Patent Literature 3).

Mature TGF-β is secreted, from latent TGF-β anchored to the cell surface of Tregs via GARP, through the cell-cell interaction between the Tregs and cells such as DC expressing αvβ6 integrin so that immunosuppressive signals of TGF-β are transduced directly to target cells (Non Patent Literatures 4 and 5). This maturation of TGF-β is shown to require the expression of GARP on cell membranes (Non Patent Literature 5). On the other hand, in the case of directly adding soluble GARP deficient in a transmembrane region to CD4-positive T cells, the proliferation of CD4-positive T cells is also suppressed (Non Patent Literature 6). Therefore, the presence of an immunosuppressive mechanism ascribable to GARP without the mediation of the TGF-β maturation mechanism on cell membranes cannot be denied.

The expression of GARP is found in peripheral blood-derived activated Tregs. In addition, its expression is clinically found in Tregs present among tumor infiltrating T cells in cancer patients (Non Patent Literature 7), and Tregs present in ascitic fluid (Non Patent Literature 8) or Tregs present in patients' peripheral blood (Non Patent Literature 9).

Antibodies described in WO2017/051888 (Patent Literature 1), WO2015/015003 (Patent Literature 2), WO2016/125017 (Patent Literature 3), and MHG-8 and LHG-10 described in WO2018/206790 (Patent Literature 4) are known as anti-GARP antibodies (Non Patent Literature 10).

Immunomodulators are drugs that activate antitumor immunity (Non Patent Literatures 11 to 13). Anti-PD-1 antibodies (nivolumab (Patent Literature 5), pembrolizumab (Patent Literature 6), etc.), anti-PD-Ll antibodies (atezolizumab (Patent Literature 7), durvalumab (Patent Literature 8), avelumab (Patent Literature 9), etc.), and anti-CTLA-4 antibodies (ipilimumab (Patent Literature 10), tremelimumab (Patent Literature 11), etc.), which are known as immune checkpoint inhibitors, are known as the immunomodulators.

However, there is not any known, specific combinatorial effect of an anti-GARP antibody and an immunomodulator .

Citation List

Patent Literature

Patent Literature 1: WO2017/051888
Patent Literature 2: WO2015/015003
Patent Literature 3: WO2016/125017
Patent Literature 4: WO2018/206790
Patent Literature 5: WO2006/121168
Patent Literature 6: WO2008/156712
Patent Literature 7: WO2010/077634
Patent Literature 8: WO2011/066389
Patent Literature 9: WO2013/079174
Patent Literature 10: WO2001/014424
Patent Literature 11: WO2000/037504

Non Patent Literature

Non Patent Literature 1: Int J Cancer. 2010 Aug 15; 127(4): 759-67.
Non Patent Literature 2: PLoS One. 2008; 3(7): e2705.
Non Patent Literature 3: Proc Natl Acad Sci U S A. 2009; 106(32): 13445-50.
Non Patent Literature 4: Eur J Immunol. 2009; 39(12): 3315-22.
Non Patent Literature 5: Mol Biol Cell. 2012; 23(6): 1129-39.
Non Patent Literature 6: Blood. 2013; 122(7): 1182-91.
Non Patent Literature 7: Eur J Immunol. 2012 Jul; 42(7): 1876-85.
Non Patent Literature 8: Clin Immunol. 2013 Oct; 149(1): 97-110.
Non Patent Literature 9: Cancer Res. 2013; 73: 2435.
Non Patent Literature 10: Sci Transl Med. 2015 Apr 22; 7(284)
Non Patent Literature 11: Cancers. 2016 Nov 8(12); 106.
Non Patent Literature 12: Nat Rev Cancer. 2012 Mar 12(4); 252-264
Non Patent Literature 13: Cell.2015 Aug 162(5); 937.

Summary of Invention

Technical Problem

The present invention provides a pharmaceutical composition for cancer treatment comprising an anti-GARP antibody and an immunomodulator or immunomodulators which are administered in combination, and a method for treating cancer, comprising administering an anti-GARP antibody and an immunomodulator or immunomodulators in combination.

Solution to Problem

The present inventors have conducted diligent studies to solve the problem and consequently found that an anti-GARP antibody and an immunomodulator administered in combination exhibit an excellent antitumor effect.
Specifically, the present invention relates to the following.

[1] A pharmaceutical composition for cancer treatment comprising an antibody having the following characteristics (1) to (3) or an antigen binding fragment thereof, and an immunomodulator which are administered in combination:

(1) specifically binding to glycoprotein-A repetitions predominant (GARP),
(2) having inhibitory activity against an immunosuppressive function of regulatory T cells, and
(3) having antibody dependent cellular cytotoxicity (ADCC) activity.

[2] The pharmaceutical composition according to [1], wherein the antibody has antitumor activity *in vivo.*
[3] The pharmaceutical composition according to [1] or [2], wherein the antibody comprises a heavy chain comprising CDRH1, CDRH2 and CDRH3 and a light chain comprising CDRL1, CDRL2 and CDRL3 as described in the following (1) or (2):

(1) CDRH1 consisting of the amino acid sequence as set forth in amino acid positions 45 to 54 of SEQ ID NO: 13, CDRH2 consisting of the amino acid sequence as set forth in amino acid positions 69 to 78 of SEQ ID NO: 13 and CDRH3 consisting of the amino acid sequence as set forth in amino acid positions 118 to 125 of SEQ ID NO: 13, and CDRL1 consisting of the amino acid sequence as set forth in amino acid positions 44 to 54 of SEQ ID NO: 15, CDRL2 consisting of the amino acid sequence as set forth in amino acid positions 70 to 76 of SEQ ID NO: 15 and CDRL3 consisting of the amino acid sequence as set forth in amino acid positions 109 to 117 of SEQ ID NO: 15, or
(2) CDRH1 consisting of the amino acid sequence as set forth in amino acid positions 45 to 54 of SEQ ID NO: 17, CDRH2 consisting of the amino acid sequence as set forth in amino acid positions 69 to 77 of SEQ ID NO: 17 and CDRH3 consisting of the amino acid sequence as set forth in amino acid positions 117 to 128 of SEQ ID NO: 17, and CDRL1 consisting of the amino acid sequence as set forth in amino acid positions 44 to 54 of SEQ ID NO: 19, CDRL2 consisting of the amino acid sequence as set forth in amino acid positions 70 to 76 of SEQ ID NO: 19 and CDRL3 consisting of the amino acid sequence as set forth in amino acid positions 109 to 117 of SEQ ID NO: 19.

[4] The pharmaceutical composition according to any one of [1] to [3], wherein the antibody comprises a heavy chain variable region and a light chain variable region as described in any of the following (1) to (3):

(1) a heavy chain variable region consisting of the amino acid sequence as set forth in amino acid positions 20 to 136 of SEQ ID NO: 21 and a light chain variable region consisting of the amino acid sequence as set forth in amino acid positions 21 to 129 of SEQ ID NO: 25,
(2) a heavy chain variable region consisting of the amino acid sequence as set forth in amino acid positions 20 to 136 of SEQ ID NO: 23 and a light chain variable region consisting of the amino acid sequence as set forth in amino acid positions 21 to 129 of SEQ ID NO: 27, or
(3) a heavy chain variable region consisting of the amino acid sequence as set forth in amino acid positions 20 to 139 of SEQ ID NO: 29 and a light chain variable region consisting of the amino acid sequence as set forth in amino acid positions 21 to 129 of SEQ ID NO: 31.

[5] The pharmaceutical composition according to any one of [1] to [4], wherein the antibody is a chimeric antibody.
[6] The pharmaceutical composition according to any one of [1] to [4], wherein the antibody is a humanized antibody.
[7] The pharmaceutical composition according to any one of [1] to [6], wherein the antibody comprises a heavy chain constant region of human IgG1, human IgG2 or human IgG4.
[8] The pharmaceutical composition according to [6] or [7], wherein the antibody comprises a heavy chain and a light chain as described in any of the following (1) to (3):

(1) a heavy chain having the amino acid sequence as set forth in amino acid positions 20 to 466 of SEQ ID NO: 21 and a light chain having the amino acid sequence as set forth in amino acid positions 21 to 234 of SEQ ID NO: 25,
(2) a heavy chain having the amino acid sequence as set forth in amino acid positions 20 to 466 of SEQ ID NO: 23 and a light chain having the amino acid sequence as set forth in amino acid positions 21 to 234 of SEQ ID NO: 27, or
(3) a heavy chain having the amino acid sequence as set forth in amino acid positions 20 to 469 of SEQ ID NO: 29 and a light chain having the amino acid sequence as set forth in amino acid positions 21 to 234 of SEQ ID NO: 31.

[9] The pharmaceutical composition according to [1], wherein the antibody competes with an antibody according to any one of [2] to [8] for binding to GARP.
[10] The pharmaceutical composition according to any one of [1] to [9], wherein the antibody comprises one or two or more modifications selected from the group consisting of N-linked glycosylation, O-linked glycosylation, N-terminal processing, C-terminal processing, deamidation, isomerization of aspartic acid, oxidation of methionine, N-terminal addition of a methionine residue, amidation of a proline residue, and deletion of one or two amino acid residues at the carboxyl terminus of a heavy chain.
[11] The pharmaceutical composition according to [10], wherein one or several amino acid residues are deleted at the carboxyl terminus of a heavy chain.
[12] The pharmaceutical composition according to [10] or [11], wherein one amino acid residue is deleted at the carboxyl termini of both heavy chains.
[13] The pharmaceutical composition according to any one of [10] to [12], wherein a carboxyl-terminal proline residue of the heavy chain is further amidated.
[14] The pharmaceutical composition according to any one of [1] to [13], wherein the immunomodulator is an anti-PD-1 antibody or an antigen binding fragment thereof, an anti-PD-Ll antibody or an antigen binding fragment thereof, an anti-PD-L2 antibody or an antigen binding fragment thereof, an anti-CTLA-4 antibody or an antigen binding fragment thereof, a multispecific antibody comprising any of these antibodies or antigen binding fragments thereof, a radiotherapy, a chemoradiotherapy, or a combination thereof.
[15] The pharmaceutical composition according to [14], wherein the anti-PD-1 antibody is nivolumab, pembrolizumab, lambrolizumab, MK-3475, AMP-224, pidilizumab or LOPD18.
[16] The pharmaceutical composition according to [14], wherein the anti-PD-Ll antibody is atezolizumab, durvalumab or avelumab.
[17] The pharmaceutical composition according to [14], wherein the anti-CTLA-4 antibody is ipilimumab or tremelimumab.
[18] The pharmaceutical composition for cancer treatment according to any one of [1] to [17], wherein the antibody and the immunomodulator are contained as active ingredients in separate preparations and administered at the same time or at different times.
[19] A pharmaceutical composition for cancer treatment comprising an antibody according to any one of [1] to [13] which is to be combined with an immunomodulator.
[20] A pharmaceutical composition for cancer treatment comprising an antibody according to any one of [1] to [13] which is to treat a cancer patient given an immunomodulator.

[21] A pharmaceutical composition for cancer treatment comprising an immunomodulator, wherein the pharmaceutical composition is combined with an antibody according to any one of [1] to [13], thereby increasing an effect of the antibody.
[22] A pharmaceutical composition for cancer treatment comprising an immunomodulator which is to be combined with an antibody according to any one of [1] to [13] .
[23] A pharmaceutical composition for cancer treatment comprising an immunomodulator which is to treat a cancer patient given an antibody according to any one of [1] to [13].
[24] A pharmaceutical composition for cancer treatment comprising an antibody according to any one of [1] to [13], wherein the pharmaceutical composition is combined with an immunomodulator, thereby increasing an effect of the immunomodulator.
[25] The pharmaceutical composition according to any one of [1] to [24] which is to treat at least one cancer selected from the group consisting of lung cancer, kidney cancer, urothelial cancer, large intestine cancer, prostatic cancer, glioblastoma multiforme, ovary cancer, pancreatic cancer, breast cancer, melanoma, liver cancer, bladder cancer, stomach cancer, esophageal cancer, head and neck cancer, blood cancer, skin cancer, thyroid gland cancer, biliary tract cancer, salivary gland cancer, small intestine cancer, adrenal cancer, testicle cancer, uterine cervical cancer, endometrial cancer, uterine sarcoma, thymoma, mesothelioma, sarcoma and their metastatic forms.
[26] A method for treating cancer, comprising administering an antibody having the following characteristics (1) to (3) or an antigen binding fragment thereof, and an immunomodulator in combination:

(1) specifically binding to glycoprotein-A repetitions predominant (GARP),
(2) having inhibitory activity against an immunosuppressive function of regulatory T cells, and
(3) having antibody dependent cellular cytotoxicity (ADCC) activity.

[27] The method for treating cancer according to [26], wherein the antibody has antitumor activity *in vivo.*
[28] The method according to [26] or [27], wherein the antibody comprises a heavy chain comprising CDRH1, CDRH2 and CDRH3 and a light chain comprising CDRL1, CDRL2 and CDRL3 as described in the following (1) or (2):

(1) CDRH1 consisting of the amino acid sequence as set forth in amino acid positions 45 to 54 of SEQ ID NO: 13, CDRH2 consisting of the amino acid sequence as set forth in amino acid positions 69 to 78 of SEQ ID NO: 13 and CDRH3 consisting of the amino acid sequence as set forth in amino acid positions 118 to 125 of SEQ ID NO: 13, and CDRL1 consisting of the amino acid sequence as set forth in amino acid positions 44 to 54 of SEQ ID NO: 15, CDRL2 consisting of the amino acid sequence as set forth in amino acid positions 70 to 76 of SEQ ID NO: 15 and CDRL3 consisting of the amino acid sequence as set forth in amino acid positions 109 to 117 of SEQ ID NO: 15, or
(2) CDRH1 consisting of the amino acid sequence as set forth in amino acid positions 45 to 54 of SEQ ID NO: 17, CDRH2 consisting of the amino acid sequence as set forth in amino acid positions 69 to 77 of SEQ ID NO: 17 and CDRH3 consisting of the amino acid sequence as set forth in amino acid positions 117 to 128 of SEQ ID NO: 17, and CDRL1 consisting of the amino acid sequence as set forth in amino acid positions 44 to 54 of SEQ ID NO: 19, CDRL2 consisting of the amino acid sequence as set forth in amino acid positions 70 to 76 of SEQ ID NO: 19 and CDRL3 consisting of the amino acid sequence as set forth in amino acid positions 109 to 117 of SEQ ID NO: 19.

[29] The method according to any one of [26] to [28], wherein the antibody comprises a heavy chain variable region and a light chain variable region as described in any of the following (1) to (3):

(1) a heavy chain variable region consisting of the amino acid sequence as set forth in amino acid positions 20 to 136 of SEQ ID NO: 21 and a light chain variable region consisting of the amino acid sequence as set forth in amino acid positions 21 to 129 of SEQ ID NO: 25,
(2) a heavy chain variable region consisting of the amino acid sequence as set forth in amino acid positions 20 to 136 of SEQ ID NO: 23 and a light chain variable region consisting of the amino acid sequence as set forth in amino acid positions 21 to 129 of SEQ ID NO: 27, or
(3) a heavy chain variable region consisting of the amino acid sequence as set forth in amino acid positions 20 to 139 of SEQ ID NO: 29 and a light chain variable region consisting of the amino acid sequence as set forth in amino acid positions 21 to 129 of SEQ ID NO: 31.

[30] The method according to any one of [26] to [29], wherein the antibody is a chimeric antibody.
[31] The method according to any one of [26] to [29], wherein the antibody is a humanized antibody.
[32] The method according to any one of [26] to [31], wherein the antibody comprises a heavy chain constant region of human IgG1, human IgG2 or human IgG4.
[33] The method according to [31] or [32], wherein the antibody comprises a heavy chain and a light chain as described in any of the following (1) to (3):

(1) a heavy chain having the amino acid sequence as set forth in amino acid positions 20 to 466 of SEQ ID NO: 21 and a light chain having the amino acid sequence as set forth in amino acid positions 21 to 234 of SEQ ID NO: 25,
(2) a heavy chain having the amino acid sequence as set forth in amino acid positions 20 to 466 of SEQ ID NO: 23 and a light chain having the amino acid sequence as set forth in amino acid positions 21 to 234 of SEQ ID NO: 27, or
(3) a heavy chain having the amino acid sequence as set forth in amino acid positions 20 to 469 of SEQ ID NO: 29 and a light chain having the amino acid sequence as set forth in amino acid positions 21 to 234 of SEQ ID NO: 31.

[34] The method according to [26], wherein the antibody competes with an antibody according to any one of [27] to [33] for binding to GARP.
[35] The method according to any one of [26] to [34], wherein the antibody comprises one or two or more modifications selected from the group consisting of N-linked glycosylation, O-linked glycosylation, N-terminal processing, C-terminal processing, deamidation, isomerization of aspartic acid, oxidation of methionine, N-terminal addition of a methionine residue, amidation of a proline residue, and deletion of one or two amino acid residues at the carboxyl terminus of a heavy chain.
[36] The method according to [35], wherein one or several amino acid residues are deleted at the carboxyl terminus of a heavy chain.
[37] The method according to [35] or [36], wherein one amino acid residue is deleted at the carboxyl termini of both heavy chains.
[38] The method according to any one of [35] to [37], wherein a carboxyl-terminal proline residue of the heavy chain is further amidated.
[39] The method according to any one of [26] to [38], wherein the immunomodulator is an anti-PD-1 antibody or an antigen binding fragment thereof, an anti-PD-L1 antibody or an antigen binding fragment thereof, an anti-PD-L2 antibody or an antigen binding fragment thereof, an anti-CTLA-4 antibody or an antigen binding fragment thereof, a multispecific antibody comprising any of these antibodies or antigen binding fragments thereof, a radiotherapy, a chemoradiotherapy, or a combination thereof.
[40] The method according to [39], wherein the anti-PD-1 antibody is nivolumab, pembrolizumab, lambrolizumab, MK-3475, AMP-224, pidilizumab or LOPD18.
[41] The method according to [39], wherein the anti-PD-Ll antibody is atezolizumab, durvalumab or avelumab.
[42] The method according to [39], wherein the anti-CTLA-4 antibody is ipilimumab or tremelimumab.
[43] A method for treating cancer, comprising administering an effective amount of an antibody according to any one of [26] to [38] to a cancer patient in need thereof, wherein the method is combined with administering an immunomodulator.
[44] A method for treating a cancer patient given an immunomodulator, comprising administering an effective amount of an antibody according to any one of [26] to [38] to the patient in need thereof.
[45] A method for treating cancer which is combined with administration of an antibody according to any one of [26] to [38], thereby increasing an effect of the antibody, the method comprising administering an effective amount of an immunomodulator to a patient in need thereof.
[46] A method for treating cancer, comprising administering an effective amount of an immunomodulator to a patient in need thereof, wherein the method is combined with administration of an antibody according to any one of [26] to [38].
[47] A method for treating a cancer patient given an antibody according to any one of [26] to [38], comprising administering an effective amount of an immunomodulator to the patient in need thereof.
[48] A method for treating cancer which is combined with administration of an immunomodulator, thereby increasing an effect of the immunomodulator, the method comprising administering an effective amount of an antibody according to any one of [26] to [38] to a patient in need thereof.
[49] The method according to any one of [26] to [48], wherein the method is to treat at least one cancer selected from the group consisting of lung cancer, kidney cancer, urothelial cancer, large intestine cancer, prostatic cancer, glioblastoma multiforme, ovary cancer, pancreatic cancer, breast cancer, melanoma, liver cancer, bladder cancer, stomach cancer, esophageal cancer, head and neck cancer, blood cancer, skin cancer, thyroid gland cancer, biliary tract cancer, salivary gland cancer, small intestine cancer, adrenal cancer, testicle cancer, uterine cervical cancer, endometrial cancer, uterine sarcoma, thymoma, mesothelioma, sarcoma and their metastatic forms.
[50] A kit preparation for cancer treatment comprising an antibody according to any one of [1] to [12], and one or two or more immunomodulators selected from the group consisting of an anti-PD-1 antibody, an anti-PD-Ll antibody, an anti-PD-L2 antibody and an anti-CTLA-4 antibody.
[51] The preparation according to [50], further comprising an instruction manual for using the kit.
[52] The pharmaceutical composition according to [14], wherein the anti-PD-1 antibody is nivolumab.
[53] The pharmaceutical composition according to [14], wherein the anti-PD-1 antibody is pembrolizumab.
[54] The method according to [39], wherein the anti-PD-1 antibody is nivolumab.
[55] The method according to [39], wherein the anti-PD-1 antibody is pembrolizumab.
[56] The pharmaceutical composition according to any one of [1] to [13], wherein the immunomodulator is an anti-PD-1 antibody or an antigen binding fragment thereof, an anti-PD-Ll antibody or an antigen binding fragment thereof, an anti-PD-L2 antibody or an antigen binding fragment thereof, an anti-CTLA-4 antibody or an antigen binding fragment thereof, a multispecific antibody comprising any of these antibodies or antigen binding fragments thereof, a radiotherapy, a chemoradiotherapy, a chemotherapeutic or a combination thereof.
[57] The method according to any one of [26] to [38], wherein the immunomodulator is an anti-PD-1 antibody or an antigen binding fragment thereof, an anti-PD-L1 antibody or an antigen binding fragment thereof, an anti-PD-L2 antibody or an antigen binding fragment thereof, an anti-CTLA-4 antibody or an antigen binding fragment thereof, a multispecific antibody comprising any of these antibodies or antigen binding fragments thereof, a radiotherapy, a chemoradiotherapy, a chemotherapeutic or a combination thereof.

## Advantageous Effects of Invention

The present invention can provide a pharmaceutical composition and a treatment method which are excellent in their antitumor effects and safety by administering a particular anti-GARP antibody and an immunomodulator or immunomodulators in combination.

## Brief Description of Drawings

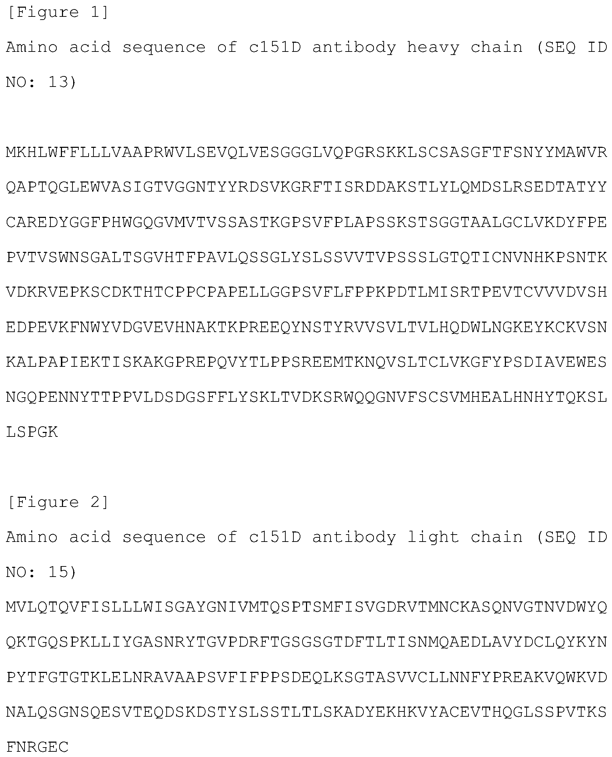

[Figure 1] Figure 1 is a diagram showing the amino acid sequence of a c151D antibody heavy chain (SEQ ID NO: 13).
[Figure 2] Figure 2 is a diagram showing the amino acid sequence of a c151D antibody light chain (SEQ ID NO: 15).

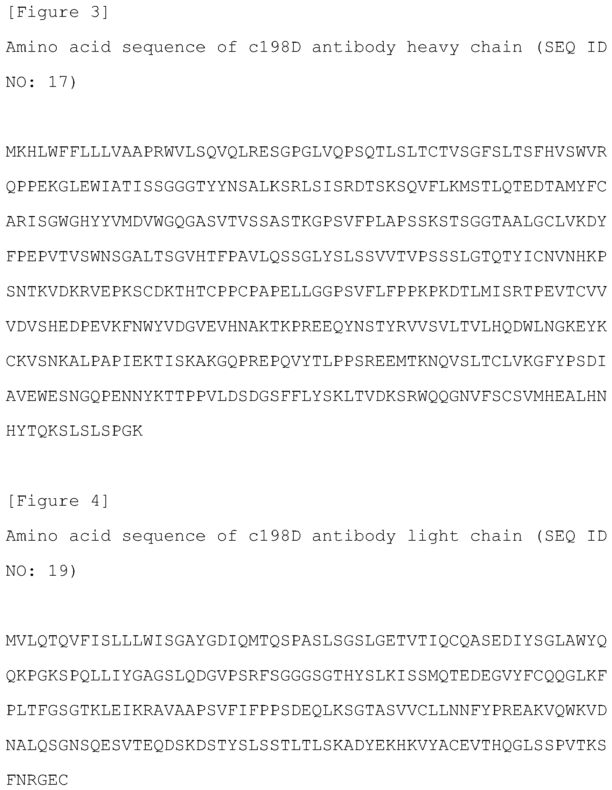

[Figure 3] Figure 3 is a diagram showing the amino acid sequence of a c198D antibody heavy chain (SEQ ID NO: 17).
[Figure 4] Figure 4 is a diagram showing the amino acid sequence of a c198D antibody light chain (SEQ ID NO: 19).

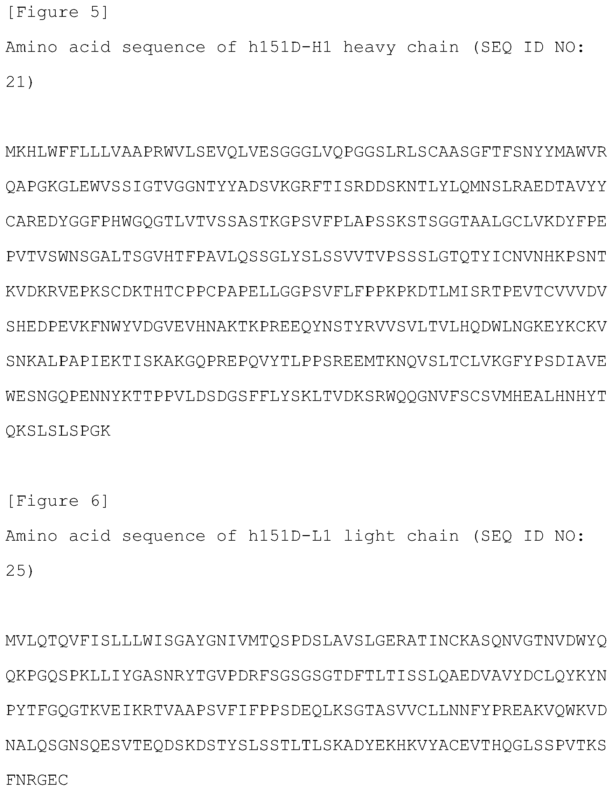

[Figure 5] Figure 5 is a diagram showing the amino acid sequence of an h151D-H1 heavy chain (SEQ ID NO: 21).
[Figure 6] Figure 6 is a diagram showing the amino acid sequence of an h151D-L1 light chain (SEQ ID NO: 25).

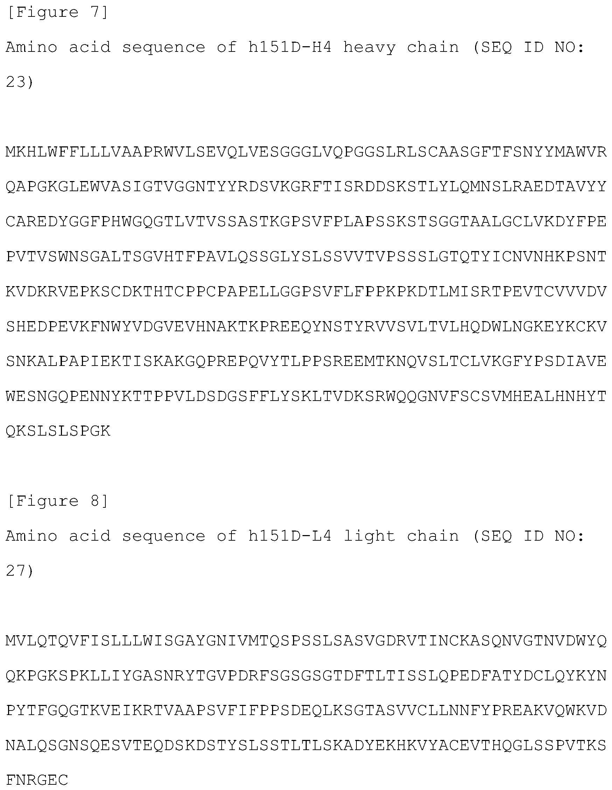

[Figure 7] Figure 7 is a diagram showing the amino acid sequence of an h151D-H4 heavy chain (SEQ ID NO: 23).
[Figure 8] Figure 8 is a diagram showing the amino acid sequence of an h151D-L4 light chain (SEQ ID NO: 27).

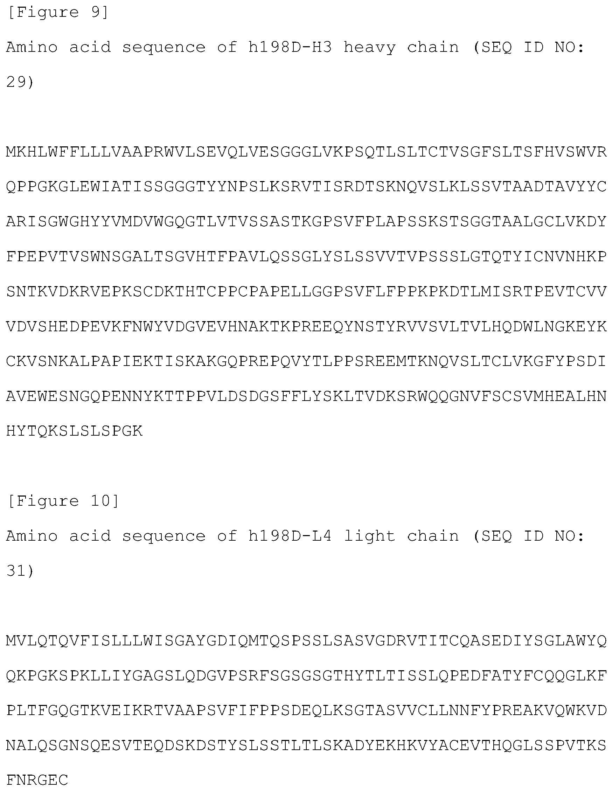

[Figure 9] Figure 9 is a diagram showing the amino acid sequence of an h198D-H3 heavy chain (SEQ ID NO: 29).
[Figure 10] Figure 10 is a diagram showing the amino acid sequence of an h198D-L4 light chain (SEQ ID NO: 31).
[Figure 11-1] Figure 11-1 is a diagram showing means and standard deviations (6 wells/group) of the amounts of [$^3$H]-thymidine taken up into Teff cells for a control group, an anti-GARP antibody h151D_H1L1 single agent treatment group, an anti-PD-1 antibody nivolumab single agent treatment group, and an anti-GARP antibody h151D_H1L1/nivolumab combined treatment group in donors A and B.
[Figure 11-2] Figure 11-2 is a diagram showing means and standard deviations (6 wells/group) of the amounts of [$^3$H]-thymidine taken up into Teff cells for a control group, an anti-GARP antibody h151D_H1L1 single agent treatment group, an anti-PD-1 antibody nivolumab single agent treatment group, and an anti-GARP antibody h151D_H1L1/nivolumab combined treatment group in donors C and D.
[Figure 12] Figure 12 is a diagram showing relative AUC values, which are an index for activation of Teff cell proliferation, for a control group, an anti-GARP antibody h151D_H1L1 single agent administration group, an anti-PD-1 antibody nivolumab single agent administration group, and an anti-GARP antibody h151D_H1L1/anti-PD-1 antibody nivolumab combined administration group in four donors.
[Figure 13] Figure 13 is a diagram showing the prediction of a clinical efficacy using CANscript(R) when the anti-GARP antibody (h151D_H1L1) and the anti-PD-1 antibody nivolumab are combined.

## Description of Embodiments

Hereinafter, preferable modes for carrying out the present invention will be described with reference to the drawings. The embodiments described below illustrate exemplary typical embodiments of the present invention, by which the scope of the present invention is not limited by any means.

1. GARP

The amino acid sequence of human GARP is described in SEQ ID NO: 1 (NP_001122394) of the Sequence Listing.

GARP also includes a protein that consists of an amino acid sequence derived from the amino acid sequence of GARP described above by the substitution, deletion and/or addition of one or several amino acids and has bioactivity equivalent to the protein.

Mature human GARP without a signal sequence corresponds to an amino acid sequence consisting of amino acid residues from positions 20 to 662 of the amino acid sequence represented by SEQ ID NO: 1.

GARP also includes a protein that consists of an amino acid sequence derived from the amino acid sequence represented by SEQ ID NO: 1 of the Sequence Listing or from an amino acid sequence obtained by the removal of a signal sequence from the sequence, by the substitution, deletion, or addition of one or several amino acids, and has bioactivity equivalent to GARP. GARP further includes a protein that consists of an amino acid sequence encoded by a splicing variant transcribed from the human GARP gene locus or an amino acid sequence derived from the amino acid sequence by the substitution, deletion, or addition of one or several amino acids and has bioactivity equivalent to GARP.

2. Anti-GARP antibody

Examples of the function of the antibody of the present invention can include, but are not limited to, antigen binding activity, the activity of neutralizing the activity of the antigen, the activity of potentiating the activity of the antigen, and effector activity (ADCC activity, antibody dependent cell phagocytosis (ADCP) activity and complement dependent cytotoxicity (CDC) activity, etc.).

The antibody of the present invention preferably has any one of GARP-specific binding activity, effector activity, Treg function inhibitory activity, and cytotoxic activity (antitumor activity) or a combination thereof and more preferably has GARP-specific binding activity or cytotoxic activity (antitumor activity) ascribable to Treg function inhibitory activity via effector activity (preferably ADCC activity) or a combination thereof.

In the present invention, "cancer" and "tumor" have the same meaning.

The antibody of the present invention recognizes the GARP protein. In other words, the antibody of the present invention binds to the GARP protein. Such an antibody is referred to as an "anti-GARP antibody". The preferable antibody of the present invention specifically recognizes the GARP protein. In other words, the preferable antibody of the present invention specifically binds to the GARP protein.

In the present invention, "specific recognition", i.e., "specific binding", means binding that is not nonspecific adsorption. Examples of the criterion for determining whether or not binding is specific can include the dissociation constant (hereinafter, referred to as "KD"). The KD value of the preferable antibody of the present invention for the GARP protein is $1 \times 10^{-5}$ M or less, $5 \times 10^{-6}$ M or less, $2 \times 10^{-6}$ M or less or $1 \times 10^{-6}$ M or less, more preferably $5 \times 10^{-7}$ M or less, $2 \times 10^{-7}$ M or less or $1 \times 10^{-7}$ M or less, still more preferably $5 \times 10^{-8}$ M or less, $2 \times 10^{-8}$ M or less or $1 \times 10^{-8}$ M or less, yet more preferably $5 \times 10^{-9}$ M or less, $2 \times 10^{-9}$ M or less or $1 \times 10^{-9}$ M or less.

In the present invention, the binding of an antibody to an antigen can be measured or determined by ELISA, RIA, a surface plasmon resonance (hereinafter, referred to as "SPR") analysis method, or the like. Examples of the equipment for use in SPR analysis can include BIAcore(TM) (manufactured by GE Healthcare Japan Corp.), ProteOn(TM) (manufactured by Bio-Rad Laboratories, Inc.), SPR-Navi(TM) (manufactured by BioNavis Ltd.), Spreeta(TM) (manufactured by Texas Instruments Inc.), SPRi-PlexII(TM) (manufactured by HORIBA, Ltd.), and Autolab SPR(TM) (manufactured by Metrohm AG). The binding of an antibody to an antigen expressed on a cell surface can be measured by flow cytometry, Cell ELISA, or the like.

In the present invention, ADCC (antibody dependent cellular cytotoxicity) refers to a cell-mediated reaction through which nonspecific cytotoxic cells (e.g., NK cells, neutrophils, and macrophages) expressing an Fcγ receptor recognize an antibody bound onto target cells and then cause the lysis of the target cells.

In the present invention, ADCP (antibody dependent cell phagocytosis) refers to a cell-mediated reaction through which phagocytes (e.g., macrophages and neutrophils) expressing an Fc receptor recognize an antibody bound onto target cells and then phagocytize the target cells.

In the present invention, CDC (complement dependent cytotoxicity) refers to action or activity by which complement damages target cells such as tumor cells via an antibody.

The ADCC activity, the CDC activity and the ADCP activity can be measured by a method known in the art.

The measurement of ADCC activity employs cells (target cells) expressing the antigen of interest and effector cells which kill the target cells. The effector cells recognize the Fc regions of an antibody bound to the target cells via the Fcγ receptor. The effector cells kill the target cells through signals transduced from the Fcγ receptor. In the case of measuring the ADCC activity of an antibody having a human-derived Fc region, human NK cells are used as the effector cells. The human NK cells can be prepared by a method known in the art from human peripheral blood mononuclear cells (PBMCs). Alternatively, PBMCs may be used directly as the effector cells.

The measurement of ADCP activity employs cells (target cells) expressing the antigen of interest and effector cells, such as monocytes or macrophages, which phagocytize the target cells. These effector cells can be prepared by inducing the differentiation of a monocyte fraction separated by a method known in the art from human peripheral blood mononuclear cells (PBMCs) into macrophages by a method known in the art.

The measurement of CDC activity employs cells (target cells) expressing the antigen of interest and a complement component. The complement component is activated by binding to an antibody bound to the target cells and thereby causes a series of reactions on the cell surface to form a membrane invasive complex, which in turn kills the target cells. Human serum or the like can be used as this complement component.

<Production of anti-GARP antibody>

The anti-GARP antibody can be obtained by immunizing an animal with GARP or an arbitrary polypeptide selected from the amino acid sequence of GARP, and collecting and purifying an antibody produced *in vivo*.

(1) Preparation of antigen

Examples of the antigen for preparing the anti-GARP antibody can include GARP, a polypeptide consisting of a partial amino acid sequence of at least six consecutive amino acids thereof, and a derivative further having an arbitrary amino acid sequence or carrier added thereto.

GARP can be purified directly from human tumor tissues or tumor cells and used, or GARP can be obtained by *in vitro* synthesis or by production from host cells through gene engineering. In such gene engineering, specifically, GARP cDNA is integrated into a vector that permits expression. Then, the antigen can be obtained by synthesis in a solution containing enzymes, substrates and energy substances necessary for transcription and translation, or by the expression of GARP from prokaryotic or eukaryotic host cells transformed with the vector.

Alternatively, the antigen may be obtained as a secretory protein by the expression of a fusion protein of the extracellular region of the membrane protein GARP linked to the constant regions of an antibody in an appropriate host-vector system.

(2) Production of anti-GARP monoclonal antibody

The antibody of the present invention can be obtained by an approach known in the art. The antibody can be obtained, for example, according to a method usually carried out in the art, by immunizing an animal with the antigen GARP or an arbitrary polypeptide selected from the amino acid sequence of GARP, and collecting and purifying an antibody produced *in vivo*. The origin of the antigen is not limited to a human. The animal may be immunized with an antigen derived from a nonhuman animal such as a mouse or a rat. In this case, an anti-GARP antibody applicable to a human disease can be selected by testing the cross-reactivity of an antibody that binds to the obtained heteroantigen with a human antigen.

Also, a monoclonal antibody can be obtained by establishing a hybridoma through the fusion of antibody-producing cells that produce an antibody against the antigen and myeloma cells (e.g., Kohler and Milstein, Nature (1975) 256, p. 495-497; and Kennet, R. ed., Monoclonal Antibodies, p. 365-367, Plenum Press, N.Y. (1980)).

Examples of the hybridoma line thus established can include hybridomas 151D and 198D against GARP. In the present specification, antibodies produced by the hybridomas 151D and 198D against GARP are referred to as a "151D antibody" and a "198D antibody", or simply as "151D" and "198D", respectively (see US2018258184).

The heavy chain variable region of the 151D antibody has the amino acid sequence represented by SEQ ID NO: 3. The light chain variable region of the 151D antibody has the amino acid sequence represented by SEQ ID NO: 5. The amino acid sequence of the heavy chain variable region represented by SEQ ID NO: 3 is encoded by the nucleotide sequence represented by SEQ ID NO: 2. The amino acid sequence of the light chain variable region represented by SEQ ID NO: 5 is encoded by the nucleotide sequence represented by SEQ ID NO: 4.

The heavy chain variable region of the 198D antibody has the amino acid sequence represented by SEQ ID NO: 7. The light chain variable region of the 198D antibody has the amino acid sequence represented by SEQ ID NO: 9. The amino acid sequence of the heavy chain variable region represented by SEQ ID NO: 7 is encoded by the nucleotide sequence represented by SEQ ID NO: 6. The amino acid sequence of the light chain variable region represented by SEQ ID NO: 9 is encoded by the nucleotide sequence represented by SEQ ID NO: 8.

(3) Other antibodies

Examples of the antibody of the present invention can include an antibody that binds to the same epitope as that for the 151D antibody or the 198D antibody. Since the 151D antibody (humanized 151D antibody, etc.) recognizes amino acid positions 352 to 392 in the sequence represented by SEQ ID NO: 1, and the 198D antibody (humanized 198D antibody, etc.) recognizes and binds to amino acid positions 18 to 112 in the sequence represented by SEQ IN NO: 1, examples of the epitope can include these regions in the amino acid sequence of GARP.

Provided that a newly prepared monoclonal antibody binds to a partial peptide or a partial conformation to which the 151D antibody, etc. binds, this monoclonal antibody can be determined as binding to the same epitope as that for the antibody such as the 151D antibody. As long as the monoclonal antibody is confirmed to compete with the antibody such as the 151D antibody for binding to GARP (i.e., the monoclonal antibody interferes with the binding of the 151D antibody, etc. to GARP), this monoclonal antibody can be determined as binding to the same epitope as that for the antibody such as the 151D antibody even if the sequence or structure of a specific epitope has not been determined. The monoclonal antibody, when confirmed to bind to the same epitope, is strongly expected to have characteristics equivalent to the antibody such as the 151D antibody.

The antibody of the present invention includes a monoclonal antibody against GARP as well as a recombinant antibody artificially engineered for the purpose of, for example, reducing heteroantigenicity against humans, for example, a chimeric antibody and a humanized antibody, and a human antibody, etc. These antibodies can be produced by use of known methods.

Examples of the anti-GARP antibody according to the present invention can also include the following chimeric antibodies and humanized antibodies.

Examples of the chimeric antibody can include an antibody comprising variable regions and constant regions of antibodies derived from different species, for example, a chimeric antibody comprising variable regions of a mouse- or rat-derived antibody joined to human-derived constant regions (see Proc. Natl. Acad. Sci. U.S.A., 81, 6851-6855, (1984)).

The chimeric antibody derived from the rat antihuman GARP antibody 151D is an antibody consisting of a heavy chain comprising a heavy chain variable region consisting of the amino acid sequence as set forth in SEQ ID NO: 3 and a light chain comprising a light chain variable region consisting of the amino acid sequence as set forth in SEQ ID NO: 5, and may have arbitrary human-derived constant regions.

The chimeric antibody derived from the rat antihuman GARP antibody 198D is an antibody consisting of a heavy chain comprising a heavy chain variable region consisting of the amino acid sequence as set forth in SEQ ID NO: 7 and a light chain comprising a light chain variable region consisting of the amino acid sequence as set forth in SEQ ID NO: 9, and may have arbitrary human-derived constant regions.

Examples of such a chimeric antibody can include an antibody consisting of a heavy chain having the amino acid sequence as set forth in amino acid positions 20 to 466 of SEQ ID NO: 13 and a light chain having the amino acid sequence as set forth in amino acid positions 21 to 234 of SEQ ID NO: 15, and an antibody consisting of a heavy chain having the amino acid sequence as set forth in amino acid positions 20 to 469 of SEQ ID NO: 17 of the Sequence Listing and a light chain having the amino acid sequence as set forth in amino acid positions 21 to 234 of SEQ ID NO: 19.

In the heavy chain sequence represented by SEQ ID NO: 13 of the Sequence Listing, the amino acid sequence as set forth in positions 1 to 19 corresponds to a signal sequence, the amino acid sequence as set forth in positions 20 to 136 corresponds to a variable region, and the amino acid sequence as set forth in positions 137 to 466 corresponds to a constant region.

In the light chain sequence represented by SEQ ID NO: 15 of the Sequence Listing, the amino acid sequence as set forth in positions 1 to 20 corresponds to a signal sequence, the amino acid sequence as set forth in positions 21 to 129 corresponds to a variable region, and the amino acid sequence as set forth in positions 130 to 234 corresponds to a constant region.

In the heavy chain sequence represented by SEQ ID NO: 17 of the Sequence Listing, the amino acid sequence as set forth in positions 1 to 19 corresponds to a signal sequence, the amino acid sequence as set forth in positions 20 to 139 corresponds to a variable region, and the amino acid sequence as set forth in positions 140 to 469 corresponds to a constant region.

In the light chain sequence represented by SEQ ID NO: 19 of the Sequence Listing, the amino acid sequence as set forth in positions 1 to 20 corresponds to a signal sequence, the amino acid sequence as set forth in positions 21 to 129 corresponds to a variable region, and the amino acid sequence as set forth in positions 130 to 234 corresponds to a constant region.

Examples of the humanized antibody can include an antibody comprising complementarity determining regions (CDRs) alone integrated into a human-derived antibody (see Nature (1986) 321, p. 522-525), and an antibody comprising the CDR sequences as well as amino acid residues of a portion of a framework region grafted into a human antibody by a CDR grafting method (WO90/07861).

However, the humanized antibody derived from the 151D antibody is not limited to a particular humanized antibody as long as the humanized antibody maintains all six CDR sequences of the 151D antibody and has antitumor activity.

The heavy chain variable region of the 151D antibody retains CDRH1 (GFTFSNYYMA) consisting of the amino acid sequence as set forth in amino acid positions 26 to 35 of SEQ ID NO: 3 of the Sequence Listing, CDRH2 (SIGTVGGNTY) consisting of the amino acid sequence as set forth in amino acid positions 50 to 59 of SEQ ID NO: 3, and CDRH3 (EDYGGFPH) consisting of the amino acid sequence as set forth in amino acid positions 99 to 106 of SEQ ID NO: 3.

The light chain variable region of the 151D antibody retains CDRL1 (KASQNVGTNVD) consisting of the amino acid sequence as set forth in amino acid positions 24 to 34 of SEQ ID NO: 5 of the Sequence Listing, CDRL2 (GASNRYT) consisting of the amino acid sequence as set forth in amino acid positions 50 to 56 of SEQ ID NO: 5, and CDRL3 (LQYKYNPYT) consisting of the amino acid sequence as set forth in amino acid positions 89 to 97 of SEQ ID NO: 5.

The heavy chain variable region of the 198D antibody retains CDRH1 (GFSLTSFHVS) consisting of the amino acid sequence as set forth in amino acid positions 26 to 35 of SEQ ID NO: 7 of the Sequence Listing, CDRH2 (TISSGGGTY) consisting of the amino acid sequence as set forth in amino acid positions 50 to 58 of SEQ ID NO: 7, and CDRH3 (ISGWGHYYVMDV) consisting of the amino acid sequence as set forth in amino acid positions 98 to 109 of SEQ ID NO: 7.

The light chain variable region of the 198D antibody retains CDRL1 (QASEDIYSGLA) consisting of the amino acid sequence as set forth in amino acid positions 24 to 34 of SEQ ID NO: 9 of the Sequence Listing, CDRL2 (GAGSLQD) consisting of the amino acid sequence as set forth in amino acid positions 50 to 56 of SEQ ID NO: 9, and CDRL3 (QQGLKFPLT) consisting of the amino acid sequence as set forth in amino acid positions 89 to 97 of SEQ ID NO: 9.

Actual examples of the humanized antibody of the rat antibody 151D can include arbitrary combinations of a heavy chain comprising a heavy chain variable region consisting of any one of (1) the amino acid sequence as set forth in amino acid positions 20 to 136 of SEQ ID NO: 21 (h151D-H1) or SEQ ID NO: 23 (h151D-H4) of the Sequence Listing, (2) an amino acid sequence having at least 95% or higher homology to the sequences of the framework regions outside of the CDR sequences in the sequence (1), and (3) an amino acid sequence derived from the sequence (1) by the deletion, substitution or addition of one or several amino acids in the sequences of the framework regions outside of the CDR sequences, and a light chain comprising a light chain variable region consisting of any one of (4) the amino acid sequence as set forth in amino acid positions 21 to 129 of SEQ ID NO: 25 (h151D-L1) or SEQ ID NO: 27 (h151D-L4), (5) an amino acid sequence having at least 95% or higher homology to the sequences of the framework regions outside of the CDR sequences in the sequence (4), and (6) an amino acid sequence derived from the sequence (4) by the deletion, substitution or addition of one or several amino acids in the sequences of the framework regions outside of the CDR sequences.

Actual examples of the humanized antibody of the rat antibody 198D can include arbitrary combinations of a heavy chain comprising a heavy chain variable region consisting of any one of (1) the amino acid sequence as set forth in amino acid positions 20 to 139 of SEQ ID NO: 29 (h198D-H3) of the Sequence Listing, (2) an amino acid sequence having at least 95% or higher homology to the sequences of the framework regions outside of the CDR sequences in the sequence (1), and (3) an amino acid sequence derived from the sequence (1) by the deletion, substitution or addition of one or several amino acids in the sequences of the framework regions outside of the CDR sequences, and a light chain comprising a light chain variable region consisting of any one of (4) the amino acid sequence as set forth in amino acid positions 21 to 129 of SEQ ID NO: 31 (h198D-L4), (5) an amino acid sequence having at least 95% or higher homology to the sequences of the framework regions outside of the CDR sequences in the sequence (4), and (6) an amino acid sequence derived from the sequence (4) by the deletion, substitution or addition of one or several amino acids in the sequences of the framework regions outside of the CDR sequences.

Preferable examples of the heavy chain and the light chain of the humanized 151D antibody can include an antibody consisting of a heavy chain having a heavy chain variable region consisting of the amino acid sequence as set forth in amino acid positions 20 to 136 of SEQ ID NO: 21 and a light chain having a light chain variable region consisting of the amino acid sequence as set forth in amino acid positions 21 to 129 of SEQ ID NO: 25, and an antibody consisting of a heavy chain having a heavy chain variable region consisting of the amino acid sequence as set forth in amino acid positions 20 to 136 of SEQ ID NO: 23 and a light chain having a light chain variable region consisting of the amino acid sequence as set forth in amino acid positions 21 to 129 of SEQ ID NO: 27.

More preferable examples of the combination can include an antibody (h151D-H1L1) consisting of a heavy chain having the amino acid sequence as set forth in amino acid positions 20 to 466 of SEQ ID NO: 21 and a light chain having the amino acid sequence as set forth in amino acid positions 21 to 234 of SEQ ID NO: 25, and an antibody (h151D-H4L4) consisting of a heavy chain having the amino acid sequence as set forth in amino acid positions 20 to 466 of SEQ ID NO: 23 and a light chain having the amino acid sequence as set forth in amino acid positions 21 to 234 of SEQ ID NO: 27.

Preferable examples of the heavy chain and the light chain of the humanized 198D antibody can include an antibody consisting of a heavy chain having a heavy chain variable region consisting of the amino acid sequence as set forth in amino acid positions 20 to 139 of SEQ ID NO: 29 and a light chain having a light chain variable region consisting of the amino acid sequence as set forth in amino acid positions 21 to 129 of SEQ ID NO: 31.

More preferable examples of the combination can include an antibody (h198D-H3L4) consisting of a heavy chain having the amino acid sequence as set forth in amino acid positions 20 to 469 of SEQ ID NO: 29 and a light chain having the amino acid sequence as set forth in amino acid positions 21 to 234 of SEQ ID NO: 31.

An antibody having cytotoxic activity equivalent to each of the antibodies described above can be selected by combining sequences that exhibit high homology to the heavy chain amino acid sequence and the light chain amino acid sequence. Such homology is generally 80% or higher homology, preferably 85% or higher homology, more preferably 90% or higher homology, still more preferably 95% or higher homology, most preferably 99% or higher homology. Also, the antibody having cytotoxic activity equivalent to each of the antibodies described above can be selected by combining amino acid sequences derived from the amino acid sequence of the heavy chain or the light chain by the substitution, deletion or addition of one to several amino acid residues.

In the present specification, the term "several" means 1 to 10, 1 to 9, 1 to 8, 1 to 7, 1 to 6, 1 to 5, 1 to 4, 1 to 3, or 1 or 2.

The identity between two types of amino acid sequences can be determined using the default parameters of Blast algorithm version 2.2.2 (Altschul, Stephen F., Thomas L. Madden, Alejandro A. Schaffer, Jinghui Zhang, Zheng Zhang, Webb Miller, and David J. Lipman (1997), "Gapped BLAST and PSI-BLAST: a new generation of protein database search programs", Nucleic Acids Res. 25: 3389-3402). The Blast algorithm is also available by access to www.ncbi.nlm.nih.gov/blast on the Internet. The homology between a nucleotide sequence related to the antibody of the present invention and the nucleotide sequence of another antibody can also be determined by the Blast algorithm.

In the amino acid sequence of the heavy chain represented by SEQ ID NO: 21 or SEQ ID NO: 23 of the Sequence Listing related to the humanized 151D antibody, the amino acid sequence as set forth in positions 1 to 19 corresponds to a signal sequence, the amino acid sequence as set forth in positions 20 to 136 corresponds to a variable region, and an amino acid sequence consisting of the amino acid residues as set forth in positions 137 to 466 corresponds to a constant region.

In the amino acid sequence of the light chain represented by SEQ ID NO: 25 or SEQ ID NO: 27 of the Sequence Listing related to the humanized 151D antibody, the amino acid sequence as set forth in positions 1 to 20 corresponds to a signal sequence, the amino acid sequence as set forth in positions 21 to 129 corresponds to a variable region, and the amino acid sequence as set forth in positions 130 to 234 corresponds to a constant region.

In the amino acid sequence of the heavy chain represented by SEQ ID NO: 29 of the Sequence Listing related to the humanized 198D antibody, the amino acid sequence as set forth in positions 1 to 19 corresponds to a signal sequence, the amino acid sequence as set forth in positions 20 to 139 corresponds to a variable region, and the amino acid sequence as set forth in 140 to 469 corresponds to a constant region.

In the amino acid sequence of the light chain represented by SEQ ID NO: 31 of the Sequence Listing related to the humanized 198D antibody, the amino acid sequence as set forth in positions 1 to 20 corresponds to a signal sequence, the amino acid sequence as set forth in positions 21 to 129 corresponds to a variable region, and the amino acid sequence as set forth in 130 to 234 corresponds to a constant region.

The amino acid sequence of the heavy chain represented by SEQ ID NO: 21 or SEQ ID NO: 23 of the Sequence Listing related to the humanized 151D antibody is encoded by the nucleotide sequence represented by SEQ ID NO: 20 or SEQ ID NO: 22, respectively, of the Sequence Listing.

The amino acid sequence of the heavy chain represented by SEQ ID NO: 29 of the Sequence Listing related to the humanized 198D antibody is encoded by the nucleotide sequence represented by SEQ ID NO: 28 of the Sequence Listing.

The amino acid sequence of the light chain represented by SEQ ID NO: 25 or SEQ ID NO: 27 of the Sequence Listing related to the humanized 151D antibody is encoded by the nucleotide sequence represented by SEQ ID NO: 24 or SEQ ID NO: 26, respectively, of the Sequence Listing.

The amino acid sequence of the light chain represented by SEQ ID NO: 31 of the Sequence Listing related to the humanized 198D antibody is encoded by the nucleotide sequence represented by SEQ ID NO: 30 of the Sequence Listing.

One example of the anti-GARP antibody of the present invention can include a human antibody. The human antibody can be obtained by a method using human antibody-producing mice having human chromosome fragments containing human antibody heavy and light chain genes (see e.g., Nature Genetics (1997) 16, p. 133-143; Nucl. Acids Res. (1998) 26, p. 3447-3448; Animal Cell Technology: Basic and Applied Aspects vol. 10, p. 69-73 (Kitagawa, Y., Matsuda, T. and Iijima, S. eds.), Kluwer Academic Publishers, 1999; and Proc. Natl. Acad. Sci. USA (2000) 97, p. 722-727).

Also, the human antibody can be obtained by a method for obtaining a phage display-derived human antibody selected from a human antibody library (see e.g., Investigative Ophthalmology & Visual Science. (2002) 43 (7), p. 2301-2308; Briefings in Functional Genomics and Proteomics (2002), 1 (2), p. 189-203; Ophthalmology (2002) 109 (3), p. 427-431; and Nature Biotechnology (2005), 23, (9), p. 1105-1116).

The gene of a phage selected on the basis of its ability to bind to the antigen can be analyzed to determine DNA sequences encoding the variable regions of the human antibody binding to the antigen. Provided that the DNA sequence of scFv binding to the antigen is determined, an IgG expression vector having this sequence can be prepared by linking the DNA sequences of antibody constant regions thereto, and transferred to appropriate hosts, followed by expression to obtain the human antibody (WO92/01047, WO92/20791, WO93/06213, WO93/11236, WO93/19172, WO95/01438, WO95/15388, Annu. Rev. Immunol (1994) 12, p.433-455, Nature Biotechnology (2005) 23(9), p.1105-1116) .

Further examples of the human antibody of the present invention can include a human antibody that binds to the same epitope as that for the humanized 151D antibody or the humanized 198D antibody.

The antibody of the present invention also includes a modified variant of the antibody. The modified variant means a variant obtained by subjecting the antibody of the present invention to chemical or biological modification. The chemically modified variant includes, for example, a variant chemically modified by linking a chemical moiety to an amino acid skeleton, and a variant chemically modified with an N-linked or O-linked carbohydrate chain. The biologically modified variant includes, for example, a variant obtained by post-translational modification (e.g., N-linked or O-linked glycosylation, N-terminal or C-terminal processing, deamidation, isomerization of aspartic acid, and oxidation of methionine), and a variant with a methionine residue N-terminally added by expression using prokaryotic host cells. Further, an antibody labeled so as to permit detection or isolation of the antibody of the invention or the antigen, for example, an enzymatically labeled antibody, a fluorescently labeled antibody, and an affinity labeled antibody, are also included in the meaning of the modified variant. Such a modified variant of the antibody of the invention is useful for improving the original stability and blood retention of the antibody of the present invention, reducing antigenicity, detecting or isolating this antibody or the antigen, etc.

Antibody dependent cellular cytotoxicity activity can be potentiated by regulating the modification of a glycan attached to the antibody of the present invention (glycosylation, defucosylation, etc.). WO99/54342, WO00/61739, WO02/31140, WO2007/133855, etc. are known as techniques for regulating the glycan modification of antibodies, though the technique is not limited thereto. The antibody of the present invention also includes an antibody in which the glycan modification has been regulated.

The antibody that is used for the object of the present invention also includes a "functional fragment of the antibody" or an "antigen binding fragment of the antibody". The "functional fragment of the antibody" means an antibody fragment that exerts at least a portion of the functions exerted by the original antibody. Examples of the "functional fragment of the antibody" or the "antigen binding fragment of the antibody" can include, but are not limited to, Fab, $F(ab')_2$, scFv, Fab', and single chain immunoglobulins. Such a functional fragment of the antibody may be obtained by treating the full-length molecule of the antibody protein with an enzyme such as papain or pepsin, and in addition, may be a recombinant protein produced in appropriate host cells using a recombinant gene.

The antibody of the present invention also includes an antibody having two or more antigen binding sites. Specifically, such an antibody is an antibody capable of binding to two or more epitopes different from each other on one antigen or epitopes different from each other on two or more antigens, and contains a plurality of antigen binding fragments different from each other. Such a multispecific antibody includes, but is not limited to, an IgG type multispecific antibody, a multispecific antibody having two or more types of variable regions, for example, antibody fragments such as a tandem scFv, a single chain diabody, a diabody, and a triabody, and antibody fragments linked through covalent binding or noncovalent binding. The multispecific antibody may contain Fc.

The antibody of the present invention may be obtained from a culture supernatant by transforming eukaryotic cells with cDNAs encoding the heavy chain and the light chain, respectively, of the antibody of the present invention, preferably with vectors comprising the cDNAs, by a gene engineering technique, and culturing the transformed cells producing a recombinant human monoclonal antibody.

In the case of preparing the antibody by temporarily isolating an antibody gene and then transferring the gene to an appropriate host, the appropriate host can be used in combination with an expression vector. Specific examples of the antibody gene can include combinations of genes encoding the heavy chain sequences of the antibodies described in the present specification, and genes encoding the light chain sequences thereof. For the transformation of host cells, the heavy chain sequence gene and the light chain sequence gene may be inserted into the same expression vector or may be inserted into separate expression vectors.

In the case of using host eukaryotic cells, animal cells, plant cells, or eukaryotic microbes can be used. Particularly, examples of the animal cells can include mammalian cells, for example, COS cells (Gluzman, Y., Cell (1981) 23, p. 175-182, ATCC CRL-1650) which are monkey cells, mouse fibroblasts NIH3T3 (ATCC No. CRL-1658), and dihydrofolate reductase-deficient lines (Urlaub, G. and Chasin, L.A., Proc. Natl. Acad. Sci. U.S.A. (1980) 77, p. 4126-4220) of Chinese hamster ovary cells (CHO cells, ATCC CCL-61).

In the case of using prokaryotic cells, examples thereof can include *E. coli* and *Bacillus subtilis.*

The antibody gene of interest is transferred to these cells by transformation, and the transformed cells are cultured *in vitro* to obtain the antibody. Such culture may differ in yield depending on the sequence of the antibody. An antibody that is easy to produce as a medicament can be selected with its yield as an index from among antibodies having equivalent binding activity. Thus, the antibody of the present invention also includes an antibody obtainable by a method for producing the antibody, comprising the steps of: culturing the transformed host cells; and collecting the antibody of interest from the cultures thus obtained.

The heavy chain of an antibody produced by cultured mammalian cells is known to lack a carboxyl-terminal lysine residue (Journal of Chromatography A, 705: 129-134 (1995)). Also, the heavy chain of such an antibody is known to lack two carboxyl-terminal amino acid residues, glycine and lysine, and instead have an amidated proline residue positioned at the carboxy terminus (Analytical Biochemistry, 360: 75-83 (2007)). However, such deletion and modification in the heavy chain sequence does not influence the ability of the antibody to bind to the antigen and its effector functions (complement activation, antibody dependent cellular cytotoxic effects, etc.). Thus, the present invention also includes an antibody that has undergone the modification. Examples thereof can include a deletion variant lacking one or two amino acids at the carboxyl terminus of a heavy chain, and an amidated form of the deletion variant (e.g., a heavy chain having an amidated proline residue at the carboxyl-terminal site). However, the deletion variant lacking carboxyl-terminal amino acid(s) of a heavy chain of the antibody according to the present invention is not limited to the types described above as long as the deletion variant maintains the ability to bind to the antigen and the effector functions. Two heavy chains constituting the antibody according to the present invention may be heavy chains of any one type selected from the group consisting of the full-length heavy chain and the deletion variants described above, or may be a combination of heavy chains of any two types selected therefrom. The quantitative ratio of each deletion variant may be influenced by the type of cultured mammalian cells producing the antibody according to the present invention, and culture conditions. Examples of such a case can include the case where one carboxyl-terminal amino acid residue is deleted in both two heavy chains as main components of the antibody according to the present invention. Specifically, examples of such an antibody can include antibodies as described in the following (1) to (3):

(1) a heavy chain having the amino acid sequence as set forth in amino acid positions 20 to 465 of SEQ ID NO: 21 and a light chain having the amino acid sequence as set forth in amino acid positions 21 to 234 of SEQ ID NO: 25,
(2) a heavy chain having the amino acid sequence as set forth in amino acid positions 20 to 465 of SEQ ID NO: 23 and a light chain having the amino acid sequence as set forth in amino acid positions 21 to 234 of SEQ ID NO: 27, and
(3) a heavy chain having the amino acid sequence as set forth in amino acid positions 20 to 468 of SEQ ID NO: 29 and a light chain having the amino acid sequence as set forth in amino acid positions 21 to 234 of SEQ ID NO: 31.

Examples of the isotype of the antibody of the present invention can include IgG (IgG1, IgG2, IgG3, and IgG4) and can preferably include IgG1, IgG2 and IgG4.

The antibody of the present invention may have enhanced affinity for the antigen by multimerization. The antibody to be multimerized may be one type of antibody or a plurality of antibodies that recognize a plurality of epitopes of the same antigen. Examples of the method for multimerizing such an antibody can include the binding of two scFvs (single chain Fvs) to an IgG CH3 domain, the binding thereof to streptavidin, and the introduction of a helix-turn-helix motif.

The antibody of the present invention may be a polyclonal antibody. One example of the polyclonal antibody can include a mixture of plural types of antibodies differing in CDRs. An antibody obtained by culturing a mixture of cells producing different antibodies, followed by purification from the cultures can be used as such a polyclonal antibody (see WO2004/061104).

An antibody conjugated with any of various molecules such as polyethylene glycol (PEG) can also be used as the modified variant of the antibody.

The antibody of the present invention may further be any of conjugates formed by these antibodies with other drugs (immunoconjugates). Examples of such an antibody can include the antibody conjugated with a radioactive material or a compound having a pharmacological effect (Nature Biotechnology (2005) 23, p. 1137-1146), for example, indium (111In) capromab pendetide, technetium (99mTc) nofetumomab merpentan, indium (111In) ibritumomab, yttrium (90Y) ibritumomab, and iodine (1311) tositumomab.

2. Immunomodulator

In the present invention, the "immunomodulator" is a drug that activates antitumor immunity and includes an immune checkpoint inhibitor, an immune inducer, or an immunostimulant, etc.

In the present invention, the "immune checkpoint inhibitor" refers to a drug that inhibits an immunosuppressive system and activates antitumor immunity.

Examples of the immune checkpoint inhibitor used in the present invention can preferably include, but are not particularly limited to, anti-PD-1 antibodies, anti-PD-L1 antibodies, anti-PD-L2 antibodies, and anti-CTLA-4 antibodies and can more preferably include anti-PD-1 antibodies and anti-PD-Ll antibodies.

In the present invention, the "anti-PD-1 antibody" refers to an antibody having an effect of specifically binding to PD-1 (programmed cell death-1; CD279; PDCD1) and thereby reducing, inhibiting, and/or interfering with signal transduction resulting from the interaction between PD-1 and its binding partner PD-L1 or PD-L2. The anti-PD-1 antibody used in the present invention is not particularly limited as long as its efficacy and safety have been clinically confirmed. Examples thereof can preferably include nivolumab (WO2006/121168, etc.), pembrolizumab (WO2008/156712, etc.),

lambrolizumab, MK-3475, AMP-224, pidilizumab, and LOPD18, more preferably nivolumab and pembrolizumab. For example, a commercially available anti-PD-1 antibody for research (e.g., clone RMP1-14) may be used for the purpose of confirming a combinatorial effect with the anti-GARP antibody used in the present invention in preclinical research.

In the present invention, the "anti-PD-Ll antibody" refers to an antibody having an effect of specifically binding to PD-L1 (programmed cell death ligand 1; CD274; B7-H1) and thereby reducing, inhibiting, and/or interfering with signal transduction resulting from the interaction between PD-L1 and its binding partner PD-1 or B7.1 (CD80). The anti-PD-Ll antibody used in the present invention is not particularly limited as long as its efficacy and safety have been clinically confirmed. Examples thereof can preferably include atezolizumab (WO 2010/077634, etc.), durvalumab (WO 2011/066389, etc.), and avelumab (WO 2013/079174, etc.). For example, a commercially available anti-PD-Ll antibody for research (e.g., clone 10F.9G2) may be used for the purpose of confirming a combinatorial effect with the anti-GARP antibody used in the present invention in preclinical research.

In the present invention, the "anti-CTLA-4 antibody" refers to an antibody having an effect of specifically binding to CTLA-4 (cytotoxic T-lymphocyte-associated protein 4; CD152) and thereby reducing, inhibiting, and/or interfering with signal transduction resulting from the interaction between CTLA-4 and its binding partner B7.1 (CD80) or B7.2 (CD86). The anti-CTLA-4 antibody used in the present invention is not particularly limited as long as its efficacy and safety have been clinically confirmed. Examples thereof can preferably include ipilimumab (WO 2001/014424, etc.) and tremelimumab (WO 2000/037504, etc.). For example, a commercially available anti-CTLA-4 antibody for research (e.g., clone 9H10) may be used for the purpose of confirming a combinatorial effect with the anti-GARP antibody used in the present invention in preclinical research.

The immune checkpoint inhibitor used in the present invention includes a multispecific antibody. Such a multispecific molecule includes, but is not limited to, an IgG type multispecific molecule, a multispecific molecule having two or more types of variable regions, for example, antibody fragments such as a tandem scFv, a single chain diabody, a diabody, and a triabody, and antibody fragments linked through covalent binding or noncovalent binding. The multispecific molecule may contain Fc. Specifically, examples thereof can include, but are not limited to, a multispecific antibody comprising two or more antibodies or antigen binding fragments thereof selected from the group consisting of an anti-PD-1 antibody or an antigen binding fragment thereof, an anti-PD-Ll antibody or an antigen binding fragment thereof, an anti-PD-L2 antibody or an antigen binding fragment thereof, and an anti-CTLA-4 antibody or an antigen binding fragment thereof, more preferably a bispecific antibody comprising an anti-PD-1 antibody or an antigen binding fragment thereof and an anti-CTLA-4 antibody or an antigen binding fragment thereof.

In the present invention, examples of the "immune inducer" can preferably include, but are not particularly limited to, radiotherapy (radiation irradiation), chemoradiotherapy, and chemotherapeutics. In the present invention, the "chemotherapeutic" refers to a drug that evokes antitumor immunity such as removal of Tregs or decrease in the number of myeloid-derived suppressor cells (MDSCs). Examples thereof include, but are not limited to, sunitinib, cyclophosphamide, oxaliplatin, cisplatin, doxorubicin, mitoxantrone, daunorubicin, methotrexate, gemcitabine, docetaxel, paclitaxel, vincristine, carboplatin, vinorelbine, erlotinib, imatinib, nilotinib, dasatinib, sorafenib, bortezomib, ABT-737, 5-FU, temozolomide, and dacarbazine (Nat Rev Drug Discov. 2012 Feb 3; 11(3): 215-33, Front Immunol. 2019 Jul 17; 10: 1654, Clin Cancer Res 2009; 15: 2148-2157, Cancer Immunol Immunother (2007) 56: 641-648).

In the present invention, examples of the "immunostimulant" can preferably include, but are not particularly limited to, agonist antibodies and adjuvants.

3. Medicament

Hereinafter, a pharmaceutical composition and a treatment method, wherein the anti-GARP antibody according to the present invention and an immunomodulator are administered in combination, and a pharmaceutical composition and a treatment method, wherein the anti-GARP antibody according to the present invention is contained and the pharmaceutical composition and the treatment method are used in the treatment of a disease that is improved by an effect of activating antitumor immunity, will be described.

In the pharmaceutical composition and the treatment method of the present invention, the anti-GARP antibody and the immunomodulator may be contained as active ingredients in separate preparations, or the anti-GARP antibody may be combined with radiotherapy or chemoradiotherapy using the immunomodulator, and the anti-GARP antibody and the immunomodulator may be administered at the same time or at different times. The anti-GARP antibody and the immunomodulator may be contained as active ingredients in a single preparation and administered. Alternatively, the anti-GARP antibody according to the present invention may be contained as one of the active ingredients in a single preparation and administered for the treatment of a disease that is improved by an effect of activating antitumor immunity.

In the present invention, the phrase "increasing an effect of an antibody" means that an antitumor effect is potentiated as compared with the case of administering the antibody as a single agent.

In the present invention, the phrase "increasing an effect of an immunomodulator" means that an antitumor immunity activating effect is potentiated as compared with the case of administering the immunomodulator as a single agent.

The pharmaceutical composition and the treatment method of the present invention can be used for the treatment of a cancer and, preferably, can be used for the treatment of at least one disease selected from the group consisting of lung cancer, kidney cancer, urothelial cancer, large intestine cancer, prostatic cancer, glioblastoma multiforme, ovary cancer, pancreatic cancer, breast cancer, melanoma, liver cancer, bladder cancer, stomach cancer, esophageal cancer, head and neck cancer, blood cancer, skin cancer, thyroid gland cancer, biliary tract cancer, salivary gland cancer, small intestine cancer, adrenal cancer, ovary cancer, uterine cervical cancer, endometrial cancer, uterine sarcoma, thymoma, mesothelioma, sarcoma and their metastatic forms. More preferably, the pharmaceutical composition and the treatment method can be used for the treatment of at least one disease selected from the group consisting of head and neck cancer, stomach cancer, esophageal cancer and their metastatic forms.

The pharmaceutical composition and the treatment method of the present invention can be selected and used as a drug or a treatment method for medication which is a primary treatment for cancers, and as a result, can delay the growth of cancer cells, inhibit their proliferation, and further destroy the cancer cells. These effects can allow cancer patients to be free from symptoms caused by cancers or achieve improvement in QOL of cancer patients and attain a therapeutic effect by sustaining the lives of the cancer patients. The pharmaceutical composition and the treatment method, even if falling short of destroying cancer cells, can achieve higher QOL of cancer patients while achieving longer-term survival, by inhibiting or controlling the proliferation of cancer cells.

The pharmaceutical composition and the treatment method of the present invention can be used as a drug or a treatment method alone in such medication, and in addition, can also be used as a drug or a treatment method in combination with an additional therapy in adjuvant therapy and can be combined with surgical operation, radiotherapy, hormone therapy, or the like. Furthermore, the pharmaceutical composition and the treatment method may be used as a drug for medication in neoadjuvant therapy.

In addition to the therapeutic use as described above, the pharmaceutical composition and the treatment method of the present invention can also be expected to have a prophylactic effect such as the inhibition and destruction of small metastatic cancer cells. For example, an effect of inhibiting and destroying cancer cells in a body fluid in the course of metastasis or an effect of, for example, inhibiting and destroying small cancer cells immediately after implantation in any tissue can be expected. Thus, the inhibition of cancer metastasis or a prophylactic effect can be expected, particularly, after surgical removal of a cancer.

The pharmaceutical composition and the treatment method of the present invention can be expected to exert a therapeutic effect by application as systemic therapy to patients as well as by local application to cancer tissues.

The pharmaceutical composition and the treatment method of the present invention can preferably be used for a mammal and can more preferably be used for a human.

The pharmaceutical composition of the present invention can be administered as a pharmaceutical composition containing one or more pharmaceutically compatible ingredients. A substance for use in the pharmaceutical composition of the present invention can be appropriately selected and applied from pharmaceutical additives and others usually used in the art, according to a dose or an administration concentration. The pharmaceutical composition typically contains, for example, one or more pharmaceutical carriers (e.g., a sterilized liquid). In this context, the liquid includes, for example, water and an oil (petroleum or an oil of animal origin, plant origin, or synthetic origin). The oil may be, for example, peanut oil, soybean oil, mineral oil, or sesame oil. Water is a more typical carrier when the pharmaceutical composition is intravenously administered. An aqueous salt solution, an aqueous dextrose solution and an aqueous glycerol solution may also be used as liquid carriers, particularly, for injectable solutions. A suitable pharmaceutical excipient can be appropriately selected from those known in the art. The composition may also contain, if desired, a small amount of a wetting agent or an emulsifier, or a pH buffering agent. Examples of suitable pharmaceutical carriers are described in "Remington's Pharmaceutical Sciences" by E.W. Martin. The prescription thereof corresponds to the form of administration.

Various delivery systems are known in the art and can be used for administering the pharmaceutical composition of the present invention. Examples of the introduction route can include, but are not limited to, intracutaneous, intramuscular, intraperitoneal, intravenous, and subcutaneous routes. The administration may be based on, for example, infusion or bolus injection. According to a particularly preferable embodiment, the anti-GARP antibody and immunomodulator used in the present invention are administered by infusion. Parenteral administration is a preferable administration route.

In a typical embodiment, the pharmaceutical composition is prescribed as a pharmaceutical composition suitable for intravenous administration to humans according to conventional procedures. The composition for intravenous administration is typically a solution in a sterile and isotonic aqueous buffer solution. If necessary, the pharmaceutical composition may also contain a solubilizing agent and a local anesthetic for alleviating pain at an injection site (e.g., lignocaine). In general, these ingredients are provided, for example, either separately or together as a mixture in a unit dosage form, as a dried or freeze-dried powder or an anhydrous concentrate in a container sealed by hermetical sealing in an ampoule or a sachet that indicates the amount of the active agent. When the pharmaceutical composition is in the form of administration by infusion, it can be administered, for example, from an infusion bottle containing water or saline of sterile pharmaceutical grade. When the pharmaceutical composition is administered by injection, an ampoule of injectable sterile water or saline can be provided such that, for example, the ingredients are mixed before administration.

The pharmaceutical composition and the treatment method of the present invention may contain a therapeutic agent for a cancer other than the anti-GARP antibody according to the present invention and the immunomodulator. The pharmaceutical composition and the treatment method of the present invention may be combined, for administration, with an additional therapeutic agent for a cancer and can thereby potentiate an antitumor effect. The additional therapeutic agent for a cancer that is used for such a purpose may be administered to an individual concurrently with, separately from, or continuously with the pharmaceutical composition of the present invention, or their administration may be staggered at intervals. Examples of such a therapeutic agent for a cancer can include 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastine, vinorelbine, everolimus, tanespimycin, bevacizumab, oxaliplatin, lapatinib, trastuzumab emtansine (T-DM1) and drugs described in WO 2003/038043, and furthermore, LH-RH analogs (leuprorelin, goserelin, etc.), estramustine phosphate, estrogen antagonists (tamoxifen, raloxifene, etc.), and aromatase inhibitors (anastrozole, letrozole, exemestane, etc.), though the therapeutic agent is not limited thereto as long as the drug has antitumor activity.

Such a pharmaceutical composition can be formulated into a freeze-dried preparation or a liquid preparation as a preparation having a selected composition and necessary purity. For formulation into a freeze-dried preparation, the preparation may contain an appropriate pharmaceutical additive that is used in the art. Likewise, a liquid agent can also be formulated as a liquid preparation containing various pharmaceutical additives that are used in the art.

The antitumor effects of the pharmaceutical composition and the treatment method of the present invention can be confirmed by measuring immunostimulatory activity or tumor cell proliferation inhibitory activity, etc. The antitumor effects may also be confirmed by transplanting tumors to test animals to prepare models, which are then subjected to the therapeutic agent or the treatment method of the present invention. The antitumor effects of the therapeutic agent and the treatment method of the present invention can be verified using a surrogate antibody when the anti-GARP antibody of the present invention does not bind to GARP in the model animals. It is desirable that the surrogate antibody should have the same or similar binding activity or functional characteristics as that of the anti-GARP antibody of the present invention.

Further, the effects can be predicted using an ex *vivo* evaluation system such as CANscript (Mitra RxDx, Inc.) (Nat Commun. 2015 Feb 27 (6); 6169).

The immunostimulatory activity and the antitumor effects can be measured by methods described in WO2017/051888.

The antitumor effects of the pharmaceutical composition and the treatment method of the present invention can also be confirmed in clinical trials. Specifically, cancer patients are subjected to the therapeutic agent or the treatment method of the present invention, and the antitumor effect can be confirmed by a response evaluation criteria in solid tumors (RECIST) evaluation method, a WHO evaluation method, a Macdonald evaluation method, body weight measurement, or other approaches and can be determined on the basis of an index such as complete response (CR), partial response (PR), progressive disease (PD), objective response rate (ORR), duration of response (DoR), progression-free survival (PFS), or overall survival (OS).

The composition and concentration of the pharmaceutical composition vary depending on the administration method. The anti-GARP antibody and the immunomodulator contained in the pharmaceutical composition of the present invention, in terms of affinity for the antigen, i.e., a dissociation constant (Kd value) for the antigen, can exert drug efficacy even at a smaller dose when the affinity is higher (the Kd value is lower). Thus, the doses of the anti-GARP antibody and the immunomodulator may be set on the basis of the degrees of affinity for their antigens. For the administration of the anti-GARP antibody according to the present invention and the immunomodulator to a human, for example, approximately 0.001 to 100 mg/kg can be administered once or a plurality of times at intervals of once every 1 to 180 days.

Examples of the method for administering the anti-GARP antibody of the present invention can include a method of administering 0.01 mg/kg to 100 mg/kg once or two or more times. Examples of the dose can include 0.01 mg/kg, 0.03 mg/kg, 0.1 mg/kg, 0.3 mg/kg, 1 mg/kg, 3 mg/kg, 5 mg/kg, 10 mg/kg, 30.0 mg/kg, and 100 mg/kg. The administration may be performed once a week (q1w), once every two weeks (q2w), once every three weeks (q3w), or once every four weeks (q4w). Preferable examples of the administration method include a method of administering 0.3 mg/kg, 1 mg/kg, 3 mg/kg, or 10 mg/kg of the antibody of the present invention once or two or more times at intervals of once every three weeks.

The immune checkpoint inhibitor used in the present invention can be intravenously administered (e.g., by intravenous drip infusion), for example, at approximately 1 to 20 mg/kg (body weight) per dose or at approximately 80 to 1500 mg per dose over approximately 30 minutes, approximately 60 minutes, approximately 90 minutes, approximately 30 minutes or longer, or approximately 60 minutes or longer at 2- to 6-week intervals. Examples of the dose per administration based on body weight include 1 mg/kg, 2 mg/kg, 3 mg/kg, 4 mg/kg, 5 mg/kg, 6 mg/kg, 7 mg/kg, 8 mg/kg, 9 mg/kg, 10 mg/kg and 20 mg/kg. Examples of the dose per administration include 80 mg, 200 mg, 240 mg, 250 mg, 280 mg, 300 mg, 320 mg, 350 mg, 360 mg, 400 mg, 420 mg, 450 mg, 480 mg, 500 mg, 540 mg, 560 mg, 600 mg, 640 mg, 700 mg, 720 mg, 750 mg, 800 mg, 840 mg, 900 mg, 1000 mg, 1080 mg, 1100 mg, 1120 mg, 1200 mg, and 1500 mg. Examples of the administration interval include 2 weeks, 3 weeks, 4 weeks, and 6 weeks. Examples of the time of one administration include approximately 30 minutes, approximately 60 minutes, approximately 90 minutes, approximately 30 minutes or longer, and approximately 60 minutes or longer.

These dosages and administrations are given for illustration and not limited thereto.

When the anti-PD-1 antibody used in the present invention is nivolumab, examples of the administration method include, but are not limited to, the following dosages and administrations. For example, 2 mg/kg (body weight) of nivolumab per dose is administered by intravenous drip infusion at 3-week intervals, or 3 mg/kg (body weight) of nivolumab per dose is administered by intravenous drip infusion at 2-week intervals.

As another dosage and administration, 1 mg/kg (body weight) of nivolumab per dose is administered by intravenous drip infusion four times at 3-week intervals, and then, 3 mg/kg (body weight) of nivolumab per dose is administered by intravenous drip infusion at 2-week intervals.

As an alternative dosage and administration, 3 mg/kg (body weight) of nivolumab per dose is administered by intravenous drip infusion four times at 3-week intervals, and then, 3 mg/kg (body weight) of nivolumab per dose is administered by intravenous drip infusion at 2-week intervals.

As an alternative dosage and administration, for example, 240 mg of nivolumab per dose is administered by intravenous drip infusion at 2-week intervals, or 480 mg of nivolumab per dose is administered by intravenous drip infusion at 4-week intervals.

As an alternative dosage and administration, for example, 80 mg of nivolumab per dose is administered by intravenous drip infusion four times at 3-week intervals, and then, 240 mg of nivolumab per dose is administered by intravenous drip infusion at 2-week intervals, or 480 mg of nivolumab per dose is administered by intravenous drip infusion at 4-week intervals.

As an alternative dosage and administration, for example, 240 mg of nivolumab per dose is administered by intravenous drip infusion four times at 3-week intervals, and then, 240 mg of nivolumab per dose is administered by intravenous drip infusion at 2-week intervals, or 480 mg of nivolumab per dose is administered by intravenous drip infusion at 4-week intervals.

Nivolumab may be administered by intravenous drip infusion over 30 minutes or longer.

For postoperative adjunctive therapy, the administration period may be, for example, up to 12 months.

When the anti-PD-1 antibody used in the present invention is pembrolizumab, examples of the administration method include, but are not limited to, the following dosages and administrations. For example, 2 mg/kg (body weight) of pembrolizumab per dose is administered by intravenous drip infusion at 3-week intervals.

As another dosage and administration, 10 mg/kg (body weight) of pembrolizumab per dose is administered by intravenous drip infusion at 2-week intervals.

As an alternative dosage and administration, 10 mg/kg (body weight) of pembrolizumab per dose is administered by intravenous drip infusion at 3-week intervals.

As an alternative dosage and administration, for example, 200 mg of pembrolizumab per dose is administered by intravenous drip infusion at 3-week intervals, or 400 mg of pembrolizumab per dose is administered by intravenous drip infusion at 6-week intervals. For example, 200 mg of pembrolizumab per dose is administered by intravenous drip infusion at 3-week intervals, and, 200 mg of pembrolizumab per dose is continuingly administered by intravenous drip infusion at 3-week intervals.

Pembrolizumab may be administered by intravenous drip infusion, for example, over 30 minutes. Thus, as an alternative dosage and administration, for example, 200 mg of pembrolizumab per dose is administered by intravenous drip infusion over 30 minutes at 3-week intervals, or 400 mg of pembrolizumab per dose is administered by intravenous drip infusion over 30 minutes at 6-week intervals.

For postoperative adjunctive therapy, the administration period may be, for example, up to 12 months.

When the anti-PD-LI antibody used in the present invention is atezolizumab, examples of the administration method include, but are not limited to, the following dosages and administrations. For example, 1200 mg of atezolizumab per dose is administered by intravenous drip infusion at 3-week intervals.

As another dosage and administration, for example, 840 mg of atezolizumab per dose is administered by intravenous drip infusion at 2-week intervals.

As an alternative dosage and administration, for example, 1200 mg of atezolizumab per dose is administered by intravenous drip infusion at 3-week intervals, and then, 1200 mg of atezolizumab per dose is administered by intravenous drip infusion at 3-week intervals.

Atezolizumab may be administered by intravenous drip infusion, for example, over 60 minutes. Thus, for example, 1200 mg of atezolizumab per dose is administered by intravenous drip infusion over 60 minutes at 3-week intervals, or 840 mg of atezolizumab per dose is administered by intravenous drip infusion over 60 minutes at 2-week intervals.

Provided that the tolerance of initial administration is favorable, the time of the second or later administration may be shortened to up to 30 minutes.

When the anti-PD-LI antibody used in the present invention is durvalumab, examples of the administration method include, but are not limited to, the following dosages and administrations. For example, 10 mg/kg (body weight) of durvalumab per dose is administered by intravenous drip infusion at 2-week intervals.

As another dosage and administration, for example, 1500 mg of durvalumab per dose is administered by intravenous drip infusion four times at 3-week intervals, and then, 1500 mg of durvalumab per dose is administered by intravenous drip infusion at 4-week intervals.

Durvalumab may be administered by intravenous drip infusion over 60 minutes or longer. Thus, as an alternative dosage and administration, for example, 10 mg/kg (body weight) of durvalumab per dose is administered by intravenous drip infusion over 60 minutes or longer at 2-week intervals. As an alternative dosage and administration, for example, 1500 mg of durvalumab per dose is administered by intravenous drip infusion over 60 minutes or longer four times at 3-week intervals, and then, 1500 mg of durvalumab per dose is administered by intravenous drip infusion over 60 minutes or longer at 4-week intervals.

The single dose for a body weight of 30 kg or lower may be 20 mg/kg (body weight).

When the anti-PD-LI antibody used in the present invention is avelumab, examples of the administration method include, but are not limited to, the following dosages and administrations. For example, 10 mg/kg (body weight) of avelumab per dose is administered by intravenous drip infusion at 2-week intervals.

Avelumab may be administered by intravenous drip infusion over 1 hour or longer. Thus, as an alternative dosage and administration, for example, 10 mg/kg (body weight) of avelumab per dose is administered by intravenous drip infusion over 1 hour or longer at 2-week intervals.

When the anti-CTLA-4 antibody used in the present invention is ipilimumab, examples of the administration method include, but are not limited to, the following dosages and administrations. For example, 3 mg/kg (body weight) of ipilimumab per dose is administered by intravenous drip infusion four times at 3-week intervals.

As another alternative dosage and administration, for example, 1 mg/kg (body weight) of ipilimumab per dose is administered by intravenous drip infusion four times at 3-week intervals.

Ipilimumab may be administered by intravenous drip infusion over 30 minutes or 90 minutes.

The present invention also includes use of the antibody of the present invention for preparing a pharmaceutical composition for cancer treatment, use of the antibody of the present invention for cancer treatment, and a kit for cancer treatment comprising the antibody of the present invention.

## Examples

Hereinafter, the present invention will be specifically described with reference to examples. However, the present invention is not limited by these examples. These examples are by no means interpreted in a limited way.

Reference Example 1. Preparation of anti-GARP antibody

Anti-GARP antibodies h151D_H1L1, h151D_H4L4, and h198D_H3L4 were prepared according to the production method described in WO2017/051888.

Example 1. Combinatorial effect of anti-GARP antibody and anti-PD-1 antibody on activation of Teff cell proliferation

### 1)-1 Preparation of Treg, Teff and accessory cells

CD4-positive T cells were isolated from peripheral blood mononuclear cells (PBMCs) of healthy donors and then stained by the addition of fluorescently labeled anti-CD4 antibody and anti-CD25 antibody. CD4-positive and CD25-negative (Teff) cells and CD4-positive and CD25-strongly positive (Treg) cells were collected by sorting using flow cytometry.

After removal of CD3-positive cells from the PBMCs, accessory cells were prepared by irradiation with X rays at an exposure dose of 0.35 C/kg.

### 1)-2 Co-culture assay

Teff cells (2000 cells/well) and accessory cells (20000 cells/well) were mixed. Then, Treg cells were further added at 1000, 500, 250, or 125 cells/well. The anti-GARP antibody (h151D_H1L1) and the anti-PD-1 antibody (nivolumab) were added thereto at 1 μg/mL each (final concentration) in the presence of an anti-CD3 antibody and an anti-CD28 antibody (1 nM each), and the cells were cultured at 37°C for 5 days under conditions of 5% $CO_2$. Then, [$^3$H]-thymidine was added thereto (final concentration: 18.5 kBq/mL), and the culture was further continued for 20 hours. After recovery of the cells, the intracellular uptake value of [$^3$H]-thymidine in each well was measured as a corrected count per minute (CCPM) using a scintillation counter.

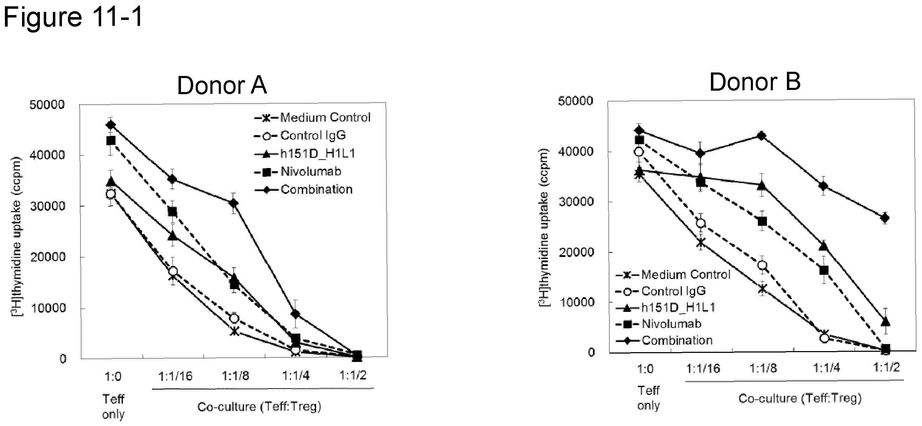

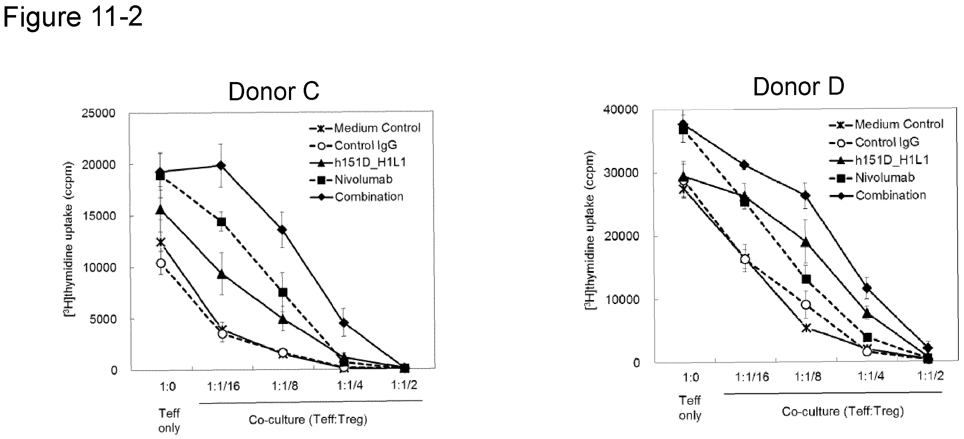

Figures 11-1 and 11-2 show means and standard deviations (6 wells/ group) of the amounts of [$^3$H]-thymidine taken up into the cells under the respective culture conditions for each of four donors. The amount of [$^3$H]-thymidine taken up into the cells decreased with the increase in the ratio of Treg cells to Teff cells, showing that Treg cells inhibited the proliferation of Teff cells. Furthermore, the amount of [$^3$H]-thymidine taken up into the cells was shown to be increased in the h151D_H1L1 treatment group and the nivolumab treatment group and further increased in the combination group. Thus, the combinatorial effect of the anti-GARP antibody and the anti-PD-1 antibody was confirmed.

1)-3 Calculation of relative activity and statistical analysis

A relative average count to the average count of Teff:Treg = 1:0 (no addition of Treg cells) in a medium control group obtained in 1)-2 was calculated for each donor. When relative average counts obtained in culture at ratios between Teff cells and Treg cells (Teff:Treg) of 1:0, 1:1/16, 1:1/8, 1:1/4, and 1:1/2 were defined as A, B, C, D, and E, respectively, relative AUC was determined according to the following expression.

$$AUC = (A + B \times 2 + C \times 2 + D \times 2 + E) / 2$$

Statistical analysis was carried out by the parametric Dunnett's test (SAS System Release 9.2, SAS Institute Inc.) using the obtained relative AUC values of the four donors as to the h151D_H1L1 treatment group and the nivolumab treatment group vs. the medium control group as the subject group and as to the h151D_H1L1 treatment group and the nivolumab treatment group vs. the combination group as the subject group.

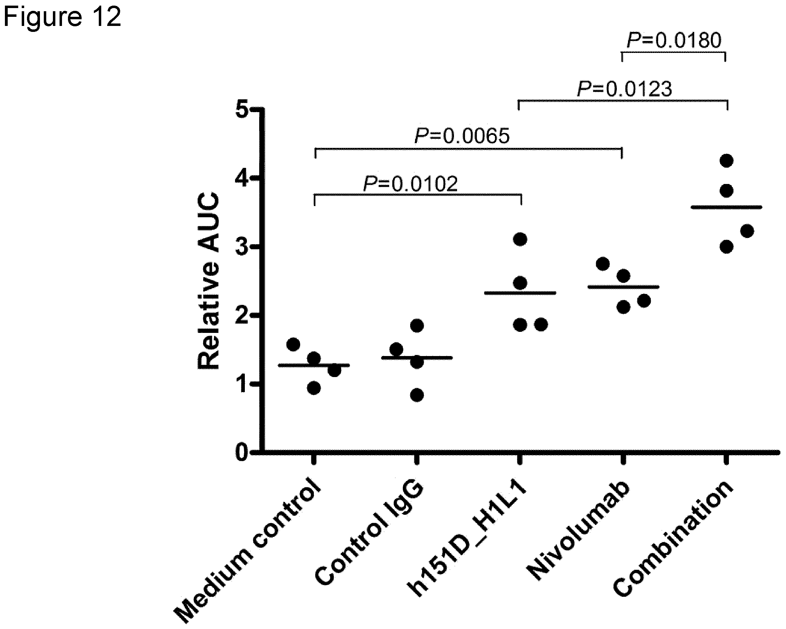

Figure 12 shows the relative AUC values of the four donors and average values thereof. A significant increase in relative AUC value, which is an index for activation of Teff cell proliferation, was found in the h151D_H1L1 treatment group (P = 0.0102) and the nivolumab treatment group (P = 0.0065) compared with the medium control group. Furthermore, a significant increase therein was found in the combination group compared with the h151D_H1L1 treatment group (P = 0.0123) and the nivolumab treatment group (P = 0.0180). The average relative AUC of the control IgG treatment group was 1.08 times that of the medium control group.

Example 2. Prediction of clinical efficacy using CANscript(R) when anti-GARP antibody and anti-PD-1 antibody are combined

CANscript(R) (Mitra RxDx, Inc. (hereinafter, referred to as Mitra)) is an *ex-vivo* culture model using cancer patient-derived cancer tissues and is for a co-culture test of the cancer patient-derived cancer tissues (which maintain a cancer microenvironmental structure and contain stromal cells, immune cells, and cancer cells) and serum and PBMCs derived from the same patients thereas (Nat Commun. 2015 Feb 27 (6); 6169). The response of the cancer tissues to a drug is quantified on the basis of pathological tissue images and metabolic activity after co-culture for 3 days. Specifically, cell proliferation and apoptosis induction are evaluated by the immunochemical staining of Ki67 and activated caspase-3, and change in cell morphology is evaluated by H&E (hematoxylin-eosin) staining. Further, time-dependent quantification is carried out by the evaluation of culture supernatants using commercially available metabolic activity and cell survival rate determination assay kits. All of these evaluation results are scored (M-score) using a learning algorithm (SVMpAUC algorithm) developed by Mitra. SVMpAUC is capable of predicting the clinical efficacy of each drug by the comparative learning of actual drug responses clinically obtained in the same patients with quantification results obtained from CANscript. Data from 4000 or more cases have been analyzed so far, and a probability (positive predictive value) that indicates CR (complete response) or PR (partial response) clinically is 87% for an M-score of higher than 25.

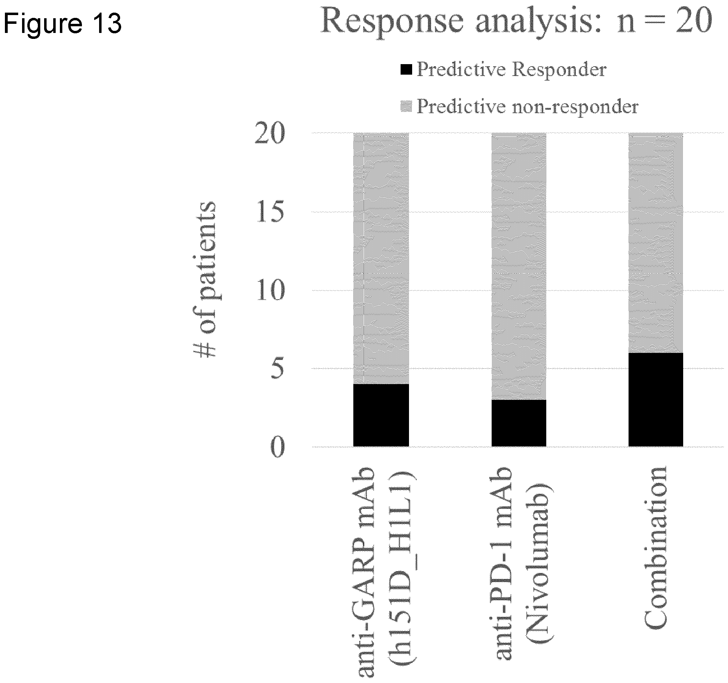

The clinical efficacies of the anti-GARP antibody (h151D_H1L1), the anti-PD-1 antibody (nivolumab), and h151D_H1L1 combined with nivolumab were evaluated and predicted by the CANscript assay using samples obtained from 20 head and neck squamous cell carcinoma patients. Before the start of co-culture, the proportion and composition of immune cells that infiltrated into cancer tissues were analyzed by RNA expression analysis using a NanoString 10360 panel. As a result, the immunological profiles differed considerably among the patients. When h151D_H1L1 and nivolumab each acted alone on these diverse samples derived from the head and neck squamous cell carcinoma patients, the proportions of patients' samples that exhibited an M-score of larger than 25 were 20% (4/20) and 15% (3/20), respectively. By contrast, when h151D_H1L1 and nivolumab were combined, the proportion was increased to 30% (6/20). Thus, a combinatorial effect of the anti-GARP antibody and the anti-PD-1 antibody on head and neck squamous cell carcinoma was predicted (Figure 13).

Example 3: Antitumor test and tumor infiltrating leucocyte (TIL) analysis using CT26.WT subcutaneously transplanted mouse model

3)-1 Combinatorial effect of anti-GARP surrogate antibody and anti-PD-1 surrogate antibody in antitumor test

A mouse large intestine cancer cell line CT26.WT purchased from American Type Culture Collection (ATCC) was suspended in physiological saline and subcutaneously transplanted at 2.0 $\times$ $10^5$ cells into the right abdominal region of female CD2F1 mice (Charles River Laboratories Japan, Inc.) (day 0). On day 3 and day 6, an anti-mouse PD-1 antibody (5 mg/kg) was administered thereto. On day 9, the mice were randomly grouped (n = 9 for each group). On day 9 and day 16, an anti-mouse GARP antibody (1 mg/kg) and an anti-mouse PD-1 antibody (5 mg/kg) were administered to their respective single agent administration groups and a combination group. A control group underwent no drug treatment on day 9 and day 16. Each antibody was diluted with D-PBS, and the dilution was administered in an amount of 10 mL/kg via the tail vein. The major axis and minor axis of each tumor were measured twice a week using electronic digital calipers (Mitutoyo Corp.), and an estimated tumor volume was calculated according to the following calculation expression.

[Expression 1]

$$\text{Estimated tumor volume (mm}^3\text{)} = 1 / 2 \times \text{Major axis (mm)} \times [\text{Minor axis (mm)}]^2$$

The anti-mouse PD-1 antibody/anti-mouse GARP antibody combination group exhibited a significant antitumor effect on the estimated tumor volume (mean $\pm$ standard deviation) on day 20, as compared with the anti-mouse PD-1 antibody single administration group.

3)-2 Combinatorial effect of anti-GARP surrogate antibody and anti-PD-1 surrogate antibody on TIL

CT26.WT was suspended in physiological saline and subcutaneously transplanted at 2.0 $\times$ $10^5$ cells into the right abdominal region of female CD2F1 mice (day 0). On day 6, the mice were randomly grouped (n = 10 for each group). On day 6 and day 10, an anti-mouse GARP antibody (1 mg/kg) and an anti-mouse PD-1 antibody (5 mg/kg) were administered to their respective single agent administration groups and a combination group. A control group underwent no drug treatment. Each antibody was diluted with D-PBS, and the dilution was administered in an amount of 10 mL/kg via the tail vein. On day 14, each tumor was excised and ground to obtain TILs. The ratio (%) of inducible T-cell co-stimulator (ICOS)-positive cells within the population of CD8-positive T cells was determined as an index for immune activation against cancer by flow cytometric analysis. A significant increase in the ratio of ICOS-positive CD8-positive T cells was found in the combination group compared with each single agent group. In this respect, the ratio (mean $\pm$ standard deviation) of GARP-positive Tregs (GARP-positive, FOXP3-positive and CD4-positive T cells) in CD4-positive T cells was confirmed to be significantly decreased in the anti-mouse GARP antibody administration group and significantly increased in the anti-mouse PD-1 antibody administration group. This ratio was significantly decreased in the combination group compared with the control group.

3)-3 Combinatorial effect of anti-GARP surrogate antibody and anti-PD-LI surrogate antibody in antitumor test

CT26.WT was suspended in physiological saline and subcutaneously transplanted at 2.0 $\times$ $10^5$ cells into the right abdominal region of female CD2F1 mice (day 0). On day 6, the mice were randomly grouped (n = 10 for each group). On day 6 and day 11, an anti-mouse GARP antibody (1 mg/kg) and an anti-mouse PD-L1 antibody (10 mg/kg) were administered to their respective single agent administration groups and a combination group. A control group underwent no drug treatment. Each antibody was diluted with D-PBS, and the dilution was administered in an amount of 10 mL/kg via the tail vein. The major axis and minor axis of each tumor were measured twice a week using electronic digital calipers, and an estimated tumor volume was calculated according to the following calculation expression.

[Expression 2]

$$\text{Estimated tumor volume (mm}^3\text{)} = 1 / 2 \times \text{Major axis (mm)} \times [\text{Minor axis (mm)}]^2$$

Reduction in estimated tumor volume was confirmed on day 18 in the combination group compared with the respective single agent groups of the anti-GARP antibody and the anti-PD-Ll antibody.

Example 4: Antitumor test using MC38 subcutaneously transplanted mouse model

4)-1 Combinatorial effect of anti-GARP surrogate antibody and anti-PD-1 surrogate antibody

A mouse large intestine cancer cell line MC38 obtained from National Cancer Institute (NCI) was suspended in physiological saline and subcutaneously transplanted at $5.0 \times 10^5$ cells into the right abdominal region of female C57BL/6 mice (Charles River Laboratories Japan, Inc.) (day 0). On day 4, the mice were randomly grouped (n = 10 for each group). On day 4 and day 10, an anti-mouse GARP antibody (1 mg/kg) and an anti-mouse PD-1 antibody (1 mg/kg) were administered to their respective single agent administration groups and a combination group. A control group underwent no drug treatment. Each antibody was diluted with D-PBS, and the dilution was administered in an amount of 10 mL/kg via the tail vein. The major axis and minor axis of each tumor were measured twice a week using electronic digital calipers, and an estimated tumor volume was calculated according to the following calculation expression.

[Expression 3]

$$\text{Estimated tumor volume (mm}^3\text{)} = 1 / 2 \times \text{Major axis (mm)} \times [\text{Minor axis (mm)}]^2$$

A reduction in estimated tumor volume was confirmed on day 18 in the combination group compared with the respective single agent groups of the anti-GARP antibody and the anti-PD-1 antibody.

4)-2 Combinatorial effect of anti-GARP surrogate antibody and anti-CTLA-4 surrogate antibody

MC38 was suspended in physiological saline and subcutaneously transplanted at $5.0 \times 10^5$ cells into the right abdominal region of female C57BL/6 mice (day 0). On day 4, the mice were randomly grouped (n = 10 for each group). On day 4 and day 11, an anti-mouse GARP antibody (1 mg/kg) and on day 4, day 7, day 11, and day 14, an anti-mouse CTLA-4 antibody (10 mg/kg) were administered to their respective single agent administration groups and a combination group. Each antibody was diluted with D-PBS, and the dilution was administered in an amount of 10 mL/kg via the tail vein. A control group underwent no drug treatment. The major axis and minor axis of each tumor were measured twice a week using electronic digital calipers, and an estimated tumor volume was calculated according to the following calculation expression.

[Expression 4]

$$\text{Estimated tumor volume (mm}^3\text{)} = 1 / 2 \times \text{Major axis (mm)} \times [\text{Minor axis (mm)}]^2$$

A reduction in estimated tumor volume was confirmed on day 18 in the combination group compared with the respective single agent groups of the anti-GARP antibody and the anti-CTLA4 antibody.

## Industrial Applicability

Use of the anti-GARP antibody of the present invention enables various cancers to be treated or prevented.

## Free Text of Sequence Listing

SEQ ID NO: 1: Amino acid sequence of GARP
SEQ ID NO: 2: Nucleotide sequence of cDNA encoding the heavy chain variable region of 151D
SEQ ID NO: 3: Amino acid sequence of the heavy chain variable region of 151D
SEQ ID NO: 4: Nucleotide sequence of cDNA encoding the light chain variable region of 151D
SEQ ID NO: 5: Amino acid sequence of the light chain variable region of 151D
SEQ ID NO: 6: Nucleotide sequence of cDNA encoding the heavy chain variable region of 198D
SEQ ID NO: 7: Amino acid sequence of the heavy chain variable region of 198D
SEQ ID NO: 8: Nucleotide sequence of cDNA encoding the light chain variable region of 198D
SEQ ID NO: 9: Amino acid sequence of the light chain variable region of 198D
SEQ ID NO: 10: Nucleotide sequence of a DNA fragment containing a sequence encoding the amino acid sequence of a human light chain signal sequence and a human κ chain constant region
SEQ ID NO: 11: Nucleotide sequence of a DNA fragment containing a sequence encoding a human heavy chain signal sequence and a human IgG1 constant region SEQ ID NO: 12: Nucleotide sequence of the heavy chain of a human chimeric antibody c151D
SEQ ID NO: 13: Amino acid sequence of the heavy chain of the human chimeric antibody c151D
SEQ ID NO: 14: Nucleotide sequence of the light chain of the human chimeric antibody c151D
SEQ ID NO: 15: Amino acid sequence of the light chain of the human chimeric antibody c151D
SEQ ID NO: 16: Nucleotide sequence of the heavy chain of a human chimeric antibody c198D
SEQ ID NO: 17: Amino acid sequence of the heavy chain of the human chimeric antibody c198D
SEQ ID NO: 18: Nucleotide sequence of the light chain of the human chimeric antibody c198D
SEQ ID NO: 19: Amino acid sequence of the light chain of the human chimeric antibody c198D
SEQ ID NO: 20: Nucleotide sequence of h151D-H1 of a humanized antibody
SEQ ID NO: 21: Amino acid sequence of h151D-H1 of the humanized antibody
SEQ ID NO: 22: Nucleotide sequence of h151D-H4 of a humanized antibody
SEQ ID NO: 23: Amino acid sequence of h151D-H4 of the humanized antibody
SEQ ID NO: 24: Nucleotide sequence of h151D-L1 of a humanized antibody
SEQ ID NO: 25: Amino acid sequence of h151D-L1 of the humanized antibody
SEQ ID NO: 26: Nucleotide sequence of h151D-L4 of a humanized antibody
SEQ ID NO: 27: Amino acid sequence of h151D-L4 of the humanized antibody
SEQ ID NO: 28: Nucleotide sequence of h198D-H3 of a humanized antibody
SEQ ID NO: 29: Amino acid sequence of h198D-H3 of the humanized antibody
SEQ ID NO: 30: Nucleotide sequence of h198D-L4 of a humanized antibody
SEQ ID NO: 31: Amino acid sequence of h198D-L4 of the humanized antibody

## Claims

**1.** A pharmaceutical composition for cancer treatment comprising an antibody having the following characteristics (1) to (3) or an antigen binding fragment thereof, and an immunomodulator which are administered in combination:

(1) specifically binding to glycoprotein-A repetitions predominant (GARP),
(2) having inhibitory activity against an immunosuppressive function of regulatory T cells, and
(3) having antibody dependent cellular cytotoxicity (ADCC) activity.

2. The pharmaceutical composition according to claim 1, wherein the antibody has antitumor activity *in vivo.*

3. The pharmaceutical composition according to claim 1 or 2, wherein the antibody comprises a heavy chain comprising CDRH1, CDRH2 and CDRH3 and a light chain comprising CDRL1, CDRL2 and CDRL3 as described in the following (1) or (2):

(1) CDRH1 consisting of the amino acid sequence as set forth in amino acid positions 45 to 54 of SEQ ID NO: 13, CDRH2 consisting of the amino acid sequence as set forth in amino acid positions 69 to 78 of SEQ ID NO: 13 and CDRH3 consisting of the amino acid sequence as set forth in amino acid positions 118 to 125 of SEQ ID NO: 13, and CDRL1 consisting of the amino acid sequence as set forth in amino acid positions 44 to 54 of SEQ ID NO: 15, CDRL2 consisting of the amino acid sequence as set forth in amino acid positions 70 to 76 of SEQ ID NO: 15 and CDRL3 consisting of the amino acid sequence as set forth in amino acid positions 109 to 117 of SEQ ID NO: 15, or
(2) CDRH1 consisting of the amino acid sequence as set forth in amino acid positions 45 to 54 of SEQ ID NO: 17, CDRH2 consisting of the amino acid sequence as set forth in amino acid positions 69 to 77 of SEQ ID NO: 17 and CDRH3 consisting of the amino acid sequence as set forth in amino acid positions 117 to 128 of SEQ ID NO: 17, and CDRL1 consisting of the amino acid sequence as set forth in amino acid positions 44 to 54 of SEQ ID NO: 19, CDRL2 consisting of the amino acid sequence as set forth in amino acid positions 70 to 76 of SEQ ID NO: 19 and CDRL3 consisting of the amino acid sequence as set forth in amino acid positions 109 to 117 of SEQ ID NO: 19.

4. The pharmaceutical composition according to any one of claims 1 to 3, wherein the antibody comprises a heavy chain variable region and a light chain variable region as described in any of the following (1) to (3):

(1) a heavy chain variable region consisting of the amino acid sequence as set forth in amino acid positions 20 to 136 of SEQ ID NO: 21 and a light chain variable region consisting of the amino acid sequence as set forth in amino acid positions 21 to 129 of SEQ ID NO: 25,
(2) a heavy chain variable region consisting of the amino acid sequence as set forth in amino acid positions 20 to 136 of SEQ ID NO: 23 and a light chain variable region consisting of the amino acid sequence as set forth in amino acid positions 21 to 129 of SEQ ID NO: 27, or
(3) a heavy chain variable region consisting of the amino acid sequence as set forth in amino acid positions 20 to 139 of SEQ ID NO: 29 and a light chain variable region consisting of the amino acid sequence as set forth in amino acid positions 21 to 129 of SEQ ID NO: 31.

5. The pharmaceutical composition according to any one of claims 1 to 4, wherein the antibody is a chimeric antibody.

6. The pharmaceutical composition according to any one of claims 1 to 4, wherein the antibody is a humanized antibody.

7. The pharmaceutical composition according to any one of claims 1 to 6, wherein the antibody comprises a heavy chain constant region of human IgG1, human IgG2 or human IgG4.

8. The pharmaceutical composition according to claim 6 or 7, wherein the antibody comprises a heavy chain and a light chain as described in any of the following (1) to (3) :

(1) a heavy chain having the amino acid sequence as set forth in amino acid positions 20 to 466 of SEQ ID NO: 21 and a light chain having the amino acid sequence as set forth in amino acid positions 21 to 234 of SEQ ID NO: 25,
(2) a heavy chain having the amino acid sequence as set forth in amino acid positions 20 to 466 of SEQ ID NO: 23 and a light chain having the amino acid sequence as set forth in amino acid positions 21 to 234 of SEQ ID NO: 27, or
(3) a heavy chain having the amino acid sequence as set forth in amino acid positions 20 to 469 of SEQ ID NO: 29 and a light chain having the amino acid sequence as set forth in amino acid positions 21 to 234 of SEQ ID NO: 31.

9. The pharmaceutical composition according to claim 1, wherein the antibody competes with an antibody according to any one of claims 2 to 8 for binding to GARP.

10. The pharmaceutical composition according to any one of claims 1 to 9, wherein the antibody comprises one or two or more modifications selected from the group consisting of N-linked glycosylation, O-linked glycosylation, N-terminal processing, C-terminal processing, deamidation, isomerization of aspartic acid, oxidation of methionine, N-terminal addition of a methionine residue, amidation of a proline residue, and deletion of one or two amino acid residues at the carboxyl terminus of a heavy chain.

**11.** The pharmaceutical composition according to claim 10, wherein one or several amino acid residues are deleted at the carboxyl terminus of a heavy chain.

**12.** The pharmaceutical composition according to claim 10 or 11, wherein one amino acid residue is deleted at the carboxyl termini of both heavy chains.

**13.** The pharmaceutical composition according to any one of claims 10 to 12, wherein a carboxyl-terminal proline residue of the heavy chain is further amidated.

**14.** The pharmaceutical composition according to any one of claims 1 to 13, wherein the immunomodulator is an anti-PD-1 antibody or an antigen binding fragment thereof, an anti-PD-Ll antibody or an antigen binding fragment thereof, an anti-PD-L2 antibody or an antigen binding fragment thereof, an anti-CTLA-4 antibody or an antigen binding fragment thereof, a multispecific antibody comprising any of these antibodies or antigen binding fragments thereof, a radiotherapy, a chemoradiotherapy, a chemotherapeutic or a combination thereof.

**15.** The pharmaceutical composition according to claim 14, wherein the anti-PD-1 antibody is nivolumab, pembrolizumab, lambrolizumab, MK-3475, AMP-224, pidilizumab or LOPD18.

**16.** The pharmaceutical composition according to claim 14, wherein the anti-PD-Ll antibody is atezolizumab, durvalumab or avelumab.

**17.** The pharmaceutical composition according to claim 14, wherein the anti-CTLA-4 antibody is ipilimumab or tremelimumab.

**18.** The pharmaceutical composition for cancer treatment according to any one of claims 1 to 17, wherein the antibody and the immunomodulator are contained as active ingredients in separate preparations and administered at the same time or at different times.

**19.** A pharmaceutical composition for cancer treatment comprising an antibody according to any one of claims 1 to 13 which is to be combined with an immunomodulator.

**20.** A pharmaceutical composition for cancer treatment comprising an antibody according to any one of claims 1 to 13 which is to treat a cancer patient given an immunomodulator.

**21.** A pharmaceutical composition for cancer treatment comprising an immunomodulator, wherein the pharmaceutical composition is combined with an antibody according to any one of claims 1 to 13, thereby increasing an effect of the antibody.

**22.** A pharmaceutical composition for cancer treatment comprising an immunomodulator which is to be combined with an antibody according to any one of claims 1 to 13.

**23.** A pharmaceutical composition for cancer treatment comprising an immunomodulator which is to treat a cancer patient given an antibody according to any one of claims 1 to 13.

**24.** A pharmaceutical composition for cancer treatment comprising an antibody according to any one of claims 1 to 13, wherein the pharmaceutical composition is combined with an immunomodulator, thereby increasing an effect of the immunomodulator.

**25.** The pharmaceutical composition according to any one of claims 1 to 24 which is to treat at least one cancer selected from the group consisting of lung cancer, kidney cancer, urothelial cancer, large intestine cancer, prostatic cancer, glioblastoma multiforme, ovary cancer, pancreatic cancer, breast cancer, melanoma, liver cancer, bladder cancer, stomach cancer, esophageal cancer, head and neck cancer, blood cancer, skin cancer, thyroid gland cancer, biliary tract cancer, salivary gland cancer, small intestine cancer, adrenal cancer, testicle cancer, uterine cervical cancer, endometrial cancer, uterine sarcoma, thymoma, mesothelioma, sarcoma and their metastatic forms.

**26.** A method for treating cancer, comprising administering an antibody having the following characteristics (1) to (3) or an antigen binding fragment thereof, and an immunomodulator in combination:

(1) specifically binding to glycoprotein-A repetitions predominant (GARP),
(2) having inhibitory activity against an immunosuppressive function of regulatory T cells, and
(3) having antibody dependent cellular cytotoxicity (ADCC) activity.

**27.** The method according to claim 26, wherein the antibody has antitumor activity *in vivo.*

**28.** The method according to claim 26 or 27, wherein the antibody comprises a heavy chain comprising CDRH1, CDRH2 and CDRH3 and a light chain comprising CDRL1, CDRL2 and CDRL3 as described in the following (1) or (2):

(1) CDRH1 consisting of the amino acid sequence as set forth in amino acid positions 45 to 54 of SEQ ID NO: 13, CDRH2 consisting of the amino acid sequence as set forth in amino acid positions 69 to 78 of SEQ ID NO: 13 and CDRH3 consisting of the amino acid sequence as set forth in amino acid positions 118 to 125 of SEQ ID NO: 13, and CDRL1 consisting of the amino acid sequence as set forth in amino acid positions 44 to 54 of SEQ ID NO: 15, CDRL2 consisting of the amino acid sequence as set forth in amino acid positions 70 to 76 of SEQ ID NO: 15 and CDRL3 consisting of the amino acid sequence as set forth in amino acid positions 109 to 117 of SEQ ID NO: 15, or
(2) CDRH1 consisting of the amino acid sequence as set forth in amino acid positions 45 to 54 of SEQ ID NO: 17, CDRH2 consisting of the amino acid sequence as set forth in amino acid positions 69 to 77 of SEQ ID NO: 17 and CDRH3 consisting of the amino acid sequence as set forth in amino acid positions 117 to 128 of SEQ ID NO: 17, and CDRL1 consisting of the amino acid sequence as set forth in amino acid positions 44 to 54 of SEQ ID NO: 19, CDRL2 consisting of the amino acid sequence as set forth in amino acid positions 70 to 76 of SEQ ID NO: 19 and CDRL3 consisting of the amino acid sequence as set forth in amino acid positions 109 to 117 of SEQ ID NO: 19.

**29.** The method according to any one of claims 26 to 28, wherein the antibody comprises a heavy chain variable region and a light chain variable region as described in any of the following (1) to (3):

(1) a heavy chain variable region consisting of the amino acid sequence as set forth in amino acid positions 20 to 136 of SEQ ID NO: 21 and a light chain variable region consisting of the amino acid sequence as set forth in amino acid positions 21 to 129 of SEQ ID NO: 25,
(2) a heavy chain variable region consisting of the amino acid sequence as set forth in amino acid positions 20 to 136 of SEQ ID NO: 23 and a light chain variable region consisting of the amino acid sequence as set forth in amino acid positions 21 to 129 of SEQ ID NO: 27, or
(3) a heavy chain variable region consisting of the amino acid sequence as set forth in amino acid positions 20 to 139 of SEQ ID NO: 29 and a light chain variable region consisting of the amino acid sequence as set forth in amino acid positions 21 to 129 of SEQ ID NO: 31.

**30.** The method according to any one of claims 26 to 29, wherein the antibody is a chimeric antibody.

**31.** The method according to any one of claims 26 to 29, wherein the antibody is a humanized antibody.

**32.** The method according to any one of claims 26 to 31, wherein the antibody comprises a heavy chain constant region of human IgG1, human IgG2 or human IgG4.

**33.** The method according to claim 31 or 32, wherein the antibody comprises a heavy chain and a light chain as described in any of the following (1) to (3):

(1) a heavy chain having the amino acid sequence as set forth in amino acid positions 20 to 466 of SEQ ID NO: 21 and a light chain having the amino acid sequence as set forth in amino acid positions 21 to 234 of SEQ ID NO: 25,
(2) a heavy chain having the amino acid sequence as set forth in amino acid positions 20 to 466 of SEQ ID NO: 23 and a light chain having the amino acid sequence as set forth in amino acid positions 21 to 234 of SEQ ID NO: 27, or

(3) a heavy chain having the amino acid sequence as set forth in amino acid positions 20 to 469 of SEQ ID NO: 29 and a light chain having the amino acid sequence as set forth in amino acid positions 21 to 234 of SEQ ID NO: 31.

**34.** The method according to claim 26, wherein the antibody competes with an antibody according to any one of claims 27 to 33 for binding to GARP.

**35.** The method according to any one of claims 26 to 34, wherein the antibody comprises one or two or more modifications selected from the group consisting of N-linked glycosylation, O-linked glycosylation, N-terminal processing, C-terminal processing, deamidation, isomerization of aspartic acid, oxidation of methionine, N-terminal addition of a methionine residue, amidation of a proline residue, and deletion of one or two amino acid residues at the carboxyl terminus of a heavy chain.

**36.** The method according to claim 35, wherein one or several amino acid residues are deleted at the carboxyl terminus of a heavy chain.

**37.** The method according to claim 35 or 36, wherein one amino acid residue is deleted at the carboxyl termini of both heavy chains.

**38.** The method according to any one of claims 35 to 37, wherein a carboxyl-terminal proline residue of the heavy chain is further amidated.

**39.** The method according to any one of claims 26 to 38, wherein the immunomodulator is an anti-PD-1 antibody or an antigen binding fragment thereof, an anti-PD-LI antibody or an antigen binding fragment thereof, an anti-PD-L2 antibody or an antigen binding fragment thereof, an anti-CTLA-4 antibody or an antigen binding fragment thereof, a multispecific antibody comprising any of these antibodies or antigen binding fragments thereof, a radiotherapy, a chemoradiotherapy, a chemotherapeutic or a combination thereof.

**40.** The method according to claim 39, wherein the anti-PD-1 antibody is nivolumab, pembrolizumab, lambrolizumab, MK-3475, AMP-224, pidilizumab or LOPD18.

**41.** The method according to claim 39, wherein the anti-PD-L1 antibody is atezolizumab, durvalumab or avelumab.

**42.** The method according to claim 39, wherein the anti-CTLA-4 antibody is ipilimumab or tremelimumab.

**43.** A method for treating cancer, comprising administering an effective amount of an antibody according to any one of claims 26 to 38 to a cancer patient in need thereof, wherein the method is combined with administering an immunomodulator.

**44.** A method for treating a cancer patient given an immunomodulator, comprising administering an effective amount of an antibody according to any one of claims 26 to 38 to the patient in need thereof.

**45.** A method for treating cancer which is combined with administration of an antibody according to any one of claims 26 to 38, thereby increasing an effect of the antibody, the method comprising administering an effective amount of an immunomodulator to a patient in need thereof.

**46.** A method for treating cancer, comprising administering an effective amount of an immunomodulator to a patient in need thereof, wherein the method is combined with administration of an antibody according to any one of claims 26 to 38.

**47.** A method for treating a cancer patient given an antibody according to any one of claims 26 to 38, comprising administering an effective amount of an immunomodulator to the patient in need thereof.

**48.** A method for treating cancer which is combined with administration of an immunomodulator, thereby increasing an effect of the immunomodulator, the method comprising administering an effective amount of an antibody according to any one of claims 26 to 38 to a patient in need thereof.

**49.** The method according to any one of claims 26 to 48, wherein the method is to treat at least one cancer selected from the group consisting of lung cancer, kidney cancer, urothelial cancer, large intestine cancer, prostatic cancer, glioblastoma multiforme, ovary cancer, pancreatic cancer, breast cancer, melanoma, liver cancer, bladder cancer, stomach cancer, esophageal cancer, head and neck cancer, blood cancer, skin cancer, thyroid gland cancer, biliary tract cancer, salivary gland cancer, small intestine cancer, adrenal cancer, testicle cancer, uterine cervical cancer, endometrial cancer, uterine sarcoma, thymoma, mesothelioma, sarcoma and their metastatic forms.

**50.** A kit preparation for cancer treatment comprising an antibody according to any one of claims 1 to 12, and one or two or more immunomodulators selected from the group consisting of an anti-PD-1 antibody, an anti-PD-Ll antibody, an anti-PD-L2 antibody and an anti-CTLA-4 antibody.

**51.** The preparation according to claim 50, further comprising an instruction manual for using the kit.

**52.** The pharmaceutical composition according to claim 14, wherein the anti-PD-1 antibody is nivolumab.

**53.** The pharmaceutical composition according to claim 14, wherein the anti-PD-1 antibody is pembrolizumab.

**54.** The method according to claim 39, wherein the anti-PD-1 antibody is nivolumab.

**55.** The method according to claim 39, wherein the anti-PD-1 antibody is pembrolizumab.

[Figure 1]

Amino acid sequence of c151D antibody heavy chain (SEQ ID NO: 13)

MKHLWFFLLLVAAPRWVLSEVQLVESGGGLVQPGRSKKLSCSASGFTFSNYYMAWVR
QAPTQGLEWVASIGTVGGNTYYRDSVKGRFTISRDDAKSTLYLQMDSLRSEDTATYY
CAREDYGGFPHWGQGVMVTVSSASTKGPSVFPLAPSSKSTSGGTAALGCLVKDYFPE
PVTVSWNSGALTSGVHTFPAVLQSSGLYSLSSVVTVPSSSLGTQTICNVNHKPSNTK
VDKRVEPKSCDKTHTCPPCPAPELLGGPSVFLFPPKPDTLMISRTPEVTCVVVDVSH
EDPEVKFNWYVDGVEVHNAKTKPREEQYNSTYRVVSVLTVLHQDWLNGKEYKCKVSN
KALPAPIEKTISKAKGPREPQVYTLPPSREEMTKNQVSLTCLVKGFYPSDIAVEWES
NGQPENNYTTPPVLDSDGSFFLYSKLTVDKSRWQQGNVFSCSVMHEALHNHYTQKSL
LSPGK

[Figure 2]

Amino acid sequence of c151D antibody light chain (SEQ ID NO: 15)

MVLQTQVFISLLLWISGAYGNIVMTQSPTSMFISVGDRVTMNCKASQNVGTNVDWYQ
QKTGQSPKLLIYGASNRYTGVPDRFTGSGSGTDFTLTISNMQAEDLAVYDCLQYKYN
PYTFGTGTKLELNRAVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

[Figure 3]

Amino acid sequence of c198D antibody heavy chain (SEQ ID NO: 17)

MKHLWFFLLLVAAPRWVLSQVQLRESGPGLVQPSQTLSLTCTVSGFSLTSFHVSWVR
QPPEKGLEWIATISSGGGTYYNSALKSRLSISRDTSKSQVFLKMSTLQTEDTAMYFC
ARISGWGHYYVMDVWGQGASVTVSSASTKGPSVFPLAPSSKSTSGGTAALGCLVKDY
FPEPVTVSWNSGALTSGVHTFPAVLQSSGLYSLSSVVTVPSSSLGTQTYICNVNHKP
SNTKVDKRVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEALHN
HYTQKSLSLSPGK

[Figure 4]

Amino acid sequence of c198D antibody light chain (SEQ ID NO: 19)

MVLQTQVFISLLLWISGAYGDIQMTQSPASLSGSLGETVTIQCQASEDIYSGLAWYQ
QKPGKSPQLLIYGAGSLQDGVPSRFSGGGSGTHYSLKISSMQTEDEGVYFCQQGLKF
PLTFGSGTKLEIKRAVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

[Figure 5]

Amino acid sequence of h151D-H1 heavy chain (SEQ ID NO: 21)

MKHLWFFLLLVAAPRWVLSEVQLVESGGGLVQPGGSLRLSCAASGFTFSNYYMAWVR
QAPGKGLEWVSSIGTVGGNTYYADSVKGRFTISRDDSKNTLYLQMNSLRAEDTAVYY
CAREDYGGFPHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNT
KVDKRVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYT
QKSLSLSPGK

[Figure 6]

Amino acid sequence of h151D-L1 light chain (SEQ ID NO: 25)

MVLQTQVFISLLLWISGAYGNIVMTQSPDSLAVSLGERATINCKASQNVGTNVDWYQ
QKPGQSPKLLIYGASNRYTGVPDRFSGSGSGTDFTLTISSLQAEDVAVYDCLQYKYN
PYTFGQGTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

[Figure 7]

Amino acid sequence of h151D-H4 heavy chain (SEQ ID NO: 23)

MKHLWFFLLLVAAPRWVLSEVQLVESGGGLVQPGGSLRLSCAASGFTFSNYYMAWVR
QAPGKGLEWVASIGTVGGNTYYRDSVKGRFTISRDDSKSTLYLQMNSLRAEDTAVYY
CAREDYGGFPHWGQGTLVTVSSASTKGPSVFPLAPSSKSTSGGTAALGCLVKDYFPE
PVTVSWNSGALTSGVHTFPAVLQSSGLYSLSSVVTVPSSSLGTQTYICNVNHKPSNT
KVDKRVEPKSCDKTHTCPPCPAPELLGGPSVFLFPPKPKDTLMISRTPEVTCVVVDV
SHEDPEVKFNWYVDGVEVHNAKTKPREEQYNSTYRVVSVLTVLHQDWLNGKEYKCKV
SNKALPAPIEKTISKAKGQPREPQVYTLPPSREEMTKNQVSLTCLVKGFYPSDIAVE
WESNGQPENNYKTTPPVLDSDGSFFLYSKLTVDKSRWQQGNVFSCSVMHEALHNHYT
QKSLSLSPGK

[Figure 8]

Amino acid sequence of h151D-L4 light chain (SEQ ID NO: 27)

MVLQTQVFISLLLWISGAYGNIVMTQSPSSLSASVGDRVTINCKASQNVGTNVDWYQ
QKPGKSPKLLIYGASNRYTGVPDRFSGSGSGTDFTLTISSLQPEDFATYDCLQYKYN
PYTFGQGTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

[Figure 9]

Amino acid sequence of h198D-H3 heavy chain (SEQ ID NO: 29)

MKHLWFFLLLVAAPRWVLSEVQLVESGGGLVKPSQTLSLTCTVSGFSLTSFHVSWVR
QPPGKGLEWIATISSGGGTYYNPSLKSRVTISRDTSKNQVSLKLSSVTAADTAVYYC
ARISGWGHYYVMDVWGQGTLVTVSSASTKGPSVFPLAPSSKSTSGGTAALGCLVKDY
FPEPVTVSWNSGALTSGVHTFPAVLQSSGLYSLSSVVTVPSSSLGTQTYICNVNHKP
SNTKVDKRVEPKSCDKTHTCPPCPAPELLGGPSVFLFPPKPKDTLMISRTPEVTCVV
VDVSHEDPEVKFNWYVDGVEVHNAKTKPREEQYNSTYRVVSVLTVLHQDWLNGKEYK
CKVSNKALPAPIEKTISKAKGQPREPQVYTLPPSREEMTKNQVSLTCLVKGFYPSDI
AVEWESNGQPENNYKTTPPVLDSDGSFFLYSKLTVDKSRWQQGNVFSCSVMHEALHN
HYTQKSLSLSPGK

[Figure 10]

Amino acid sequence of h198D-L4 light chain (SEQ ID NO: 31)

MVLQTQVFISLLLWISGAYGDIQMTQSPSSLSASVGDRVTITCQASEDIYSGLAWYQ
QKPGKSPKLLIYGAGSLQDGVPSRFSGSGSGTHYTLTISSLQPEDFATYFCQQGLKF
PLTFGQGTKVEIKRTVAAPSVFIFPPSDEQLKSGTASVVCLLNNFYPREAKVQWKVD
NALQSGNSQESVTEQDSKDSTYSLSSTLTLSKADYEKHKVYACEVTHQGLSSPVTKS
FNRGEC

Figure 11-1

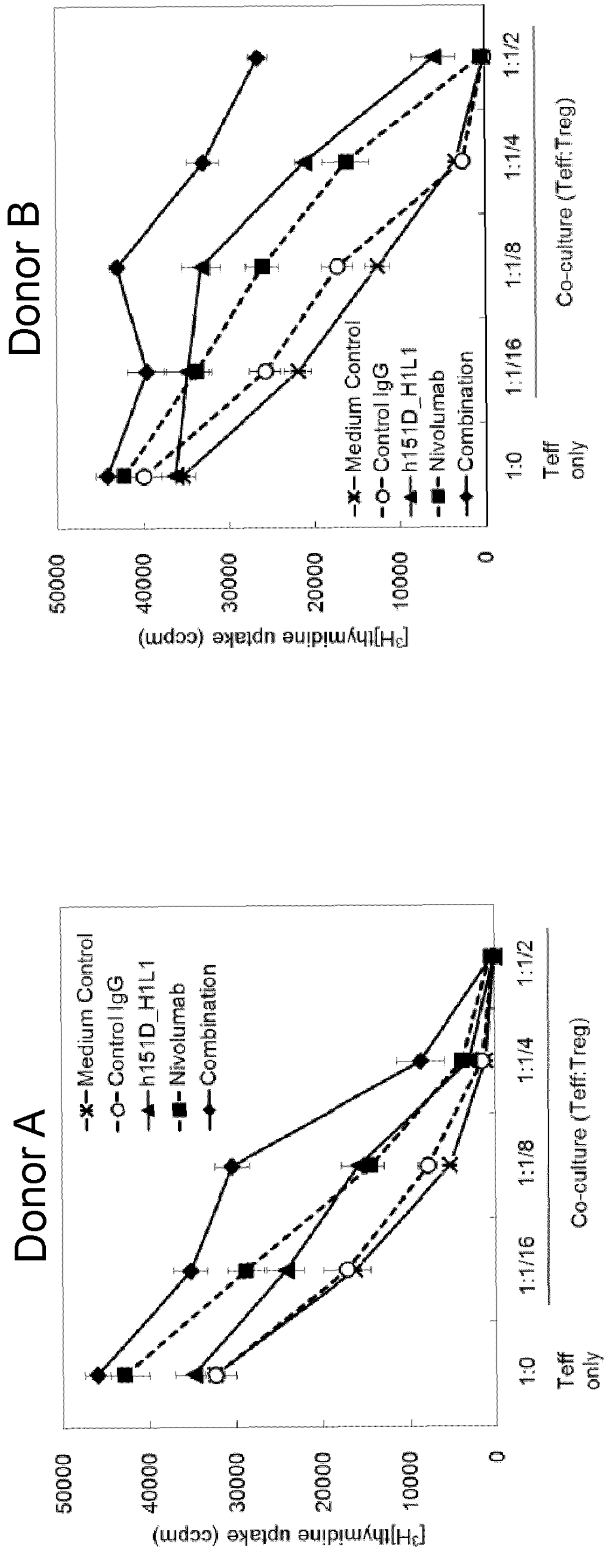
Donor A
Donor B
[3H]thymidine uptake (ccpm)
0
10000
20000
30000
40000
50000
1:0
Teff only
1:1/16
1:1/8
1:1/4
1:1/2
Co-culture (Teff:Treg)
Medium Control
Control IgG
h151D_H1L1
Nivolumab
Combination

Figure 11-2

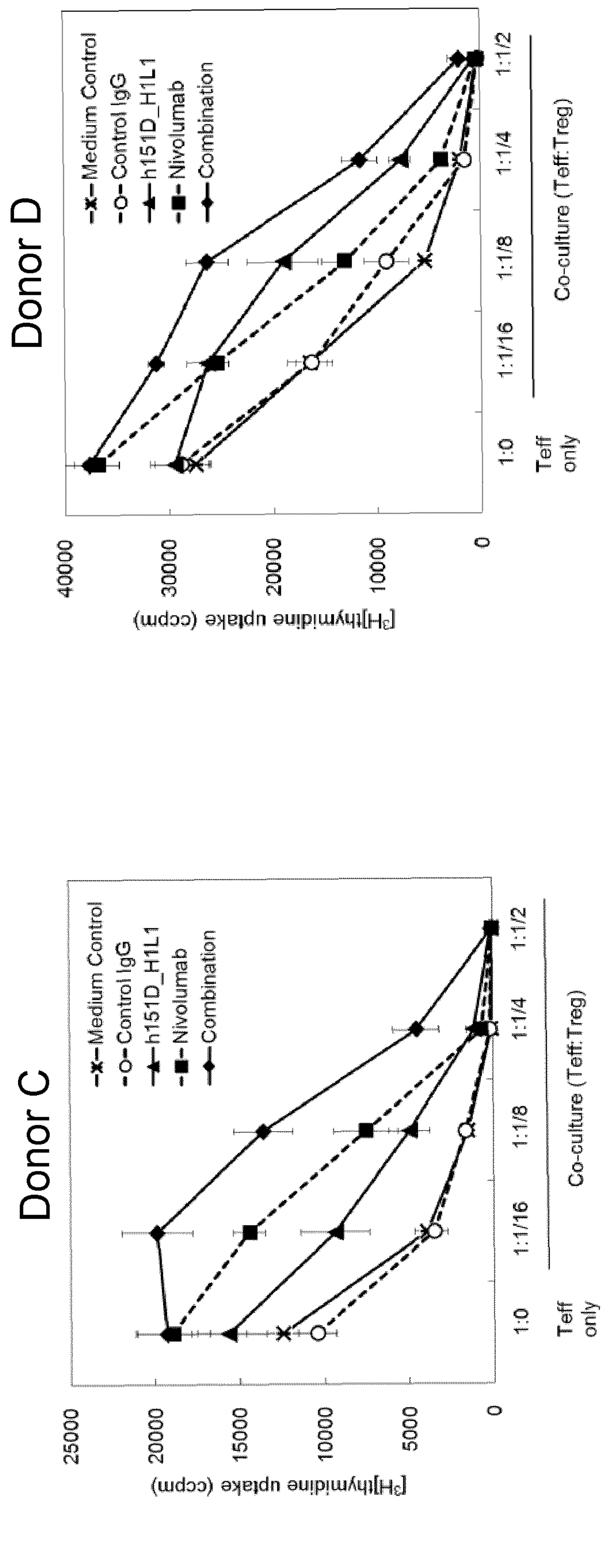
Donor C
-x- Medium Control
-o- Control IgG
-▲- h151D_H1L1
-■- Nivolumab
-◆- Combination
[3H]thymidine uptake (ccpm)
25000
20000
15000
10000
5000
0
1:0
Teff only
1:1/16
1:1/8
1:1/4
1:1/2
Co-culture (Teff:Treg)
Donor D
-x- Medium Control
-o- Control IgG
-▲- h151D_H1L1
-■- Nivolumab
-◆- Combination
[3H]thymidine uptake (ccpm)
40000
30000
20000
10000
0
1:0
Teff only
1:1/16
1:1/8
1:1/4
1:1/2
Co-culture (Teff:Treg)

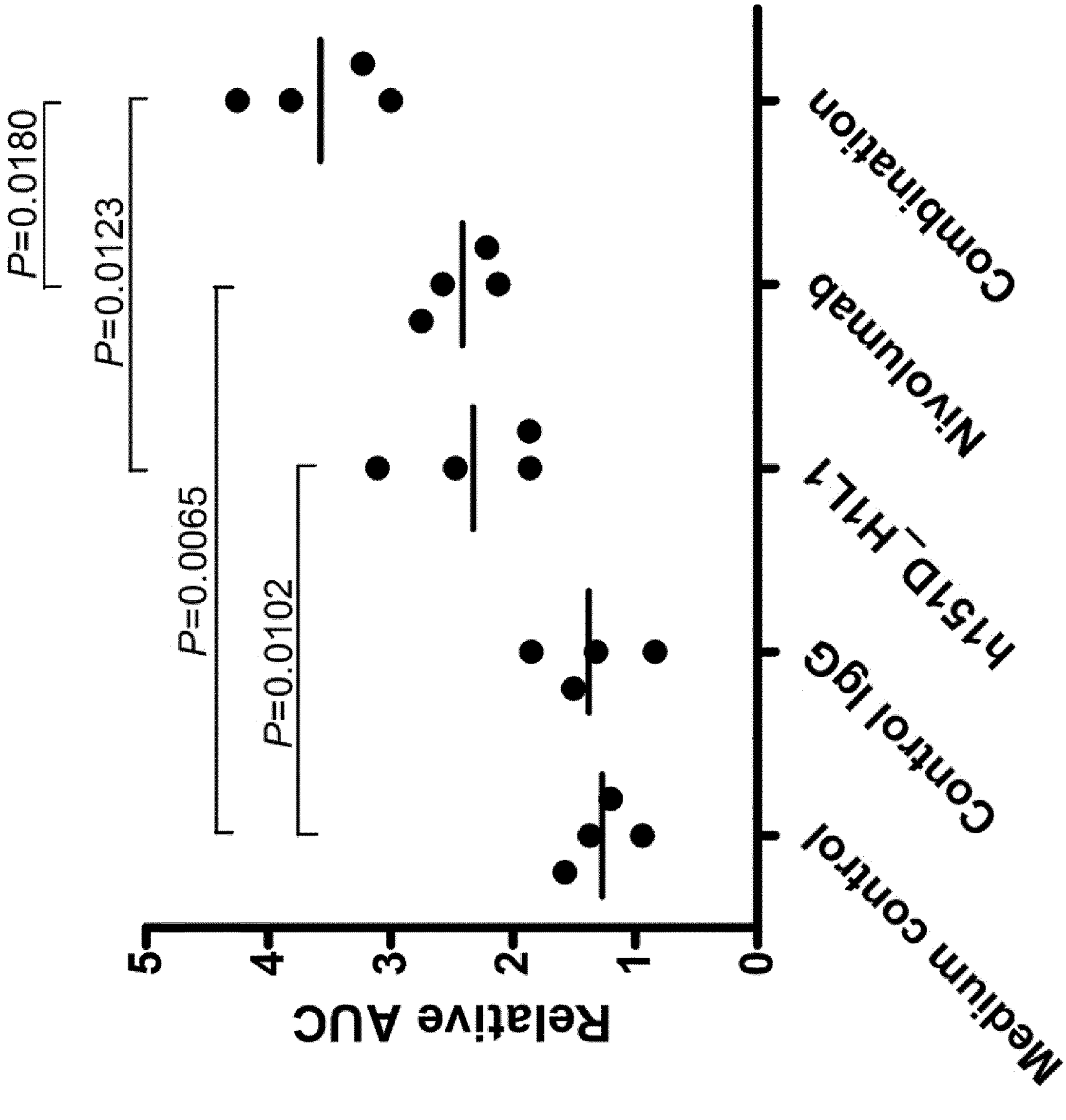
P=0.0180
P=0.0123
P=0.0065
P=0.0102
Relative AUC
5
4
3
2
1
0
Medium control
Control IgG
h151D_H1L1
Nivolumab
Combination

Figure 12

Figure 13

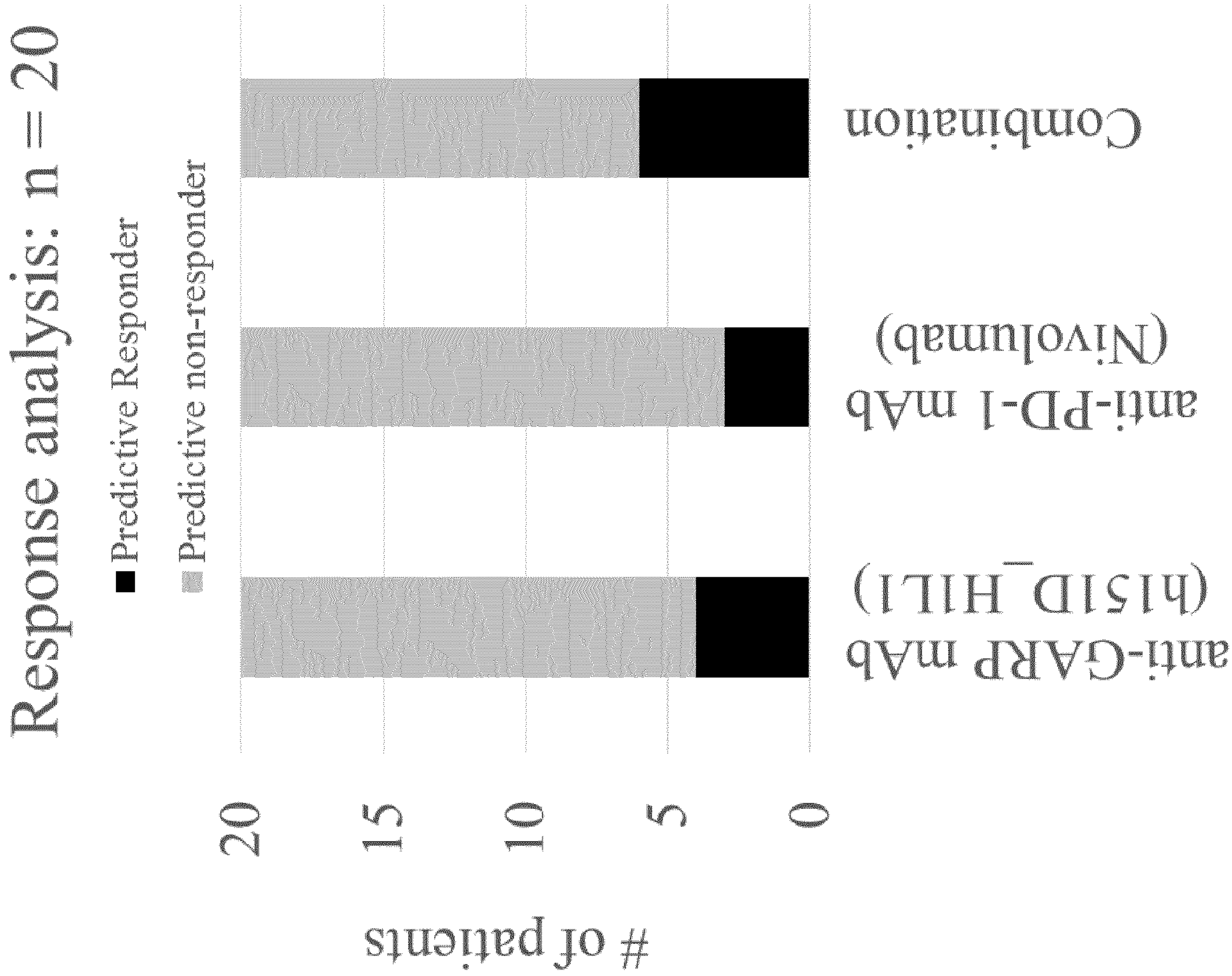
Response analysis: n = 20
Predictive Responder
Predictive non-responder
# of patients
20
15
10
5
0
anti-GARP mAb (h151D_H1L1)
anti-PD-1 mAb (Nivolumab)
Combination

## INTERNATIONAL SEARCH REPORT

International application No.
PCT/JP2020/039802

A. CLASSIFICATION OF SUBJECT MATTER
A61K 39/395(2006.01)i; A61K 45/00(2006.01)i; A61P35/00(2006.01)i; A61P37/02(2006.01)i; A61P43/00(2006.01)i; C12N 15/13(2006.01)n
FI: A61K39/395 D ZNA; A61K39/395 N; A61P35/00; A61P43/00 121; A61P43/00 111; A61P37/02; A61K45/00; C12N15/13
According to International Patent Classification (IPC) or to both national classification and IPC

B. FIELDS SEARCHED
Minimum documentation searched (classification system followed by classification symbols)
A61K39/395; A61K45/00; A61P35/00; A61P37/02; A61P43/00; C12N15/13

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN); GenBank/EMBL/DDBJ/GeneSeq; UniProt/GeneSeq

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2017/051888 A1 (DAIICHI SANKYO COMPANY, LIMITED) 30 March 2017 (2017-03-30) claims, paragraphs [0150]-[0160], examples | 1-14, 18-39, 43-49<br>1-55 |
| Y | WO 2016/125017 A1 (UNIVERSITE CATHOLUQUE DE LOUVAIN) 11 August 2016 (2016-08-11) claims, page 74, line 16 to page 75, line 9, page 77, lines 13-14 | 1-55 |
| Y | WO 2018/222718 A1 (BRISTOL-MYERS SQUIBB COMPANY) 06 December 2018 (2018-12-06) claims, paragraph [0276] | 1-55 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 December 2020 (11.12.2020) | 28 December 2020 (28.12.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2020/039802

C (Continuation). DOCUMENTS CONSIDERED TO BE RELEVANT

| Category* | Citation of document, with indication, where appropriate, of the relevant passages | Relevant to claim No. |
|---|---|---|
| Y | VARGAS, FA., et al., "Fe-Optimized Anti-CD25 Depletes Tumor-Infiltrating 1-55 Regulatory T Cells and Synergizes with PD-1 Blockade to Eradicate Established Tumors", Immunity, 2017, vol. 46, issue 4, pp. 577-586 summary, page 578, right column, page 582, fig. 1-3 | 1-55 |
| Y | WO 2018/181425 A1 (SHIONOGI & CO., LTD.) 04 October 2018 (2018-10-04) claims, examples | 1-55 |
| Y | JP 2012-500006 A (MEDAREX, INC.) 05 January 2012 (2012-01-05) claims, paragraph [0003], examples | 1-55 |

Form PCT/ISA/210 (continuation of second sheet) (January 2015)

**INTERNATIONAL SEARCH REPORT**
Information on patent family members

International application No.
PCT/JP2020/039802

| Patent Documents referred in the Report | Publication Date | Patent Family | Publication Date |
|---|---|---|---|
| WO 2017/051888 A1 | 30 Mar. 2017 | US 2018/0258184 A1 claims, paragraphs [0256]-[0267], examples<br>EP 3354729 A1 | |
| WO 2016/125017 A1 | 11 Aug. 2016 | US 2016/0251438 A1<br>EP 3253796 A1 | |
| WO 2018/222718 A1 | 06 Dec. 2018 | JP 2020-522691 A<br>EP 3631454 A1 | |
| WO 2018/181425 A1 | 04 Oct. 2018 | US 2019/0071508 A1 claims, examples<br>EP 3431105 A1 | |
| JP 2012-500006 A | 05 Jan. 2012 | WO 2010/019570 A2 claims, page 1, lines 18-33, examples<br>US 2011/0150892 A1<br>EP 2320940 A2 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017051888 A
- WO 2015015003 A
- WO 2016125017 A
- WO 2018206790 A
- WO 2006121168 A
- WO 2008156712 A
- WO 2010077634 A
- WO 2011066389 A
- WO 2013079174 A
- WO 2001014424 A
- WO 2000037504 A
- US 2018258184 A
- WO 9007861 A
- WO 9201047 A
- WO 9220791 A
- WO 9306213 A
- WO 9311236 A
- WO 9319172 A
- WO 9501438 A
- WO 9515388 A
- WO 9954342 A
- WO 0061739 A
- WO 0231140 A
- WO 2007133855 A
- WO 2004061104 A
- WO 2003038043 A


### Non-patent literature cited in the description


- *Int J Cancer.,* 15 August 2010, vol. 127 (4), 759-67
- *PLoS One.,* 2008, vol. 3 (7), e2705
- *Proc Natl Acad Sci U S A.,* 2009, vol. 106 (32), 13445-50
- *Eur J Immunol.,* 2009, vol. 39 (12), 3315-22
- *Mol Biol Cell,* 2012, vol. 23 (6), 1129-39
- *Blood.,* 2013, vol. 122 (7), 1182-91
- *Eur J Immunol.,* July 2012, vol. 42 (7), 1876-85
- *Clin Immunol.,* October 2013, vol. 149 (1), 97-110
- *Cancer Res.,* 2013, vol. 73, 2435
- *Sci Transl Med.,* 22 April 2015, vol. 7 (284
- *Cancers.,* November 2016, vol. 8 (12), 106
- *Nat Rev Cancer.,* March 2012, vol. 12 (4), 252-264
- *Cell,* 16 August 2015, vol. 2 (5), 937
- **KOHLER ; MILSTEIN.** *Nature,* 1975, vol. 256, 495-497
- Monoclonal Antibodies. Plenum Press, 1980, 365-367
- *Proc. Natl. Acad. Sci. U.S.A.,* 1984, vol. 81, 6851-6855
- *Nature,* 1986, vol. 321, 522-525
- **ALTSCHUL ; STEPHEN F. ; THOMAS L. MADDEN ; ALEJANDRO A. SCHAFFER ; JINGHUI ZHANG ; ZHENG ZHANG ; WEBB MILLER ; DAVID J. LIPMAN.** Gapped BLAST and PSI-BLAST: a new generation of protein database search programs. *Nucleic Acids Res.,* 1997, vol. 25, 3389-3402
- *Nature Genetics,* 1997, vol. 16, 133-143
- *Nucl. Acids Res.,* 1998, vol. 26, 3447-3448
- *Animal Cell Technology: Basic and Applied Aspects,* vol. 10, 69-73
- Proc. Natl. Acad. Sci. USA. Kluwer Academic Publishers, 1999, vol. 97, 722-727
- *Investigative Ophthalmology & Visual Science.,* 2002, vol. 43 (7), 2301-2308
- *Briefings in Functional Genomics and Proteomics,* 2002, vol. 1 (2), 189-203
- *Ophthalmology,* 2002, vol. 109 (3), 427-431
- *Nature Biotechnology,* 2005, vol. 23 (9), 1105-1116
- *Annu. Rev. Immunol,* 1994, vol. 12, 433-455
- **GLUZMAN, Y.** *Cell,* 1981, vol. 23, 175-182
- **URLAUB, G. ; CHASIN, L.A.** *Proc. Natl. Acad. Sci. U.S.A.,* 1980, vol. 77, 4126-4220
- *Journal of Chromatography A,* 1995, vol. 705, 129-134
- *Analytical Biochemistry,* 2007, vol. 360, 75-83
- *Nature Biotechnology,* 2005, vol. 23, 1137-1146
- *Nat Rev Drug Discov.,* 03 February 2012, vol. 11 (3), 215-33
- *Front Immunol.,* 17 July 2019, vol. 10, 1654
- *Clin Cancer Res,* 2009, vol. 15, 2148-2157
- *Cancer Immunol Immunother,* 2007, vol. 56, 641-648
- *Nat Commun.,* 27 February 2015, (6), 6169

SEQUENCE LISTING

<110> DAIICHI SANKYO COMPANY, LIMITED

<120> Combination of Anti-GARP Antibody and Immunomodulator

<130> DSFP2018WO

<150> JP2019-194717
<151> 2019-10-25

<160> 31

<170> PatentIn version 3.5

<210> 1
<211> 662
<212> PRT
<213> Human

<400> 1

```
Met Arg Pro Gln Ile Leu Leu Leu Leu Ala Leu Leu Thr Leu Gly Leu
1               5                   10                  15

Ala Ala Gln His Gln Asp Lys Val Pro Cys Lys Met Val Asp Lys Lys
            20                  25                  30

Val Ser Cys Gln Val Leu Gly Leu Leu Gln Val Pro Ser Val Leu Pro
        35                  40                  45

Pro Asp Thr Glu Thr Leu Asp Leu Ser Gly Asn Gln Leu Arg Ser Ile
    50                  55                  60

Leu Ala Ser Pro Leu Gly Phe Tyr Thr Ala Leu Arg His Leu Asp Leu
65                  70                  75                  80

Ser Thr Asn Glu Ile Ser Phe Leu Gln Pro Gly Ala Phe Gln Ala Leu
                85                  90                  95

Thr His Leu Glu His Leu Ser Leu Ala His Asn Arg Leu Ala Met Ala
            100                 105                 110

Thr Ala Leu Ser Ala Gly Gly Leu Gly Pro Leu Pro Arg Val Thr Ser
        115                 120                 125

Leu Asp Leu Ser Gly Asn Ser Leu Tyr Ser Gly Leu Leu Glu Arg Leu
    130                 135                 140

Leu Gly Glu Ala Pro Ser Leu His Thr Leu Ser Leu Ala Glu Asn Ser
145                 150                 155                 160

Leu Thr Arg Leu Thr Arg His Thr Phe Arg Asp Met Pro Ala Leu Glu
```

165 170 175

Gln Leu Asp Leu His Ser Asn Val Leu Met Asp Ile Glu Asp Gly Ala
180 185 190

Phe Glu Gly Leu Pro Arg Leu Thr His Leu Asn Leu Ser Arg Asn Ser
195 200 205

Leu Thr Cys Ile Ser Asp Phe Ser Leu Gln Gln Leu Arg Val Leu Asp
210 215 220

Leu Ser Cys Asn Ser Ile Glu Ala Phe Gln Thr Ala Ser Gln Pro Gln
225 230 235 240

Ala Glu Phe Gln Leu Thr Trp Leu Asp Leu Arg Glu Asn Lys Leu Leu
245 250 255

His Phe Pro Asp Leu Ala Ala Leu Pro Arg Leu Ile Tyr Leu Asn Leu
260 265 270

Ser Asn Asn Leu Ile Arg Leu Pro Thr Gly Pro Pro Gln Asp Ser Lys
275 280 285

Gly Ile His Ala Pro Ser Glu Gly Trp Ser Ala Leu Pro Leu Ser Ala
290 295 300

Pro Ser Gly Asn Ala Ser Gly Arg Pro Leu Ser Gln Leu Leu Asn Leu
305 310 315 320

Asp Leu Ser Tyr Asn Glu Ile Glu Leu Ile Pro Asp Ser Phe Leu Glu
325 330 335

His Leu Thr Ser Leu Cys Phe Leu Asn Leu Ser Arg Asn Cys Leu Arg
340 345 350

Thr Phe Glu Ala Arg Arg Leu Gly Ser Leu Pro Cys Leu Met Leu Leu
355 360 365

Asp Leu Ser His Asn Ala Leu Glu Thr Leu Glu Leu Gly Ala Arg Ala
370 375 380

Leu Gly Ser Leu Arg Thr Leu Leu Leu Gln Gly Asn Ala Leu Arg Asp
385 390 395 400

Leu Pro Pro Tyr Thr Phe Ala Asn Leu Ala Ser Leu Gln Arg Leu Asn
405 410 415

Leu Gln Gly Asn Arg Val Ser Pro Cys Gly Gly Pro Asp Glu Pro Gly
420 425 430

Pro Ser Gly Cys Val Ala Phe Ser Gly Ile Thr Ser Leu Arg Ser Leu
435 440 445

Ser Leu Val Asp Asn Glu Ile Glu Leu Leu Arg Ala Gly Ala Phe Leu
450 455 460

His Thr Pro Leu Thr Glu Leu Asp Leu Ser Ser Asn Pro Gly Leu Glu
465 470 475 480

Val Ala Thr Gly Ala Leu Gly Gly Leu Glu Ala Ser Leu Glu Val Leu
485 490 495

Ala Leu Gln Gly Asn Gly Leu Met Val Leu Gln Val Asp Leu Pro Cys
500 505 510

Phe Ile Cys Leu Lys Arg Leu Asn Leu Ala Glu Asn Arg Leu Ser His
515 520 525

Leu Pro Ala Trp Thr Gln Ala Val Ser Leu Glu Val Leu Asp Leu Arg
530 535 540

Asn Asn Ser Phe Ser Leu Leu Pro Gly Ser Ala Met Gly Gly Leu Glu
545 550 555 560

Thr Ser Leu Arg Arg Leu Tyr Leu Gln Gly Asn Pro Leu Ser Cys Cys
565 570 575

Gly Asn Gly Trp Leu Ala Ala Gln Leu His Gln Gly Arg Val Asp Val
580 585 590

Asp Ala Thr Gln Asp Leu Ile Cys Arg Phe Ser Ser Gln Glu Glu Val
595 600 605

Ser Leu Ser His Val Arg Pro Glu Asp Cys Glu Lys Gly Gly Leu Lys
610 615 620

Asn Ile Asn Leu Ile Ile Ile Leu Thr Phe Ile Leu Val Ser Ala Ile
625 630 635 640

Leu Leu Thr Thr Leu Ala Ala Cys Cys Cys Val Arg Arg Gln Lys Phe
645 650 655

Asn Gln Gln Tyr Lys Ala
660

<210> 2
<211> 351
<212> DNA
<213> Rat

<400> 2
gaggtgcagc tggtggagtc tgggggaggc ttagtgcagc ctggaaggtc caagaaactc 60

tcctgttcag cctcaggatt cactttcagt aactattaca tggcctgggt ccgccaggct 120

ccaacgcagg gtctggagtg ggtcgcatcc attggtactg ttggtggtaa cacttactat 180

cgagactccg tgaagggccg attcactatc tccagagatg atgcaaaaag caccctatac 240

ctgcaaatgg acagtctgag gtctgaggac acggccactt attactgtgc aagagaggat 300

tacggagggt ttccccactg gggccaagga gtcatggtca cagtctcctc a 351

<210> 3
<211> 117
<212> PRT
<213> Rat

<400> 3

Glu Val Gln Leu Val Glu Ser Gly Gly Gly Leu Val Gln Pro Gly Arg
1 5 10 15

Ser Lys Lys Leu Ser Cys Ser Ala Ser Gly Phe Thr Phe Ser Asn Tyr
20 25 30

Tyr Met Ala Trp Val Arg Gln Ala Pro Thr Gln Gly Leu Glu Trp Val
35 40 45

Ala Ser Ile Gly Thr Val Gly Gly Asn Thr Tyr Tyr Arg Asp Ser Val
50 55 60

Lys Gly Arg Phe Thr Ile Ser Arg Asp Asp Ala Lys Ser Thr Leu Tyr
65 70 75 80

Leu Gln Met Asp Ser Leu Arg Ser Glu Asp Thr Ala Thr Tyr Tyr Cys
85 90 95

Ala Arg Glu Asp Tyr Gly Gly Phe Pro His Trp Gly Gln Gly Val Met
100 105 110

Val Thr Val Ser Ser
115

<210> 4
<211> 327
<212> DNA
<213> Rat

<400> 4
aatattgtga tgactcagtc tcccacatcc atgttcatat cagtcggaga cagggtcacc 60

atgaactgta aggccagtca gaatgtggga actaatgtag actggtacca gcagaaaaca 120

gggcagtctc ctaaactgct tatctatggg gcgtccaacc gctacactgg agtccctgat 180

cgcttcacag gcagtggatc tggaacagat ttcactctca ccatcagcaa catgcaggct 240

gaagacctgg ctgtttatga ctgtctacag tataagtaca atccatacac gtttggaact 300

gggaccaagc tggaactgaa ccgggct 327

<210> 5
<211> 109
<212> PRT
<213> Rat

<400> 5

Asn Ile Val Met Thr Gln Ser Pro Thr Ser Met Phe Ile Ser Val Gly
1 5 10 15

Asp Arg Val Thr Met Asn Cys Lys Ala Ser Gln Asn Val Gly Thr Asn
20 25 30

Val Asp Trp Tyr Gln Gln Lys Thr Gly Gln Ser Pro Lys Leu Leu Ile
35 40 45

Tyr Gly Ala Ser Asn Arg Tyr Thr Gly Val Pro Asp Arg Phe Thr Gly
50 55 60

Ser Gly Ser Gly Thr Asp Phe Thr Leu Thr Ile Ser Asn Met Gln Ala
65 70 75 80

Glu Asp Leu Ala Val Tyr Asp Cys Leu Gln Tyr Lys Tyr Asn Pro Tyr
85 90 95

Thr Phe Gly Thr Gly Thr Lys Leu Glu Leu Asn Arg Ala
100 105

<210> 6
<211> 360
<212> DNA
<213> Rat

<400> 6
caggtgcagc tgagggagtc aggacctggt ctggtgcagc cctcacagac cctgtccctc 60

acctgcactg tctctgggtt ctcactaacc agctttcatg taagctgggt tcgccagcct 120

ccagagaagg gtctggagtg gattgcaaca atttcaagtg gtggaggtac atattataat 180

tcagctctca aatcccgact gagcatcagc agggacacct ccaagagcca agttttctta 240

aagatgagca ctctgcaaac tgaagacaca gccatgtact tctgtgcccg gatttcgggc 300

tggggccatt actatgttat ggatgtctgg ggtcaaggag cttcagtcac tgtctcctca 360

<210> 7
<211> 120
<212> PRT
<213> Rat

<400> 7

Gln Val Gln Leu Arg Glu Ser Gly Pro Gly Leu Val Gln Pro Ser Gln
1 5 10 15

Thr Leu Ser Leu Thr Cys Thr Val Ser Gly Phe Ser Leu Thr Ser Phe
20 25 30

His Val Ser Trp Val Arg Gln Pro Pro Glu Lys Gly Leu Glu Trp Ile
35 40 45

Ala Thr Ile Ser Ser Gly Gly Gly Thr Tyr Tyr Asn Ser Ala Leu Lys
50 55 60

Ser Arg Leu Ser Ile Ser Arg Asp Thr Ser Lys Ser Gln Val Phe Leu
65 70 75 80

Lys Met Ser Thr Leu Gln Thr Glu Asp Thr Ala Met Tyr Phe Cys Ala
85 90 95

Arg Ile Ser Gly Trp Gly His Tyr Tyr Val Met Asp Val Trp Gly Gln
100 105 110

Gly Ala Ser Val Thr Val Ser Ser
115 120

<210> 8
<211> 327
<212> DNA
<213> Rat

<400> 8
gacatccaga tgacacagtc tccagcttcc ctgtctggat ctctgggaga aactgtcacc 60

atccaatgtc aagcaagtga ggacatttac agtggtttag cgtggtatca gcagaagcca 120

gggaaatctc ctcagctcct gatctatggt gcaggtagct tacaagacgg cgtcccatca 180

cgattcagtg gcggtggatc tggcacacat tattctctca agatcagcag catgcaaact 240

gaagatgaag ggtttattt ctgtcaacag ggtttaaagt tccgctcac gttcggttct 300

gggaccaagc tggagatcaa acgggct 327

<210> 9
<211> 109
<212> PRT
<213> Rat

<400> 9

```
Asp Ile Gln Met Thr Gln Ser Pro Ala Ser Leu Ser Gly Ser Leu Gly
1               5                   10                  15

Glu Thr Val Thr Ile Gln Cys Gln Ala Ser Glu Asp Ile Tyr Ser Gly
            20                  25                  30

Leu Ala Trp Tyr Gln Gln Lys Pro Gly Lys Ser Pro Gln Leu Leu Ile
        35                  40                  45

Tyr Gly Ala Gly Ser Leu Gln Asp Gly Val Pro Ser Arg Phe Ser Gly
    50                  55                  60

Gly Gly Ser Gly Thr His Tyr Ser Leu Lys Ile Ser Ser Met Gln Thr
65                  70                  75                  80

Glu Asp Glu Gly Val Tyr Phe Cys Gln Gln Gly Leu Lys Phe Pro Leu
                85                  90                  95

Thr Phe Gly Ser Gly Thr Lys Leu Glu Ile Lys Arg Ala
            100                 105
```

<210> 10
<211> 449
<212> DNA
<213> Human

<400> 10

```
gcctccggac tctagagcca ccatggtgct gcagacccag gtgttcatct ccctgctgct      60

gtggatctcc ggcgcgtacg gcgatatcgt gatgattaaa cgtacggtgg ccgccccctc     120

cgtgttcatc ttccccccct ccgacgagca gctgaagtcc ggcaccgcct ccgtggtgtg     180

cctgctgaat aacttctacc ccagagaggc caaggtgcag tggaaggtgg acaacgccct     240

gcagtccggg aactcccagg agagcgtgac cgagcaggac agcaaggaca gcacctacag     300

cctgagcagc accctgaccc tgagcaaagc cgactacgag aagcacaagg tgtacgcctg     360

cgaggtgacc caccagggcc tgagctcccc cgtcaccaag agcttcaaca gggggagtg     420

ttaggggccc gtttaaacgg gggaggcta                                       449
```

<210> 11
<211> 1132
<212> DNA
<213> Human

<400> 11
gcctccggac tctagagcca ccatgaaaca cctgtggttc ttcctcctgc tggtggcagc 60

tcccagatgg gtgctgagcc aggtgcaatt gtgcaggcgg ttagctcagc ctccaccaag 120

ggcccaagcg tcttcccccct ggcaccctcc tccaagagca cctctggcgg cacagccgcc 180

ctgggctgcc tggtcaagga ctacttcccc gaacccgtga ccgtgagctg gaactcaggc 240

gccctgacca gcggcgtgca caccttcccc gctgtcctgc agtcctcagg actctactcc 300

ctcagcagcg tggtgaccgt gccctccagc agcttgggca cccagaccta catctgcaac 360

gtgaatcaca agcccagcaa caccaaggtg gacaagagag ttgagcccaa atcttgtgac 420

aaaactcaca catgcccacc ctgcccagca cctgaactcc tgggggggacc ctcagtcttc 480

ctcttccccc caaaacccaa ggacaccctc atgatctccc ggacccctga ggtcacatgc 540

gtggtggtgg acgtgagcca cgaagaccct gaggtcaagt tcaactggta cgtggacggc 600

gtggaggtgc ataatgccaa gacaaagccc cgggaggagc agtacaacag cacgtaccgg 660

gtggtcagcg tcctcaccgt cctgcaccag gactggctga atggcaagga gtacaagtgc 720

aaggtctcca acaaagccct cccagccccc atcgagaaaa ccatctccaa agccaaaggc 780

cagccccggg aaccacaggt gtacaccctg cccccatccc gggaggagat gaccaagaac 840

caggtcagcc tgacctgcct ggtcaaaggc ttctatccca gcgacatcgc cgtggagtgg 900

gagagcaatg gccagcccga gaacaactac aagaccaccc ctcccgtgct ggactccgac 960

ggctccttct tcctctacag caagctcacc gtggacaaga gcaggtggca gcagggcaac 1020

gtcttctcat gctccgtgat gcatgaggct ctgcacaacc actacaccca gaagagcctc 1080

tccctgtctc ccggcaaatg agatatcggg cccgtttaaa cgggggaggc ta 1132

<210> 12
<211> 1398
<212> DNA
<213> Artificial Sequence

<220>
<223> Chimeric Antibody

<400> 12
atgaaacacc tgtggttctt cctcctgctg gtggcagctc ccagatgggt gctgagcgag 60

gtgcagctgg tggagtctgg gggaggctta gtgcagcctg gaaggtccaa gaaactctcc 120

tgttcagcct caggattcac tttcagtaac tattacatgg cctgggtccg ccaggctcca 180

acgcagggtc tggagtgggt cgcatccatt ggtactgttg gtggtaacac ttactatcga 240

gactccgtga agggccgatt cactatctcc agagatgatg caaaaagcac cctatacctg 300

caaatggaca gtctgaggtc tgaggacacg gccacttatt actgtgcaag agaggattac 360

ggagggtttc cccactgggg ccaaggagtc atggtcacag tcagctcagc ctccaccaag 420

ggcccaagcg tcttccccct ggcaccctcc tccaagagca cctctggcgg cacagccgcc 480

ctgggctgcc tggtcaagga ctacttcccc gaacccgtga ccgtgagctg gaactcaggc 540

gccctgacca gcggcgtgca caccttcccc gctgtcctgc agtcctcagg actctactcc 600

ctcagcagcg tggtgaccgt gccctccagc agcttgggca cccagaccta catctgcaac 660

gtgaatcaca agcccagcaa caccaaggtg gacaagagag ttgagcccaa atcttgtgac 720

aaaactcaca catgcccacc ctgcccagca cctgaactcc tggggggacc ctcagtcttc 780

ctcttccccc caaaacccaa ggacaccctc atgatctccc ggacccctga ggtcacatgc 840

gtggtggtgg acgtgagcca cgaagaccct gaggtcaagt tcaactggta cgtggacggc 900

gtggaggtgc ataatgccaa gacaaagccc cgggaggagc agtacaacag cacgtaccgg 960

gtggtcagcg tcctcaccgt cctgcaccag gactggctga atggcaagga gtacaagtgc 1020

aaggtctcca acaaagccct cccagccccc atcgagaaaa ccatctccaa agccaaaggc 1080

cagccccggg aaccacaggt gtacaccctg cccccatccc gggaggagat gaccaagaac 1140

caggtcagcc tgacctgcct ggtcaaaggc ttctatccca gcgacatcgc cgtggagtgg 1200

gagagcaatg gccagcccga gaacaactac aagaccaccc ctcccgtgct ggactccgac 1260

ggctccttct tcctctacag caagctcacc gtggacaaga gcaggtggca gcagggcaac 1320

gtcttctcat gctccgtgat gcatgaggct ctgcacaacc actacaccca gaagagcctc 1380

tccctgtctc ccggcaaa 1398

<210> 13
<211> 466
<212> PRT
<213> Artificial Sequence

<220>
<223> Chimeric Antibody

<400> 13

Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Arg Ser Lys Lys Leu Ser Cys Ser Ala Ser Gly Phe Thr Phe
        35                  40                  45

Ser Asn Tyr Tyr Met Ala Trp Val Arg Gln Ala Pro Thr Gln Gly Leu
    50                  55                  60

Glu Trp Val Ala Ser Ile Gly Thr Val Gly Gly Asn Thr Tyr Tyr Arg

65 70 75 80

Asp Ser Val Lys Gly Arg Phe Thr Ile Ser Arg Asp Asp Ala Lys Ser
85 90 95

Thr Leu Tyr Leu Gln Met Asp Ser Leu Arg Ser Glu Asp Thr Ala Thr
100 105 110

Tyr Tyr Cys Ala Arg Glu Asp Tyr Gly Gly Phe Pro His Trp Gly Gln
115 120 125

Gly Val Met Val Thr Val Ser Ser Ala Ser Thr Lys Gly Pro Ser Val
130 135 140

Phe Pro Leu Ala Pro Ser Ser Lys Ser Thr Ser Gly Gly Thr Ala Ala
145 150 155 160

Leu Gly Cys Leu Val Lys Asp Tyr Phe Pro Glu Pro Val Thr Val Ser
165 170 175

Trp Asn Ser Gly Ala Leu Thr Ser Gly Val His Thr Phe Pro Ala Val
180 185 190

Leu Gln Ser Ser Gly Leu Tyr Ser Leu Ser Ser Val Val Thr Val Pro
195 200 205

Ser Ser Ser Leu Gly Thr Gln Thr Tyr Ile Cys Asn Val Asn His Lys
210 215 220

Pro Ser Asn Thr Lys Val Asp Lys Arg Val Glu Pro Lys Ser Cys Asp
225 230 235 240

Lys Thr His Thr Cys Pro Pro Cys Pro Ala Pro Glu Leu Leu Gly Gly
245 250 255

Pro Ser Val Phe Leu Phe Pro Pro Lys Pro Lys Asp Thr Leu Met Ile
260 265 270

Ser Arg Thr Pro Glu Val Thr Cys Val Val Val Asp Val Ser His Glu
275 280 285

Asp Pro Glu Val Lys Phe Asn Trp Tyr Val Asp Gly Val Glu Val His
290 295 300

Asn Ala Lys Thr Lys Pro Arg Glu Glu Gln Tyr Asn Ser Thr Tyr Arg
305 310 315 320

Val Val Ser Val Leu Thr Val Leu His Gln Asp Trp Leu Asn Gly Lys
325 330 335

Glu Tyr Lys Cys Lys Val Ser Asn Lys Ala Leu Pro Ala Pro Ile Glu
340 345 350

Lys Thr Ile Ser Lys Ala Lys Gly Gln Pro Arg Glu Pro Gln Val Tyr
355 360 365

Thr Leu Pro Pro Ser Arg Glu Glu Met Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Lys Leu Thr Val Asp
420 425 430

Lys Ser Arg Trp Gln Gln Gly Asn Val Phe Ser Cys Ser Val Met His
435 440 445

Glu Ala Leu His Asn His Tyr Thr Gln Lys Ser Leu Ser Leu Ser Pro
450 455 460

Gly Lys
465

<210> 14
<211> 702
<212> DNA
<213> Artificial Sequence

<220>
<223> Chimeric Antibody

<400> 14
atggtgctgc agacccaggt gttcatctcc ctgctgctgt ggatctccgg cgcgtacggc 60

aatattgtga tgactcagtc tcccacatcc atgttcatat cagtcggaga cagggtcacc 120

atgaactgta aggccagtca gaatgtggga actaatgtag actggtacca gcagaaaaca 180

gggcagtctc ctaaactgct tatctatggg gcgtccaacc gctacactgg agtccctgat 240

cgcttcacag gcagtggatc tggaacagat ttcactctca ccatcagcaa catgcaggct 300

gaagacctgg ctgtttatga ctgtctacag tataagtaca atccatacac gtttggaact 360

gggaccaagc tggaactgaa ccgggctgtg gccgcccct ccgtgttcat cttcccccc 420

```
tccgacgagc agctgaagtc cggcaccgcc tccgtggtgt gcctgctgaa taacttctac      480

cccagagagg ccaaggtgca gtggaaggtg gacaacgccc tgcagtccgg gaactcccag      540

gagagcgtga ccgagcagga cagcaaggac agcacctaca gcctgagcag caccctgacc      600

ctgagcaaag ccgactacga gaagcacaag gtgtacgcct gcgaggtgac ccaccagggc      660

ctgagctccc ccgtcaccaa gagcttcaac aggggggagt gt                         702


<210>  15
<211>  234
<212>  PRT
<213>  Artificial Sequence

<220>
<223>  Chimeric Antibody

<400>  15

Met Val Leu Gln Thr Gln Val Phe Ile Ser Leu Leu Leu Trp Ile Ser
1               5                   10                  15


Gly Ala Tyr Gly Asn Ile Val Met Thr Gln Ser Pro Thr Ser Met Phe
            20                  25                  30


Ile Ser Val Gly Asp Arg Val Thr Met Asn Cys Lys Ala Ser Gln Asn
        35                  40                  45


Val Gly Thr Asn Val Asp Trp Tyr Gln Gln Lys Thr Gly Gln Ser Pro
    50                  55                  60


Lys Leu Leu Ile Tyr Gly Ala Ser Asn Arg Tyr Thr Gly Val Pro Asp
65                  70                  75                  80


Arg Phe Thr Gly Ser Gly Ser Gly Thr Asp Phe Thr Leu Thr Ile Ser
                85                  90                  95


Asn Met Gln Ala Glu Asp Leu Ala Val Tyr Asp Cys Leu Gln Tyr Lys
            100                 105                 110


Tyr Asn Pro Tyr Thr Phe Gly Thr Gly Thr Lys Leu Glu Leu Asn Arg
        115                 120                 125


Ala Val Ala Ala Pro Ser Val Phe Ile Phe Pro Pro Ser Asp Glu Gln
    130                 135                 140


Leu Lys Ser Gly Thr Ala Ser Val Val Cys Leu Leu Asn Asn Phe Tyr
145                 150                 155                 160


Pro Arg Glu Ala Lys Val Gln Trp Lys Val Asp Asn Ala Leu Gln Ser
                165                 170                 175
```

```
Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```

```
<210>  16
<211>  1407
<212>  DNA
<213>  Artificial Sequence

<220>
<223>  Chimeric Antibody

<400>  16
atgaaacacc tgtggttctt cctcctgctg gtggcagctc ccagatgggt gctgagccag      60

gtgcagctga gggagtcagg acctggtctg gtgcagccct cacagaccct gtccctcacc     120

tgcactgtct ctgggttctc actaaccagc tttcatgtaa gctgggttcg ccagcctcca     180

gagaagggtc tggagtggat tgcaacaatt tcaagtggtg gaggtacata ttataattca     240

gctctcaaat cccgactgag catcagcagg gacacctcca agagccaagt tttcttaaag     300

atgagcactc tgcaaactga agacacagcc atgtacttct gtgcccggat ttcgggctgg     360

ggccattact atgttatgga tgtctggggt caaggagctt cagtcactgt cagctcagcc     420

tccaccaagg gcccaagcgt cttccccctg gcaccctcct ccaagagcac ctctggcggc     480

acagccgccc tgggctgcct ggtcaaggac tacttccccg aacccgtgac cgtgagctgg     540

aactcaggcg ccctgaccag cggcgtgcac accttccccg ctgtcctgca gtcctcagga     600

ctctactccc tcagcagcgt ggtgaccgtg ccctccagca gcttgggcac ccagacctac     660

atctgcaacg tgaatcacaa gcccagcaac accaaggtgg acaagagagt tgagcccaaa     720

tcttgtgaca aaactcacac atgcccaccc tgcccagcac ctgaactcct ggggggaccc     780

tcagtcttcc tcttcccccc aaaacccaag gacaccctca tgatctcccg gacccctgag     840

gtcacatgcg tggtggtgga cgtgagccac gaagaccctg aggtcaagtt caactggtac     900

gtggacggcg tggaggtgca taatgccaag acaaagcccc gggaggagca gtacaacagc     960

acgtaccggg tggtcagcgt cctcaccgtc ctgcaccagg actggctgaa tggcaaggag    1020

tacaagtgca aggtctccaa caaagccctc ccagccccca tcgagaaaac catctccaaa    1080
```

gccaaaggcc agccccggga accacaggtg tacaccctgc ccccatcccg ggaggagatg 1140

accaagaacc aggtcagcct gacctgcctg gtcaaaggct tctatcccag cgacatcgcc 1200

gtggagtggg agagcaatgg ccagcccgag aacaactaca agaccacccc tcccgtgctg 1260

gactccgacg gctccttctt cctctacagc aagctcaccg tggacaagag caggtggcag 1320

cagggcaacg tcttctcatg ctccgtgatg catgaggctc tgcacaacca ctacacccag 1380

aagagcctct ccctgtctcc cggcaaa 1407

<210> 17
<211> 469
<212> PRT
<213> Artificial Sequence

<220>
<223> Chimeric Antibody

<400> 17

Met Lys His Leu Trp Phe Phe Leu Leu Leu Val Ala Ala Pro Arg Trp
1 5 10 15

Val Leu Ser Gln Val Gln Leu Arg Glu Ser Gly Pro Gly Leu Val Gln
20 25 30

Pro Ser Gln Thr Leu Ser Leu Thr Cys Thr Val Ser Gly Phe Ser Leu
35 40 45

Thr Ser Phe His Val Ser Trp Val Arg Gln Pro Pro Glu Lys Gly Leu
50 55 60

Glu Trp Ile Ala Thr Ile Ser Ser Gly Gly Gly Thr Tyr Tyr Asn Ser
65 70 75 80

Ala Leu Lys Ser Arg Leu Ser Ile Ser Arg Asp Thr Ser Lys Ser Gln
85 90 95

Val Phe Leu Lys Met Ser Thr Leu Gln Thr Glu Asp Thr Ala Met Tyr
100 105 110

Phe Cys Ala Arg Ile Ser Gly Trp Gly His Tyr Tyr Val Met Asp Val
115 120 125

Trp Gly Gln Gly Ala Ser Val Thr Val Ser Ser Ala Ser Thr Lys Gly
130 135 140

Pro Ser Val Phe Pro Leu Ala Pro Ser Ser Lys Ser Thr Ser Gly Gly
145 150 155 160

```
Thr Ala Ala Leu Gly Cys Leu Val Lys Asp Tyr Phe Pro Glu Pro Val
                165                 170                 175

Thr Val Ser Trp Asn Ser Gly Ala Leu Thr Ser Gly Val His Thr Phe
            180                 185                 190

Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Arg Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Glu Glu Met Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415
```

Pro Pro Val Leu Asp Ser Asp Gly Ser Phe Phe Leu Tyr Ser Lys Leu
420 425 430

Thr Val Asp Lys Ser Arg Trp Gln Gln Gly Asn Val Phe Ser Cys Ser
435 440 445

Val Met His Glu Ala Leu His Asn His Tyr Thr Gln Lys Ser Leu Ser
450 455 460

Leu Ser Pro Gly Lys
465

<210> 18
<211> 702
<212> DNA
<213> Artificial Sequence

<220>
<223> Chimeric Antibody

<400> 18
atggtgctgc agacccaggt gttcatctcc ctgctgctgt ggatctccgg cgcgtacggc 60

gacatccaga tgacacagtc tccagcttcc ctgtctggat ctctgggaga aactgtcacc 120

atccaatgtc aagcaagtga ggacatttac agtggtttag cgtggtatca gcagaagcca 180

gggaaatctc ctcagctcct gatctatggt gcaggtagct tacaagacgg cgtcccatca 240

cgattcagtg gcggtggatc tggcacacat tattctctca agatcagcag catgcaaact 300

gaagatgaag ggtttattt ctgtcaacag ggtttaaagt ttccgctcac gttcggttct 360

gggaccaagc tggagatcaa acgggctgtg gccgccccct ccgtgttcat cttccccccc 420

tccgacgagc agctgaagtc cggcaccgcc tccgtggtgt gcctgctgaa taacttctac 480

cccagagagg ccaaggtgca gtggaaggtg gacaacgccc tgcagtccgg gaactcccag 540

gagagcgtga ccgagcagga cagcaaggac agcacctaca gcctgagcag caccctgacc 600

ctgagcaaag ccgactacga gaagcacaag gtgtacgcct gcgaggtgac ccaccagggc 660

ctgagctccc ccgtcaccaa gagcttcaac aggggggagt gt 702

<210> 19
<211> 234
<212> PRT
<213> Artificial Sequence

<220>
<223> Chimeric Antibody

<400> 19

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ala Ser Leu Ser
20 25 30

Gly Ser Leu Gly Glu Thr Val Thr Ile Gln Cys Gln Ala Ser Glu Asp
35 40 45

Ile Tyr Ser Gly Leu Ala Trp Tyr Gln Gln Lys Pro Gly Lys Ser Pro
50 55 60

Gln Leu Leu Ile Tyr Gly Ala Gly Ser Leu Gln Asp Gly Val Pro Ser
65 70 75 80

Arg Phe Ser Gly Gly Gly Ser Gly Thr His Tyr Ser Leu Lys Ile Ser
85 90 95

Ser Met Gln Thr Glu Asp Glu Gly Val Tyr Phe Cys Gln Gln Gly Leu
100 105 110

Lys Phe Pro Leu Thr Phe Gly Ser Gly Thr Lys Leu Glu Ile Lys Arg
115 120 125

Ala Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 20
<211> 1398
<212> DNA
<213> Artificial Sequence

<220>
<223> Humanized Antibody

<400> 20
atgaagcacc tgtggttctt tctgctgctg gtggccgctc ccagatgggt gctgtctgaa 60

gtgcagctgg tggaatccgg cggaggcctg gtgcagcctg gcggatctct gagactgtct 120

tgtgccgcct ccggcttcac cttctccaac tactacatgg cctgggtgcg acaggcccct 180

ggcaagggac tggaatgggt gtcctctatc ggcaccgtgg gcggcaacac ctactacgcc 240

gattctgtga agggccggtt caccatctcc cgggacgact ccaagaacac cctgtacctg 300

cagatgaact ccctgcgggc cgaggacacc gccgtgtact actgtgccag agaggactac 360

ggcggcttcc ctcattgggg ccagggcaca ctcgtgaccg tgtcctctgc ttccaccaag 420

ggcccctccg tgtttcctct ggccccttcc agcaagtcca cctctggcgg aacagccgct 480

ctgggctgcc tcgtgaagga ctacttcccc gagcccgtga cagtgtcttg gaactctggc 540

gccctgacct ccggcgtgca cacctttcca gctgtgctgc agtcctccgg cctgtactcc 600

ctgtcctccg tcgtgactgt gccctccagc tctctgggca cccagaccta catctgcaac 660

gtgaaccaca agccctccaa caccaaggtg gacaagcggg tggaacccaa gtcctgcgac 720

aagacccaca cctgtccccc ttgtcctgcc cctgaactgc tgggcggacc ttccgtgttc 780

ctgttccccc caaagcctaa ggacaccctg atgatctccc ggacccccga agtgacctgc 840

gtggtggtgg atgtgtccca cgaggaccct gaagtgaagt tcaattggta cgtggacggc 900

gtggaagtgc acaacgccaa gaccaagcct agagaggaac agtacaactc cacctaccgg 960

gtggtgtccg tgctgaccgt gctgcatcag gactggctga acggcaaaga gtacaagtgc 1020

aaggtgtcca acaaggccct gcctgccccc atcgaaaaga ccatctccaa ggccaagggc 1080

cagccccggg aaccccaggt gtacacactg ccccctagcc gggaagagat gaccaagaac 1140

caggtgtccc tgacctgtct cgtgaaaggc ttctacccct ccgatatcgc cgtggaatgg 1200

gagagcaacg gccagcccga gaacaactac aagaccaccc cccctgtgct ggactccgac 1260

ggctcattct tcctgtacag caagctgaca gtggacaagt cccggtggca gcagggcaac 1320

gtgttctcct gctccgtgat gcacgaggcc ctgcacaacc actacaccca gaagtccctg 1380

tccctgagcc ccggcaaa 1398

<210> 21
<211> 466
<212> PRT
<213> Artificial Sequence

<220>
<223> Humanized Antibody

<400> 21

```
Met Lys His Leu Trp Phe Phe Leu Leu Leu Val Ala Ala Pro Arg Trp
1               5                   10                  15

Val Leu Ser Glu Val Gln Leu Val Glu Ser Gly Gly Gly Leu Val Gln
            20                  25                  30

Pro Gly Gly Ser Leu Arg Leu Ser Cys Ala Ala Ser Gly Phe Thr Phe
        35                  40                  45

Ser Asn Tyr Tyr Met Ala Trp Val Arg Gln Ala Pro Gly Lys Gly Leu
    50                  55                  60

Glu Trp Val Ser Ser Ile Gly Thr Val Gly Gly Asn Thr Tyr Tyr Ala
65                  70                  75                  80

Asp Ser Val Lys Gly Arg Phe Thr Ile Ser Arg Asp Asp Ser Lys Asn
                85                  90                  95

Thr Leu Tyr Leu Gln Met Asn Ser Leu Arg Ala Glu Asp Thr Ala Val
            100                 105                 110

Tyr Tyr Cys Ala Arg Glu Asp Tyr Gly Gly Phe Pro His Trp Gly Gln
        115                 120                 125

Gly Thr Leu Val Thr Val Ser Ser Ala Ser Thr Lys Gly Pro Ser Val
    130                 135                 140

Phe Pro Leu Ala Pro Ser Ser Lys Ser Thr Ser Gly Gly Thr Ala Ala
145                 150                 155                 160

Leu Gly Cys Leu Val Lys Asp Tyr Phe Pro Glu Pro Val Thr Val Ser
                165                 170                 175

Trp Asn Ser Gly Ala Leu Thr Ser Gly Val His Thr Phe Pro Ala Val
            180                 185                 190

Leu Gln Ser Ser Gly Leu Tyr Ser Leu Ser Ser Val Val Thr Val Pro
        195                 200                 205

Ser Ser Ser Leu Gly Thr Gln Thr Tyr Ile Cys Asn Val Asn His Lys
    210                 215                 220

Pro Ser Asn Thr Lys Val Asp Lys Arg Val Glu Pro Lys Ser Cys Asp
225                 230                 235                 240

Lys Thr His Thr Cys Pro Pro Cys Pro Ala Pro Glu Leu Leu Gly Gly
                245                 250                 255
```

Pro Ser Val Phe Leu Phe Pro Pro Lys Pro Lys Asp Thr Leu Met Ile
260 265 270

Ser Arg Thr Pro Glu Val Thr Cys Val Val Val Asp Val Ser His Glu
275 280 285

Asp Pro Glu Val Lys Phe Asn Trp Tyr Val Asp Gly Val Glu Val His
290 295 300

Asn Ala Lys Thr Lys Pro Arg Glu Glu Gln Tyr Asn Ser Thr Tyr Arg
305 310 315 320

Val Val Ser Val Leu Thr Val Leu His Gln Asp Trp Leu Asn Gly Lys
325 330 335

Glu Tyr Lys Cys Lys Val Ser Asn Lys Ala Leu Pro Ala Pro Ile Glu
340 345 350

Lys Thr Ile Ser Lys Ala Lys Gly Gln Pro Arg Glu Pro Gln Val Tyr
355 360 365

Thr Leu Pro Pro Ser Arg Glu Glu Met Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Lys Leu Thr Val Asp
420 425 430

Lys Ser Arg Trp Gln Gln Gly Asn Val Phe Ser Cys Ser Val Met His
435 440 445

Glu Ala Leu His Asn His Tyr Thr Gln Lys Ser Leu Ser Leu Ser Pro
450 455 460

Gly Lys
465

<210> 22
<211> 1398
<212> DNA
<213> Artificial Sequence

<220>

<223> Humanized Antibody

<400> 22
atgaagcacc tgtggttctt tctgctgctg gtggccgctc ccagatgggt gctgtctgaa 60

gtgcagctgg tggaatccgg cggaggcctg gtgcagcctg gcggatctct gagactgtct 120

tgtgccgcct ccggcttcac cttctccaac tactacatgg cctgggtgcg acaggcccct 180

ggcaagggac tggaatgggt ggcctctatc ggcaccgtgg gcggcaacac ctactaccgg 240

gattctgtga agggccggtt caccatctcc cgggacgact ccaagtccac cctgtacctg 300

cagatgaact ccctgcgggc cgaggacacc gccgtgtact actgtgccag agaggactac 360

ggcggcttcc ctcattgggg ccagggcaca ctcgtgaccg tgtcctctgc ttccaccaag 420

ggcccctccg tgtttcctct ggccccttcc agcaagtcta cctccggcgg aacagccgct 480

ctgggctgcc tcgtgaagga ctacttcccc gagcccgtga cagtgtcttg gaactctggc 540

gccctgacca gcggcgtgca cacctttcca gctgtgctgc agtcctccgg cctgtactcc 600

ctgtcctccg tcgtgactgt gccctccagc tctctgggca cccagaccta catctgcaac 660

gtgaaccaca agccctccaa caccaaggtg gacaagcggg tggaacccaa gtcctgcgac 720

aagacccaca cctgtccccc ttgtcctgcc cctgaactgc tgggcggacc ttccgtgttc 780

ctgttccccc caaagcccaa ggacaccctg atgatctccc ggacccccga agtgacctgc 840

gtggtggtgg atgtgtccca cgaggaccct gaagtgaagt tcaattggta cgtggacggc 900

gtggaagtgc acaacgccaa gaccaagcct agagaggaac agtacaactc cacctaccgg 960

gtggtgtccg tgctgaccgt gctgcatcag gactggctga acggcaaaga gtacaagtgc 1020

aaggtgtcca acaaggccct gcctgccccc atcgaaaaga ccatctccaa ggccaagggc 1080

cagccccggg aaccccaggt gtacacactg ccccctagcc gggaagagat gaccaagaac 1140

caggtgtccc tgacctgtct cgtgaaaggc ttctacccct ccgatatcgc cgtggaatgg 1200

gagtccaacg gccagcctga gaacaactac aagaccaccc cccctgtgct ggactccgac 1260

ggctcattct tcctgtacag caagctgaca gtggacaagt cccggtggca gcagggcaac 1320

gtgttctcct gctccgtgat gcacgaggcc ctgcacaacc actacaccca gaagtccctg 1380

tccctgagcc ccggcaaa 1398

<210> 23
<211> 466
<212> PRT
<213> Artificial Sequence

<220>
<223> Humanized Antibody

<400> 23

Met Lys His Leu Trp Phe Phe Leu Leu Leu Val Ala Ala Pro Arg Trp

1 5 10 15

Val Leu Ser Glu Val Gln Leu Val Glu Ser Gly Gly Gly Leu Val Gln
20 25 30

Pro Gly Gly Ser Leu Arg Leu Ser Cys Ala Ala Ser Gly Phe Thr Phe
35 40 45

Ser Asn Tyr Tyr Met Ala Trp Val Arg Gln Ala Pro Gly Lys Gly Leu
50 55 60

Glu Trp Val Ala Ser Ile Gly Thr Val Gly Gly Asn Thr Tyr Tyr Arg
65 70 75 80

Asp Ser Val Lys Gly Arg Phe Thr Ile Ser Arg Asp Asp Ser Lys Ser
85 90 95

Thr Leu Tyr Leu Gln Met Asn Ser Leu Arg Ala Glu Asp Thr Ala Val
100 105 110

Tyr Tyr Cys Ala Arg Glu Asp Tyr Gly Gly Phe Pro His Trp Gly Gln
115 120 125

Gly Thr Leu Val Thr Val Ser Ser Ala Ser Thr Lys Gly Pro Ser Val
130 135 140

Phe Pro Leu Ala Pro Ser Ser Lys Ser Thr Ser Gly Gly Thr Ala Ala
145 150 155 160

Leu Gly Cys Leu Val Lys Asp Tyr Phe Pro Glu Pro Val Thr Val Ser
165 170 175

Trp Asn Ser Gly Ala Leu Thr Ser Gly Val His Thr Phe Pro Ala Val
180 185 190

Leu Gln Ser Ser Gly Leu Tyr Ser Leu Ser Ser Val Val Thr Val Pro
195 200 205

Ser Ser Ser Leu Gly Thr Gln Thr Tyr Ile Cys Asn Val Asn His Lys
210 215 220

Pro Ser Asn Thr Lys Val Asp Lys Arg Val Glu Pro Lys Ser Cys Asp
225 230 235 240

Lys Thr His Thr Cys Pro Pro Cys Pro Ala Pro Glu Leu Leu Gly Gly
245 250 255

Pro Ser Val Phe Leu Phe Pro Pro Lys Pro Lys Asp Thr Leu Met Ile
260 265 270

Ser Arg Thr Pro Glu Val Thr Cys Val Val Val Asp Val Ser His Glu
275 280 285

Asp Pro Glu Val Lys Phe Asn Trp Tyr Val Asp Gly Val Glu Val His
290 295 300

Asn Ala Lys Thr Lys Pro Arg Glu Glu Gln Tyr Asn Ser Thr Tyr Arg
305 310 315 320

Val Val Ser Val Leu Thr Val Leu His Gln Asp Trp Leu Asn Gly Lys
325 330 335

Glu Tyr Lys Cys Lys Val Ser Asn Lys Ala Leu Pro Ala Pro Ile Glu
340 345 350

Lys Thr Ile Ser Lys Ala Lys Gly Gln Pro Arg Glu Pro Gln Val Tyr
355 360 365

Thr Leu Pro Pro Ser Arg Glu Glu Met Thr Lys Asn Gln Val Ser Leu
370 375 380

Thr Cys Leu Val Lys Gly Phe Tyr Pro Ser Asp Ile Ala Val Glu Trp
385 390 395 400

Glu Ser Asn Gly Gln Pro Glu Asn Asn Tyr Lys Thr Thr Pro Pro Val
405 410 415

Leu Asp Ser Asp Gly Ser Phe Phe Leu Tyr Ser Lys Leu Thr Val Asp
420 425 430

Lys Ser Arg Trp Gln Gln Gly Asn Val Phe Ser Cys Ser Val Met His
435 440 445

Glu Ala Leu His Asn His Tyr Thr Gln Lys Ser Leu Ser Leu Ser Pro
450 455 460

Gly Lys
465

<210> 24
<211> 702
<212> DNA
<213> Artificial Sequence

<220>
<223> Humanized Antibody

<400> 24
atggtgctgc agacccaggt gttcatctcc ctgctgctgt ggatctccgg cgcctacggc 60

aacatcgtga tgacccagtc ccccgactcc ctggctgtgt ctctgggcga gagagccacc 120

atcaactgca aggcctccca gaacgtgggc accaacgtgg actggtatca gcagaagccc 180

ggccagtccc ctaagctgct gatctacggc gccagcaacc ggtacaccgg cgtgcccgat 240

agattctccg gctctggctc tggcaccgac tttaccctga caatcagctc tctgcaggcc 300

gaggacgtgg ccgtgtacga ctgcctgcag tacaagtaca acccctacac cttcggccag 360

ggcacaaagg tggaaatcaa gcggaccgtg gccgctccct ccgtgtttat cttcccaccc 420

tccgacgagc agctgaagtc cggcacagct tccgtcgtgt gcctgctgaa caacttctac 480

ccccgcgagg ccaaggtgca gtggaaggtg gacaacgccc tgcagtccgg caactcccag 540

gaatccgtga ccgagcagga ctccaaggac agcacctact ccctgtcctc caccctgacc 600

ctgtccaagg ccgactacga gaagcacaag gtgtacgcct gcgaagtgac ccaccagggc 660

ctgtctagcc ccgtgaccaa gtctttcaac cggggcgagt gc 702

<210> 25
<211> 234
<212> PRT
<213> Artificial Sequence

<220>
<223> Humanized Antibody

<400> 25

Met Val Leu Gln Thr Gln Val Phe Ile Ser Leu Leu Leu Trp Ile Ser
1 5 10 15

Gly Ala Tyr Gly Asn Ile Val Met Thr Gln Ser Pro Asp Ser Leu Ala
20 25 30

Val Ser Leu Gly Glu Arg Ala Thr Ile Asn Cys Lys Ala Ser Gln Asn
35 40 45

Val Gly Thr Asn Val Asp Trp Tyr Gln Gln Lys Pro Gly Gln Ser Pro
50 55 60

Lys Leu Leu Ile Tyr Gly Ala Ser Asn Arg Tyr Thr Gly Val Pro Asp
65 70 75 80

Arg Phe Ser Gly Ser Gly Ser Gly Thr Asp Phe Thr Leu Thr Ile Ser
85 90 95

Ser Leu Gln Ala Glu Asp Val Ala Val Tyr Asp Cys Leu Gln Tyr Lys
100 105 110

Tyr Asn Pro Tyr Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 26
<211> 702
<212> DNA
<213> Artificial Sequence

<220>
<223> Humanized Antibod

<400> 26
atggtgctgc agacccaggt gttcatctcc ctgctgctgt ggatctccgg cgcctacggc 60

aacatcgtga tgacccagtc cccctccagc ctgtctgctt ccgtgggcga cagagtgacc 120

atcaactgca aggcctccca gaacgtgggc accaacgtgg actggtatca gcagaagccc 180

ggcaagtccc ccaagctgct gatctacggc gccagcaaca gatacaccgg cgtgcccgac 240

agattctccg gctctggctc tggcaccgac tttaccctga ccatcagctc cctgcagccc 300

gaggacttcg ccacctacga ctgcctgcag tacaagtaca acccctacac cttcggccag 360

ggcacaaagg tggaaatcaa gcggaccgtg gccgctccct ccgtgtttat cttcccaccc 420

tccgacgagc agctgaagtc cggcacagct tctgtcgtgt gcctgctgaa caacttctac 480

cccgcgagg ccaaggtgca gtggaaggtg gacaacgccc tgcagtccgg caactcccag 540

gaatccgtga ccgagcagga ctccaaggac agcacctact ccctgtcctc caccctgacc 600

ctgtccaagg ccgactacga gaagcacaag gtgtacgcct gcgaagtgac ccaccagggc 660

ctgtctagcc ccgtgaccaa gtctttcaac cggggcgagt gc 702

<210> 27
<211> 234
<212> PRT
<213> Artificial Sequence

<220>
<223> Humanized Antibody

<400> 27

Met Val Leu Gln Thr Gln Val Phe Ile Ser Leu Leu Leu Trp Ile Ser
1 5 10 15

Gly Ala Tyr Gly Asn Ile Val Met Thr Gln Ser Pro Ser Ser Leu Ser
20 25 30

Ala Ser Val Gly Asp Arg Val Thr Ile Asn Cys Lys Ala Ser Gln Asn
35 40 45

Val Gly Thr Asn Val Asp Trp Tyr Gln Gln Lys Pro Gly Lys Ser Pro
50 55 60

Lys Leu Leu Ile Tyr Gly Ala Ser Asn Arg Tyr Thr Gly Val Pro Asp
65 70 75 80

Arg Phe Ser Gly Ser Gly Ser Gly Thr Asp Phe Thr Leu Thr Ile Ser
85 90 95

Ser Leu Gln Pro Glu Asp Phe Ala Thr Tyr Asp Cys Leu Gln Tyr Lys
100 105 110

Tyr Asn Pro Tyr Thr Phe Gly Gln Gly Thr Lys Val Glu Ile Lys Arg
115 120 125

Thr Val Ala Ala Pro Ser Val Phe Ile Phe Pro Pro Ser Asp Glu Gln
130 135 140

Leu Lys Ser Gly Thr Ala Ser Val Val Cys Leu Leu Asn Asn Phe Tyr
145 150 155 160

Pro Arg Glu Ala Lys Val Gln Trp Lys Val Asp Asn Ala Leu Gln Ser
165 170 175

Gly Asn Ser Gln Glu Ser Val Thr Glu Gln Asp Ser Lys Asp Ser Thr
180 185 190

Tyr Ser Leu Ser Ser Thr Leu Thr Leu Ser Lys Ala Asp Tyr Glu Lys
195 200 205

His Lys Val Tyr Ala Cys Glu Val Thr His Gln Gly Leu Ser Ser Pro
210 215 220

Val Thr Lys Ser Phe Asn Arg Gly Glu Cys
225 230

<210> 28
<211> 1407
<212> DNA
<213> Artificial Sequence

<220>
<223> Humanized Antibody

<400> 28
atgaagcacc tgtggttctt tctgctgctg gtggccgctc ccagatgggt gctgtctgaa 60

gtgcagctgg tggaatccgg cggaggcctc gtgaagcctt cccagaccct gtctctgacc 120

tgcaccgtgt ccggcttctc cctgacctcc ttccacgtgt catgggtgcg acagcctcca 180

ggcaagggcc tggaatggat cgccaccatc tcctctggcg gcggaaccta ctacaacccc 240

agcctgaagt ccagagtgac catctcccgg gacacctcca agaaccaggt gtccctgaag 300

ctgtcctccg tgaccgccgc tgataccgcc gtgtactact gcgccagaat ctccggctgg 360

ggccactact acgtgatgga cgtgtggggc cagggcaccc tcgtgacagt gtcctctgct 420

tccaccaagg gcccctccgt gtttcctctg gccccttcca gcaagtctac ctccggcgga 480

acagccgctc tgggctgcct cgtgaaagac tacttccccg agcccgtgac cgtgtcttgg 540

aactctggcg ctctgaccag cggcgtgcac acctttccag ctgtgctgca gtcctccggc 600

ctgtactccc tgtccagcgt cgtgactgtg ccctccagct ctctgggcac ccagacctac 660

atctgcaacg tgaaccacaa gccctccaac accaaggtgg acaagcgggt ggaacccaag 720

tcctgcgaca agacccacac ctgtccccct tgtcctgccc ctgaactgct gggcggacct 780

tccgtgttcc tgttcccccc aaagcccaag gacaccctga tgatctcccg gacccccgaa 840

gtgacctgcg tggtggtgga tgtgtcccac gaggaccctg aagtgaagtt caattggtac 900

gtggacggcg tggaagtgca caacgccaag accaagccta gagaggaaca gtacaactcc 960

acctaccggg tggtgtccgt gctgaccgtg ctgcaccagg attggctgaa cggcaaagag 1020

tacaagtgca aggtgtccaa caaggccctg cctgccccca tcgaaaagac catcagcaag 1080

gccaagggcc agccccggga accccaggtg tacacactgc cccctagccg ggaagagatg 1140

acaaaaaatc aggtgtcact gacctgtctc gtgaagggct tctacccctc cgatatcgcc 1200

gtggaatggg agtccaacgg ccagcctgag aacaactaca agaccacccc ccctgtgctg 1260

gactccgacg gctcattctt cctgtacagc aagctgacag tggacaagtc ccggtggcag 1320

cagggcaacg tgttctcctg ctccgtgatg cacgaggccc tgcacaacca ctacacccag 1380

aagtccctgt ccctgagccc cggcaaa 1407

<210> 29
<211> 469
<212> PRT
<213> Artificial Sequence

<220>
<223> Humanized Antibody

<400> 29

Met Lys His Leu Trp Phe Phe Leu Leu Leu Val Ala Ala Pro Arg Trp
1 5 10 15

Val Leu Ser Glu Val Gln Leu Val Glu Ser Gly Gly Gly Leu Val Lys
20 25 30

Pro Ser Gln Thr Leu Ser Leu Thr Cys Thr Val Ser Gly Phe Ser Leu
35 40 45

Thr Ser Phe His Val Ser Trp Val Arg Gln Pro Pro Gly Lys Gly Leu
50 55 60

Glu Trp Ile Ala Thr Ile Ser Ser Gly Gly Gly Thr Tyr Tyr Asn Pro
65 70 75 80

Ser Leu Lys Ser Arg Val Thr Ile Ser Arg Asp Thr Ser Lys Asn Gln
85 90 95

Val Ser Leu Lys Leu Ser Ser Val Thr Ala Ala Asp Thr Ala Val Tyr
100 105 110

Tyr Cys Ala Arg Ile Ser Gly Trp Gly His Tyr Tyr Val Met Asp Val
115 120 125

Trp Gly Gln Gly Thr Leu Val Thr Val Ser Ser Ala Ser Thr Lys Gly
130 135 140

Pro Ser Val Phe Pro Leu Ala Pro Ser Ser Lys Ser Thr Ser Gly Gly
145 150 155 160

Thr Ala Ala Leu Gly Cys Leu Val Lys Asp Tyr Phe Pro Glu Pro Val
165 170 175

Thr Val Ser Trp Asn Ser Gly Ala Leu Thr Ser Gly Val His Thr Phe
180 185 190

```
Pro Ala Val Leu Gln Ser Ser Gly Leu Tyr Ser Leu Ser Ser Val Val
        195                 200                 205

Thr Val Pro Ser Ser Ser Leu Gly Thr Gln Thr Tyr Ile Cys Asn Val
    210                 215                 220

Asn His Lys Pro Ser Asn Thr Lys Val Asp Lys Arg Val Glu Pro Lys
225                 230                 235                 240

Ser Cys Asp Lys Thr His Thr Cys Pro Pro Cys Pro Ala Pro Glu Leu
                245                 250                 255

Leu Gly Gly Pro Ser Val Phe Leu Phe Pro Pro Lys Pro Lys Asp Thr
            260                 265                 270

Leu Met Ile Ser Arg Thr Pro Glu Val Thr Cys Val Val Val Asp Val
        275                 280                 285

Ser His Glu Asp Pro Glu Val Lys Phe Asn Trp Tyr Val Asp Gly Val
    290                 295                 300

Glu Val His Asn Ala Lys Thr Lys Pro Arg Glu Glu Gln Tyr Asn Ser
305                 310                 315                 320

Thr Tyr Arg Val Val Ser Val Leu Thr Val Leu His Gln Asp Trp Leu
                325                 330                 335

Asn Gly Lys Glu Tyr Lys Cys Lys Val Ser Asn Lys Ala Leu Pro Ala
            340                 345                 350

Pro Ile Glu Lys Thr Ile Ser Lys Ala Lys Gly Gln Pro Arg Glu Pro
        355                 360                 365

Gln Val Tyr Thr Leu Pro Pro Ser Arg Glu Glu Met Thr Lys Asn Gln
    370                 375                 380

Val Ser Leu Thr Cys Leu Val Lys Gly Phe Tyr Pro Ser Asp Ile Ala
385                 390                 395                 400

Val Glu Trp Glu Ser Asn Gly Gln Pro Glu Asn Asn Tyr Lys Thr Thr
                405                 410                 415

Pro Pro Val Leu Asp Ser Asp Gly Ser Phe Phe Leu Tyr Ser Lys Leu
            420                 425                 430

Thr Val Asp Lys Ser Arg Trp Gln Gln Gly Asn Val Phe Ser Cys Ser
```

435 440 445

Val Met His Glu Ala Leu His Asn His Tyr Thr Gln Lys Ser Leu Ser
450 455 460

Leu Ser Pro Gly Lys
465

<210> 30
<211> 702
<212> DNA
<213> Artificial Sequence

<220>
<223> Humanized Antibody

<400> 30
atggtgctgc agacccaggt gttcatctcc ctgctgctgt ggatctccgg cgcctacggc 60

gacatccaga tgacccagag cccttccagc ctgtccgctt ccgtgggcga cagagtgacc 120

atcacctgtc aggcctccga ggacatctac tccggcctgg cctggtatca gcagaagccc 180

ggcaagtccc ccaagctgct gatctacggc gctggatctc tgcaggacgg cgtgccctct 240

agattctccg gctctggatc cggcacccac tacaccctga ccatctccag cctgcagccc 300

gaggacttcg ctacctactt ctgtcagcaa ggcctgaagt tccccctgac cttcggccag 360

ggcaccaagg tggaaatcaa gcggaccgtg gccgctccct ccgtgtttat cttcccaccc 420

tccgacgagc agctgaagtc cggcacagct tctgtcgtgt gcctgctgaa caacttctac 480

ccccgcgagg ccaaggtgca gtggaaggtg gacaacgccc tgcagtccgg caactcccag 540

gaatccgtga ccgagcagga ctccaaggac agcacctact ccctgtcctc taccctgacc 600

ctgtccaagg ccgactacga gaagcacaag gtgtacgcct gcgaagtgac ccaccagggc 660

ctgtctagcc ccgtgaccaa gtctttcaac cggggcgagt gc 702

<210> 31
<211> 234
<212> PRT
<213> Artificial Sequence

<220>
<223> Humanized Antibody

<400> 31

Met Val Leu Gln Thr Gln Val Phe Ile Ser Leu Leu Leu Trp Ile Ser
1 5 10 15

Gly Ala Tyr Gly Asp Ile Gln Met Thr Gln Ser Pro Ser Ser Leu Ser
20 25 30

```
Ala Ser Val Gly Asp Arg Val Thr Ile Thr Cys Gln Ala Ser Glu Asp
        35                  40                  45

Ile Tyr Ser Gly Leu Ala Trp Tyr Gln Gln Lys Pro Gly Lys Ser Pro
    50                  55                  60

Lys Leu Leu Ile Tyr Gly Ala Gly Ser Leu Gln Asp Gly Val Pro Ser
65                  70                  75                  80

Arg Phe Ser Gly Ser Gly Ser Gly Thr His Tyr Thr Leu Thr Ile Ser
                85                  90                  95

Ser Leu Gln Pro Glu Asp Phe Ala Thr Tyr Phe Cys Gln Gln Gly Leu
            100                 105                 110

Lys Phe Pro Leu Thr Phe Gly Gln Gly Thr Lys Val Glu Ile Lys Arg
        115                 120                 125

Thr Val Ala Ala Pro Ser Val Phe Ile Phe Pro Pro Ser Asp Glu Gln
    130                 135                 140

Leu Lys Ser Gly Thr Ala Ser Val Val Cys Leu Leu Asn Asn Phe Tyr
145                 150                 155                 160

Pro Arg Glu Ala Lys Val Gln Trp Lys Val Asp Asn Ala Leu Gln Ser
                165                 170                 175

Gly Asn Ser Gln Glu Ser Val Thr Glu Gln Asp Ser Lys Asp Ser Thr
            180                 185                 190

Tyr Ser Leu Ser Ser Thr Leu Thr Leu Ser Lys Ala Asp Tyr Glu Lys
        195                 200                 205

His Lys Val Tyr Ala Cys Glu Val Thr His Gln Gly Leu Ser Ser Pro
    210                 215                 220

Val Thr Lys Ser Phe Asn Arg Gly Glu Cys
225                 230
```